# EUROPEAN PATENT SPECIFICATION

(11) **EP 0 500 136 B1**
(45) Date of publication and mention of the grant of the patent: **15.05.1996**
(21) Application number: 92102981.5
(22) Date of filing: 24.02.1992
(51) Int. Cl.: C07D 487/04, C07D 495/14, C07D 471/14, A61K 31/505

(54) **1,2,4-Triazolo[1,5-a]pyrimidine derivatives, a process for preparing them as well as medicaments containing them and the use of them**
1,2,4-Triazolo[1,5-a]pyrimidinderivate, Verfahren zu ihrer Herstellung, diese enthaltende Arzneimittel und ihre Verwendung
Dérivés de la 1,2,4-triazolo[1,5-a]pyrimidine, procédé pour leur préparation, médicaments les contenant et leur utilisation

(30) Priority: 22.02.1991 HU 58791
(43) Date of publication of application: 26.08.1992
(73) Proprietor: EGIS GYOGYSZERGYAR, H-1106 Budapest (HU)
(72) Inventor: Reiter, Jozsef, Dr., H-1022 Budapest (HU); Berecz, Gábor, H-2750 Nagykörös (HU); Zsila, Giella, H-1131 Budapest (HU); Petöcz, Lujza, Dr., H-1084 Budapest (HU); Gigler, Gábor, H-1067 Budapest (HU); Fekete, Márton, Dr., H-1027 Budapest (HU); Szécsey, Mária, H-1089 Budapest (HU); Szirt, Enikö, H-1105 Budapest (HU); Rohács, Ludmila, Dr., H-1119 Budapest (HU); Görgényi, Frigyes, Dr., H-1115 Budapest (HU); Csörgo, Margit, Dr., H-1174 Budapest (HU)
(74) Representative: Beszédes, Stephan G., Dr.

(56) References cited:
- DD-A- 280 111
- DE-C- 1 792 811
- GB-A- 1 423 266
- US-A- 4 497 814
- JOURNAL OF HETEROCYCLIC CHEMISTRY. vol. 27, 1990, PROVO US pages 1559 - 1563; R. L. TOLMAN ET AL.: 'Annelated piperazinyl-7,8-dihydro-6H-thiopyrano(2,3-d)pyrimidines'
- CHEMICAL ABSTRACTS, vol. 114, 1991, Columbus, Ohio, US; abstract no. 62114W, R. LUDWIG: 'Preparation of 5,6-trimethylenetriazolo(1,5-a)pyrimidine-7-amines as cardiovascular agents' page 693; DD 279675 (VEB), 13.06.90
- INDIAN JOURNAL OF CHEMISTRY vol. 28B, 1989, NEW DELHI, INDIA pages 242 - 246; VISHNU J. RAM: 'Chemotherapeutic agents : Part XIII- Synthesis of 2-pyridyl-1,2,4-triazolo(1,5-a)pyrimidines as antimicrobial agent'
- CHEMICAL ABSTRACTS, vol. 110, 1989, Columbus, Ohio, US; abstract no. 212752M, VISHNU J. RAM: 'Chemotherapeutic agents. Part X. Synthesis of 2-pyridyl(1,2,4)triazolo(1,5-a)pyrimidines as leishmanicides' page 747; & Indian J. Chem., Sect B 1988, 27 B (9), 825-9
- JOURNAL OF HETEROCYCLIC CHEMISTRY. vol. 24, 1987, PROVO US pages 1149 - 1154; P. DVORTSAK: 'On triazoles. VIII (1). The reaction of 5-amino-1,2,4-triazoles with ethyl 2-cyano-3-ethoxyacrylate and 2-cyano-3-ethoxyacrylonitrile'

## Description

The invention is concerned with novel 1,2,4-triazolo-[1,5-a]pyrimidine derivatives of formula 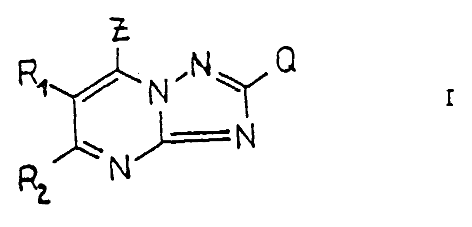 with the definitions indicated below including their salts, a process for preparing them as well as medicaments containing these compounds and the use of them.

From ex-DDR patent DD 280 111 and Chemical Abstracts, Vol. 114, 1991, Abstract No. 62114w, page 693 compounds have been known, in which
- R₁ and R₂: together represent the formula C
with
Y = CH₂ group
n = 1 and
m = 2,
- Q: means hydrogen and
- Z: represents the formula E
with
R₇ and/or R₈ = hydrogen, C₁₋₈ alkyl, optionally substituted by hydroxy, further furfuryl and/or benzyl
or
formula F
with
W = oxygen,
j = 2 and
k = 2
or 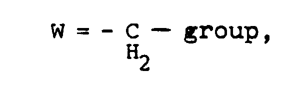
j = 2 and
k = 2
or 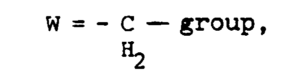
j = 2 and
k = 3.

For the compounds of this reference the application in the heart/circulation therapies has been indicated.

In Journal of Heterocyclic Chemistry, Vol. 27, 1990, pages 1 559 to 1 563 there have been described compounds, in which
- R₁ and R₂: together represent the formula C
with
Y = sulphur,
n = 0 and
m = 3,
- Q: means hydrogen and
- Z: represents the formula E
with
R₇ and R₈ = formula F
in the latter with
W = formula G
   in the latter with
   R₁₀ = hydrogen or tertiary butoxy carbonyl,
j = 2 and
k = 2.

At the beginning of the said reference globally antidiabetic properties had been indicated but it is questionable if even this is to be construed as applying for the said compounds.

From Indian Journal of Chemistry, Vol. 28B, 1989, pages 242 to 246 compounds have been known, in which
- R₁: stands for hydrogen,
- R₂: means a methyl group,
- Q: stands for a 2-, 3- or 4-pyridyl group and
- Z: represents the formula H
with
R₉ = ethoxycarbonylmethyl
or
- R₁: stands for hydrogen,
- R₂: means a methyl group,
- Q: represents 2-, 3- or 4-pyridyl and
- Z: represents the formula E
with
R₇ = hydrogen and
R₈ = 3,4-di-(chloro)-benzyl or 3,4-di-(methoxy)-phenylethyl.

According to page 244, the left column, lines 5 to 6 these compounds have not even any worth mentioning antimicrobial activity.

In Chemical Abstracts, Vol. 110, 1989, Abstract No. 212752m, page 747 there have been described compounds, in which
- R₁: stands for hydrogen,
- R₂: means a methyl group,
- Q: stands for a 2- or 4-pyridyl group and
- Z: represents the formula E
with
R₇ + R₈ = formula F
the latter with
W = formula G
   the latter with
   R₁₀ = methyl
j = 2 and
k = 2
or
- R₁: stands for hydrogen,
- R₂: means a methyl group,
- Q: stands for a 2- or 4-pyridyl group and
- Z: represents the formula H
with
R₉ = hydrogen.

These compounds are at most chemotherapeutic agents.

From US patent 4,497,814 there have been known compounds, in which
- R₁: stands for hydrogen or a C₁₋₆ alkyl group,
- R₂: means hydrogen or a C₁₋₆ alkyl group,
- Q: stands for a 2-, 3- or 4-pyridyl group, optionally substituted by a methyl group in the 2-position, and
- Z: represents the formula E
with
R₇ = hydrogen or C₁₋₆ alkyl and
R₈ = hydrogen or C₁₋₆ alkyl or
R₇ + R₈ = formula F
with
W = - CH₂ - group,
j = 2 and
k = 2.

As activity of these compounds the increase of cardiac contractility has been indicated.

In Journal of Heterocyclic Chemistry, Vol. 24, 1987, pages 1 149 to 1 154 it has been described a compound, in which
- R₁: stands for hydrogen,
- R₂: means hydrogen,
- Q: stands for a morpholino group and
- Z: represents formula E
with
R₇ = R₈ = hydrogen.
No application of this compound has been indicated in the said reference.

In British patent 1,423,266 there have been described compounds, in which
- R₁: stands for hydrogen or a methyl group,
- R₂: means hydrogen,
- Q: represents the formula A
with
p = 0 and
R₃ = benzyl group and
- Z: represents the formula E
with
R₇ = C₁-C₆-alkyl group, optionally substituted by a hydroxy group, and
R₈ = hydrogen or a C₁-C₆-alkyl group, optionally substituted by a hydroxy group
or
- Z: represents the formula E
with
R₇ and R₈ = formula F
with
j = k = 2 and 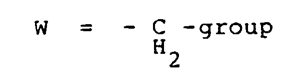 or
- Z: represents the formula E
with
R₇ and R₈ = formula F
with
j = k = 2 and
W = oxygen.

As pharmaceutical activity the coronary dilation including the positive inotropic effect has been indicated. As mechanism of effect the inhibition of phosphodiesterase enzyme has been described.

From German patent 1 792 811 the compound, in which
- R₁: stands for hydrogen,
- R₂: means a methyl group,
- Q: stands for hydrogen and
- Z: represents the formula E
with
R₇ = R₈ = ethyl group
has been known.

For this compound coronary dilating and antianginal activities have been indicated.

The problem underlying to the invention is to create novel 1,2,4-triazolo[1,5-a]pyrimidine derivatives showing valuable pharmacological properties, particularly positive inotropic and antianginal effects, supplemented by acute antiinflammatory, ulcus and gastric acid secretion inhibiting, furthermore by weaker tranquillant, spasmolytic and analgesic properties, a process for preparing them as well as medicaments containing these compounds and the use of them.

Surprisingly this has been solved by the invention.

Thus a subject-matter of the invention are 1,2,4-Triazolo[1,5-a]pyrimidine derivatives of the general formula 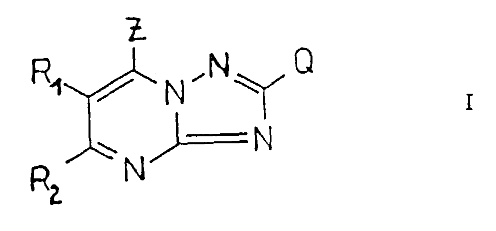 wherein
- Q: represents a morpholino, piperazino or piperidino group, optionally carrying a C₁₋₄ alkyl or benzyl substituent; or a group of the formula

S(O)ₚ-R₃ A

wherein
p stands for 0, 1 or 2 and
R₃ denotes straight or branched chained C₁₋₈ alkyl, C₂₋₆ alkenyl or phenyl-(C₁₋₄ alkyl), which latter may optionally carry one or more halogen or nitro substituent(s), or phenyl, optionally substituted by one or more nitro and/or trifluoromethyl; or a group of the formula 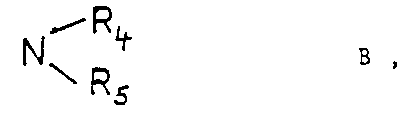 wherein
R₄ and R₅ independently each represent hydrogen, straight or branched chained C₁₋₁₂ alkyl, C₂₋₆ alkenyl, phenyl--(C₁₋₄ alkyl) including such having more than 1 phenyl group or di-(C₁₋₄ alkyl)-amino-(C₁₋₆ alkyl),
- R₁ and R₂: independently each represent hydrogen or straight or branched chained C₁₋₄ alkyl or
- R₁ and R₂: together form a group of the formula 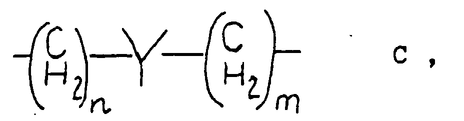 wherein
Y stands for a CH₂ group, a sulfur atom or a group of the formula 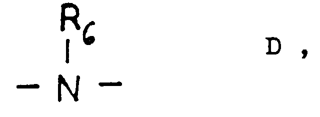 in which latter
R₆ denotes hydrogen or phenyl-(C₁₋₄ alkyl),
n and m each represent 0, 1, 2, 3, 4 or 5
and
- Z: represents a group of the formula 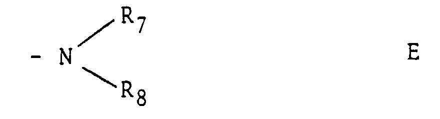 in which latter
R₇ and R₈ independently each represent hydrogen, straight or branched chained C₁₋₁₂ alkyl, optionally carrying one or more substituents(s) selected from the group consisting of hydroxy, carboxy, amino, morpholinocarbonyl, [4-(2'-{hydroxy}-ethyl)-piperazin-1'-yl]-carbonyl and (C₁₋₄ alkoxy)-carbonyl or optionally carrying a morpholino, piperazino or piperidino group, which optionally carry a C₁₋₄ alkyl or benzyl substituent; C₂₋₆ alkenyl, C₃₋₈ cycloalkyl, adamantyl or phenyl-(C₁₋₄ alkyl), which latter may optionally carry one or more substituent(s) selected from the group consisting of halogen, hydroxy, C₁₋₄ alkyl, C₁₋₄ alkoxy, amino and nitro; (C₁₋₄ alkyl)-amino-(C₁₋₈ alkyl) and di-(C₁₋₄ alkyl)-amino-(C₁₋₈ alkyl);
or
R₇ and R₈ together form a group of the formula 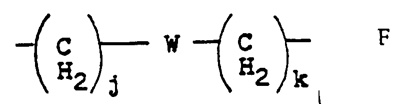 in which latter
j and k independently each represent 1, 2 or 3
and
W stands for oxygen or a 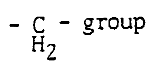 or a 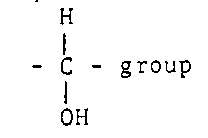 or a group of the formula 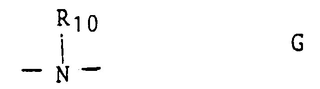 in which latter
R₁₀ denotes hydrogen, C₁₋₄ alkoxycarbonyl or C₁₋₄ alkyl, which latter may optionally carry one or more substituent(s) selected from the group consisting of hydroxy, (C₁₋₄-alkoxy)-carbonyl and phenyl;
or
- Z: stands for a group of the formula

-S-R₉ H

wherein
R₉ represents C₁₋₄ alkyl, optionally substituted by a (C₁₋₄ alkoxy)--carbonyl group,
with the provisos that
a) if Q represents a group of the formula A
   - R₁ and R₂: are other than hydrogen or C₁₋₄ alkyl,
b) if Q represents a morpholino group,
   - Z: represents formula E
   with
   R₇ = R₈ = hydrogen
   and
   - R₁: stands for hydrogen,
   R₂ is other than hydrogen,
and their salts.

The compounds according to the invention differ in the structure from the compounds from which in references an activity regarding heart/circulation has been indicated, particularly in that in the former Q must be different from hydrogen or in case of certain meanings of R₁ , R₂ and Z Q is different from a pyridyl group attached to the triazole ring through a carbon atom or in case that Q represents a group of the formula A R₁ and R₂ must be different from hydrogen or C₁₋₄ alkyl. Moreover the compounds according to the invention have the advantage that additionally to the positive inotropic and antianginal effects they have acute antiinflammatory, ulcus and gastric acid secretion inhibiting and furthermore weaker tranquillant, spasmolytic and analgesic properties not having been indicated in the references for the compounds described there.

The invention encompasses all the tautomeric forms of the 1,2,4-triazolo[1,5-a]pyrimidine derivatives of the general formula I.

The term "alkyl group" used throughout the specification relates to straight or branched chained saturated aliphatic hydrocarbon groups containing the given number of carbon atom(s), e.g. methyl, ethyl, tert.-butyl, n-butyl or n-octyl groups. The term "alkenyl group" designates straight or branched chained aliphatic hydrocarbon groups comprising at least one double bond, e.g. vinyl, allyl, prop-2-enyl, methylallyl or butenyl groups. The term "phenyl-(C₁₋₄ alkyl)" relates to C₁₋₄ alkyl groups in which 1 or more, preferably 2, hydrogen atom(s) is/are replaced by [a] phenyl group(s), e.g. benzyl, 1-phenylethyl or 2-phenylethyl groups or di-(phenyl)-methyl, 1,1-di-(phenyl)-ethyl, 2,2-di-(phenyl)-ethyl or 1,2-di-(phenyl)-ethyl groups. The "alkylaminoalkyl" and "dialkylaminoalkyl" groups comprise alkyl groups containing the given number of carbon atom(s), e.g. methylaminomethyl, methylaminoethyl, ethylaminoisopropyl,dimethylaminopropyl, dimethylaminoethyl or diisopropylaminoethyl groups.

The term "alkoxy group" also as a part of a group relates to alkyl ether groups comprising C₁₋₄ alkyl groups. Examples for C₁₋₄ alkoxy groups are methoxy, ethoxy and tert.-butoxy groups; examples for C₁₋₄ alkoxycarbonyl groups are methoxycarbonyl, ethoxycarbonyl and butoxycarbonyl groups.

The "C₃₋₈ cycloalkyl groups" may be e.g. cyclopropyl, cyclobutyl, cyclopentyl and cyclohexyl groups.

The term "halogen" encompasses the halogen atoms, that is fluorine, chlorine, bromine and iodine.

Preferably the halogen(s) which may be carried by the phenyl-(C₁₋₄ alkyl) group which may be represented by R₃ is/are chlorine and/or bromine.

Furthermore it is preferred that the C₁₋₄ alkyl substituent which may be carried by the morpholino, piperazino or piperidino group which may be represented by Q and/or the alkyl part(s) of the phenyl-(C₁₋₄ alkyl) group(s) which may be represented by R₃ and/or R₄ and/or R₅ and or R₆ and/or R₇ and/or R₈ and/or the C₁₋₄ alkyl group(s) of the di-(C₁₋₄ alkyl)-amino part(s) of the di-(C₁₋₄ alkyl)-amino-(C₁₋₆ alkyl) group(s) which may be represented by R₄ and/or R₅ and/or the C₁₋₄ alkyl group(s) of the (C₁₋₄ alkyl)-amino parts of the (C₁₋₄ alkyl)-amino-(C₁₋₈ alkyl) group(s) and/or of the di-(C₁₋₄ alkyl)-amino-(C₁₋₈ alkyl) group(s) which may be represented by R₇ and/or R₈ and/or the C₁₋₄ alkyl group which may be represented by R₉ or R₁₀ and/or the alkoxy part of the C₁₋₄ alkoxycarbonyl group which may be represented by R₁₀ and/or the C₁₋₄ alkyl group which may be represented by R₁ and/or R₂ is/are such one(s) having 1 or 2, particularly 1, carbon atom(s); and/or the C₁₋₈ alkyl group which may be represented by R₃ is such one having from 1 to 6, particularly from 1 to 3, carbon atom(s), most particularly a methyl, ethyl, propyl or 1-(methyl)-ethyl group, above all the first one; and/or the C₂₋₆ alkenyl group(s) which may be represented by R₃ or R₄ and/or R₅ and/or R₇ and/or R₈ is/are such one(s) having from 2 to 4, particularly 3, carbon atoms, most particularly [an] allyl group(s) and/or the C₁₋₁₂ alkyl group which may be represented by R₄ and/or R₅ and/or R₇ and/or R₈ is/are such one[s] having from 1 to 8, particularly 1 to 6, very particularly 1 to 4, most particularly 1 to 3, above all 1 or 2, carbon atom(s); and/or the C₁₋₆ alkyl group(s) of the amino-(C₁₋₆ alkyl) part(s) of the di-(C₁₋₄ alkyl)-amino-(C₁₋₆ alkyl) group(s) which may be represented by R₄ and/or R₅ is/are such one(s) having from 1 to 4, particularly 1 to 3, most particularly 3, carbon atom(s); and/or the C₁₋₈ alkyl group(s) of the amino-(C₁₋₈ alkyl) part(s) of the (C₁₋₄ alkyl)-amino-(C₁₋₈ alkyl) and/or di-(C₁₋₄ alkyl)-amino-(C₁₋₈ alkyl) group(s) which may be represented by R₇ and/or R₈ is/are such one(s) having from 1 to 6, particularly 1 to 3, carbon atom(s); and/or the C₃₋₈ cycloalkyl group(s) which may be represented by R₇ and/or R₈ is/are such one(s) having 5 or 6 carbon atom(s).

Particularly preferred 1,2,4-triazolo[1,5-a]pyrimidine derivatives are those in which
- Q: represents a morpholino, piperazino or piperidino group or a (C₁₋₆ alkyl)-thio group,
- R₁ and R₂: independently each represent hydrogen or straight or branched chained C₁₋₄ alkyl or together form a 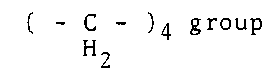 and
- Z: stands for a group of formula E, wherein R₇ and R₈ independently each represent straight or branched chained C₁₋₆ alkyl, which may optionally carry a morpholino, piperazino or piperidino group.

Conveniently the salts of the 1,2,4-triazolo[1,5-a]pyrimidine derivatives of the general formula I are pharmaceutically acceptable ones.They can be formed with inorganic or organic acids. Examples for such acid-addition salts are the hydrochlorides, hydrobromides, sulfates, citrates, maleates, fumarates and ethanesulfonates.

Particularly preferred representatives of the 1,2,4-triazolo[1,5-a]pyrimidine derivatives according to the invention are 2-(Methylthio)-5-{N-[3'-(morpholino)-propyl]}--amino-6,7,8,9-tetrahydro-1,2,4-triazolo[5,1-b]quinazoline, 5-(diethylamino)-7-methyl-2-morpholino-1,2,4-triazolo[1,5-a]pyrimidine, 2-[1'-(methyl)-ethyl-thio]-5-{N-[3'-(morpholino)--propyl]}-amino-6,7,8,9-tetrahydro-1,2,4-triazolo[5,1-b]quinazoline, 2-(ethylthio)-5-{N-[2'-(morpholino)-ethyl]}-amino-6,7,8,9-tetrahydro-1,2,4-triazolo[5,1-b]quinazoline and 2-(methylthio)-5-{N-[2'-(morpholino)-ethyl]}-amino-6,7,8, 9,10,11,12,13,14,15-decahydro-cyclododeca[d]-1,2,4-triazolo[1,5-a]pyrimidine and salts thereof.

As regards the nomenclature of the above compounds and in the entire further text it is referred to J. Heterocyclic Chem. 24 [1987], 1 503.

According to a further aspect of the present invention there is provided a process for preparing the compounds according to the invention, which is characterized by reacting 1,2,4-triazolo[1,5-a]pyrimidine derivatives of the general formula 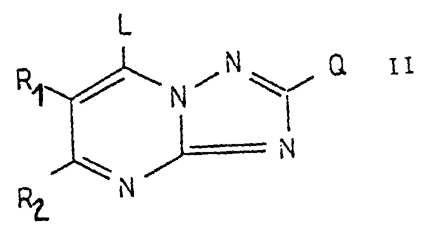 wherein
- Q, R₁ and R₂: are as above stated
and
- L: represents a leaving group, selected from halogen, tri-(C₁₋₄ alkyl)-silyloxy, C₁₋₄ alkylsulfonyloxy, arylsulfonyloxy or C₁₋₄ alkylarylsulfonyloxy,
with an amine or thiol of the general formula

H - Z III

wherein
- Z: is as above stated
and optionally in a manner known per se converting a 1,2,4-triazolo[1,5-a]pyrimidine derivative of the formula I thus obtained into a salt or converting a salt of the 1,2,4-triazolo[1,5-a]pyrimidine derivative of the general formula I thus obtained into the free base of formula I or into another salt.

If the reaction of the 1,2,4-triazolo[1,5-a]pyrimidine derivative of the general formula II is carried out with an amine, that is with a compound of the general formula III, wherein Z is a group of the formula E, suitably this may be done with an excess thereof which may serve as a solvent. In this case preferably at least 2 moles of amine of the general formula III related to 1 mole of the 1,2,4-triazolo [1,5-a]pyrimidine derivative of the general formula II is used.

When using the amine of the general formula III in an equimolar amount, or when reacting a thiol of the general formula III, advantageously the reaction is performed in a solvent. Preferably the reaction of the 1,2,4-triazolo[1,5-a] pyrimidine derivative of the general formula II, wherein L represent halogen, C₁₋₄ alkylsulfonyloxy, arylsulfonyloxy or C₁₋₄ alkylarylsulfonyloxy, with the amine or thiol of the formula III is carried out in a protic, polar aprotic or apolar aprotic solvent, in the presence of a basic acid-binding agent. As protic solvents preferably aliphatic alcohols and/or diols, as polar aprotic solvents preferably acetonitrile, dimethylformamide and/or tetrahydrofuran and as apolar aprotic solvents preferably benzene or homologues thereof, 1,2 -di-(chloro)-ethane, chlorobenzene and/or chloroform are used. As basic acid-binding agents preferably tertiary amine bases, particularly triethylamine, are applied.

When reacting 1,2,4-triazolo[1,5-a]pyrimidine derivatives of the general formula II, wherein L stands for tri-(C₁₋₄ alkyl)-silyl, with an equimolar amount of amine or with a thiol of the general formula III, wherein Z denotes a group of the formula H, the reaction is preferably performed in a polar aprotic or apolar aprotic solvent. As polar aprotic solvents preferably acetonitrile, dimethylformamide and/or tetrahydrofurane and as apolar aprotic solvents preferably benzene or homologues thereof, 1,2-di-(chloro)-ethane, chloroform and/or hexamethyldisilazane are used. As another preferred alternative as a 1,2,4-triazolo[1,5-a]pyrimidine derivative of the general formula II, wherein L represents tri-(C₁₋₄ alkyl)-silyl, such prepared in situ is reacted with amine or thiol of the general formula III, that is the reaction is carried out in the reaction mixture where it was formed. In this case an excess of the silylating agent used for the preparation of the 1,2,4-triazolo[1,5-a]pyrimidine derivatives of the general formula II may serve as solvent.

The reaction can be performed at any temperature between room temperature and the boiling point of the solvent or amine of the general formula III used in an excess. Preferably it is carried out at temperatures from 30°C to 80°C.

The 1,2,4-triazolo[1,5-a]pyrimidine derivatives of the general formula I thus obtained can be separated from the reaction mixture by known methods. They crystallize from the reaction mixture either directly or upon adding some water and can be filtered off. If they separate in an oily form, they can be extracted with a water-immiscible organic solvent, evaporated and clarified by methods known per se.

The 1,2,4-triazolo[1,5-a]pyrimidine derivatives of the general formula II used as starting substances can be prepared from the known compounds of the general formula 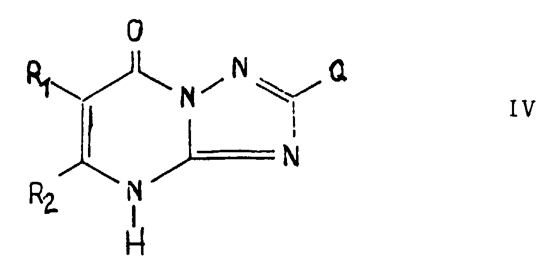 wherein
- R₁ , R₂ and Q: are as above defined
by converting the oxo group of the latter into a leaving group, preferably with a mineral acid halide, silylating agent or alkyl- or aryl- or alkylarylsulfonyl halide. This process has been described in detail in copending European patent application of the Applicant claiming the priority of Hungarian patent application No. 588/91 simultaneously filed.

The starting amines and thiols of the general formula III are commercial products.

As already mentioned, the 1,2,4-triazolo[1,5-a]pyrimidine derivatives of the general formula I possess valuable pharmacological, particularly positive inotropic and antianginal effects and these activities are supplemented by acute antiinflammatory, ulcus and gastric acid secretion inhibiting, furthermore by weaker tranquillant, spasmolytic and analgesic properties.

Hence by the invention also there are provided for medicaments, which are characterized in that they contain as [an] active ingredient(s) at least one compound according to the invention, suitably in admixture with at least one inert solid and/or liquid pharmaceutical carrier and/or diluent and or additive.

The medicaments according to the invention can be in the form of pharmaceutical preparations. These can be prepared by using methods known per se and thus suitably by admixing the 1 or more 1,2,4-triazolo[1,5-a]pyrimidine derivative(s) according to the invention with 1 or more inert solid and/or liquid carrier(s) and/or diluent(s) and/or additive(s) and bringing the mixture to a galenic form.

The pharmaceutical preparations of the present invention may be suitable for oral (e.g. tablet, pill, coated pill, dragee, solid or soft gelatine capsule, solution, emulsion or suspension), parenteral (e.g. injection solution) or rectal (e.g. suppository) administration.

The carrier(s) for the preparation of tablets, coated tablets, dragees and solid gelatine capsules can be e.g. lactose, corn starch, potato starch, talc, magnesium carbonate, magnesium stearate, calcium carbonate and/or stearic acid and/or [a] salt(s) thereof. The carrier(s) for the soft gelatine capsules can be e.g. 1 or more vegetable oil(s), fat(s), wax(es) and/or polyol(s) of suitable consistency. The carrier(s) for the solutions and syrups can be e.g. water, 1 or more polyol(s), such as polyethylene glycol(s), saccharose and/or glucose. The injection solutions can contain e.g. water, 1 or more alcohol(s), polyol(s), glycerol and/or vegetable oil(s) as carrier. The carriers of the suppositories can be e.g. 1 or more oil(s), wax(es), fat(s) and/or polyol(s) of suitable consistency.

In addition, the pharmaceutical preparations may comprise 1 or more additive(s) [auxiliaries] usually applied in the pharmaceutical industry, e.g. 1 or more wetting and/or sweetening agent(s) and/or aroma substance(s), salt(s) causing the change of osmotic pressure and/or buffer(s). The pharmaceutical preparations may further contain 1 or more other active ingredient(s), too.

The 1,2,4-triazolo[1,5-a]pyrimidine derivatives according to the invention can preferably be used in therapy orally in the form of tablets or capsules. Especially preferred are the capsules or tablets comprising about 50 mg of active ingredient according to the invention.

The daily dose of the 1,2,4-triazolo[1,5-a]pyrimidine derivatives according to the invention can vary within wide ranges depending on several factors, e.g. on the activity of the active ingredient(s), the patient's condition and age i and the severity of the disease. The oral dose is generally 1 to 1 000 mg/day, preferably 10 to 200 mg/day. It has to be stressed that these dose values are only of informative character and the administered dose must always be determined by the physician therapeutist.

Hence a further subject-matter of the invention is the use of the compounds according to the invention for preparing medicaments, particularly such having positive inotropic, antianginal, antiinflammatory and ulcus and gastric acid secretion inhibiting effects.

The preferred application of the 1,2,4-triazolo[1,5-a] pyrimidine derivatives according to the invention is the cardiotonic and antianginal treatment which comprises administering to the patient an effective amount of 1 or more 1,2,4-triazolo[1,5-a]pyrimidine derivatives according to the invention.

The activity of the 1,2,4-triazolo[1,5-a]pyrimidine derivatives according to the invention is shown by the following tests.

### 1. Positive inotropic effect in anaesthetized cat

The experiments were carried out on male and female cats anaesthetized with chloralose-urethane (40/300 mg/kg i.p. in a volume of 1 ml/kg). A polyethylene catheter was inserted into the left ventricle via the right subclavian artery. The inotropic activity was measured with the aid of the first derivative by time of the left ventricular pressure curve (dP/dtₘₐₓ). The blood pressure was measured by means of another catheter introduced from the left femoral artery into the upper third part of the abdominal artery. A Statham P23Db transducer and HSE (Hugo Sachs Electronics) amplifiers were used for both pressure measurements. The signals thus obtained were recorded by a Lineacorder Mark VII polygraph of WR 3101 type. The test compounds were administered via a femoral venous cannula.

The effects of the compounds are characterized by the positive inotropic responses (dP/dtₘₐₓ) shown in the following Table I.

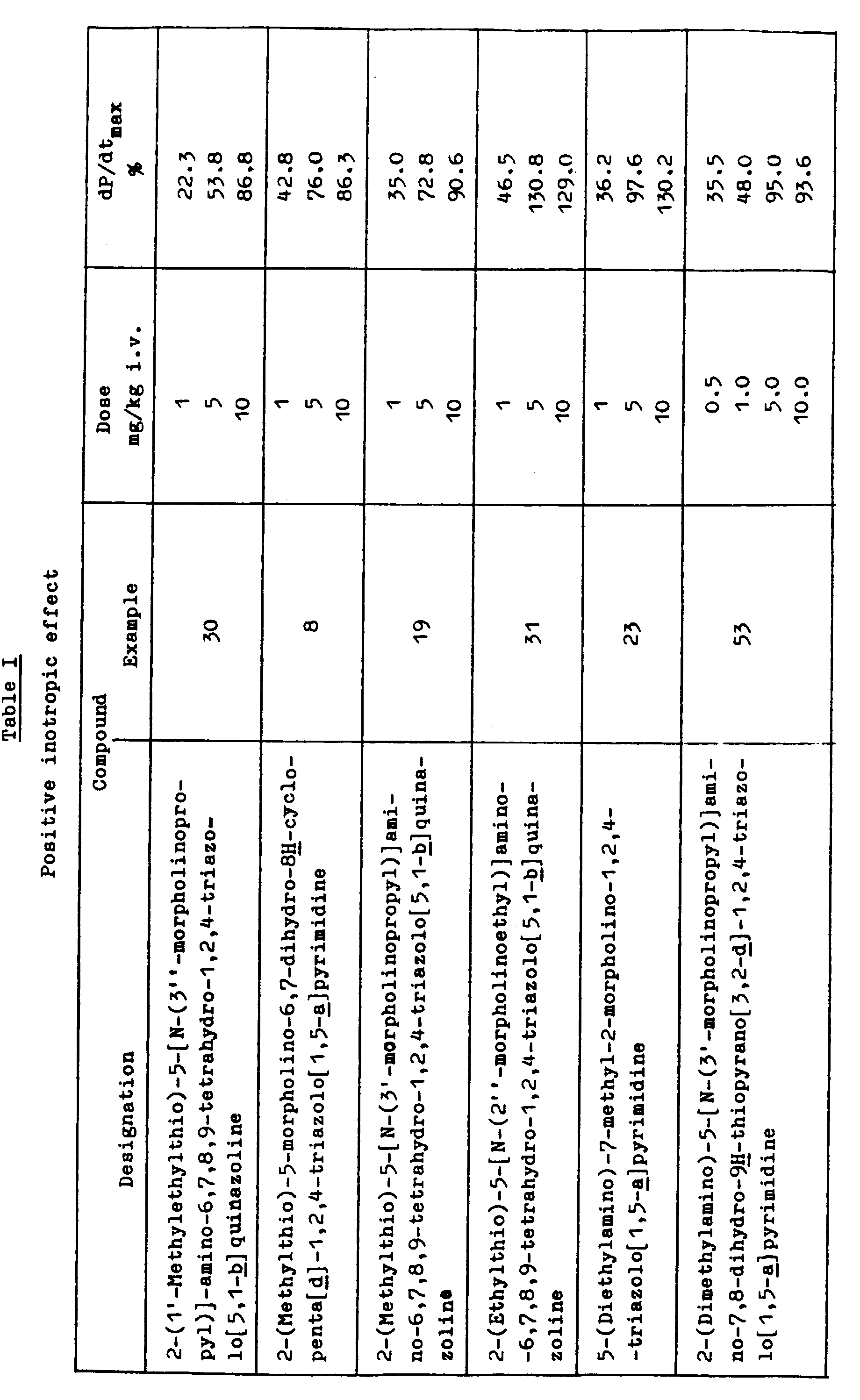

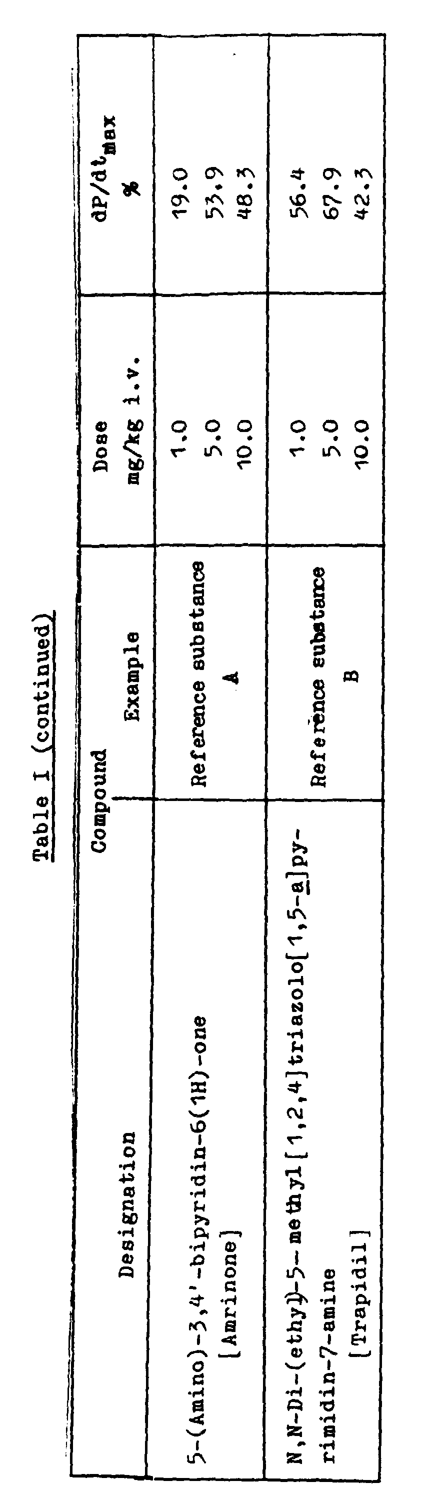

From the above Table I it can be established that the tested 1,2,4-triazolo[1,5-a]pyrimidine derivatives according to the invention increase the dP/dtₘₐₓ values in a dose-dependent way, the said values being characteristic of the myocardial force. At the same time, in case of both reference compounds (A and B) the effect does not exceed 50% even in the highest dose (10 mg/kg). Considering equal doses the compounds according to the invention are 1.5 to 3.5 times more effective than the reference compounds.

### 2. Positive inotropic effect in guinea pig

The experiments were carried out by the method of Alousi (Alousi, A. A. et al.: J. Cardiovasc. Pharmacol. 5, 804 to 811 [1983]). Animals weighing 400 to 600 g were stunned with a blow to the head and exsanguinated. The heart was excised and the right atrium was removed and placed into a modified Tyrode solution of 37°C. It was then preloaded with 2.1 g and stimulated electrically at a rate of 2 Hz by rectangular pulses 0.5 ms in duration. The first derivative by time of the contraction force of the isolated organ (dP/dt) and the atrial frequency were recorded by a polygraph. From the data thus obtained percentage changes were calculated. The results are shown in the following Table II.

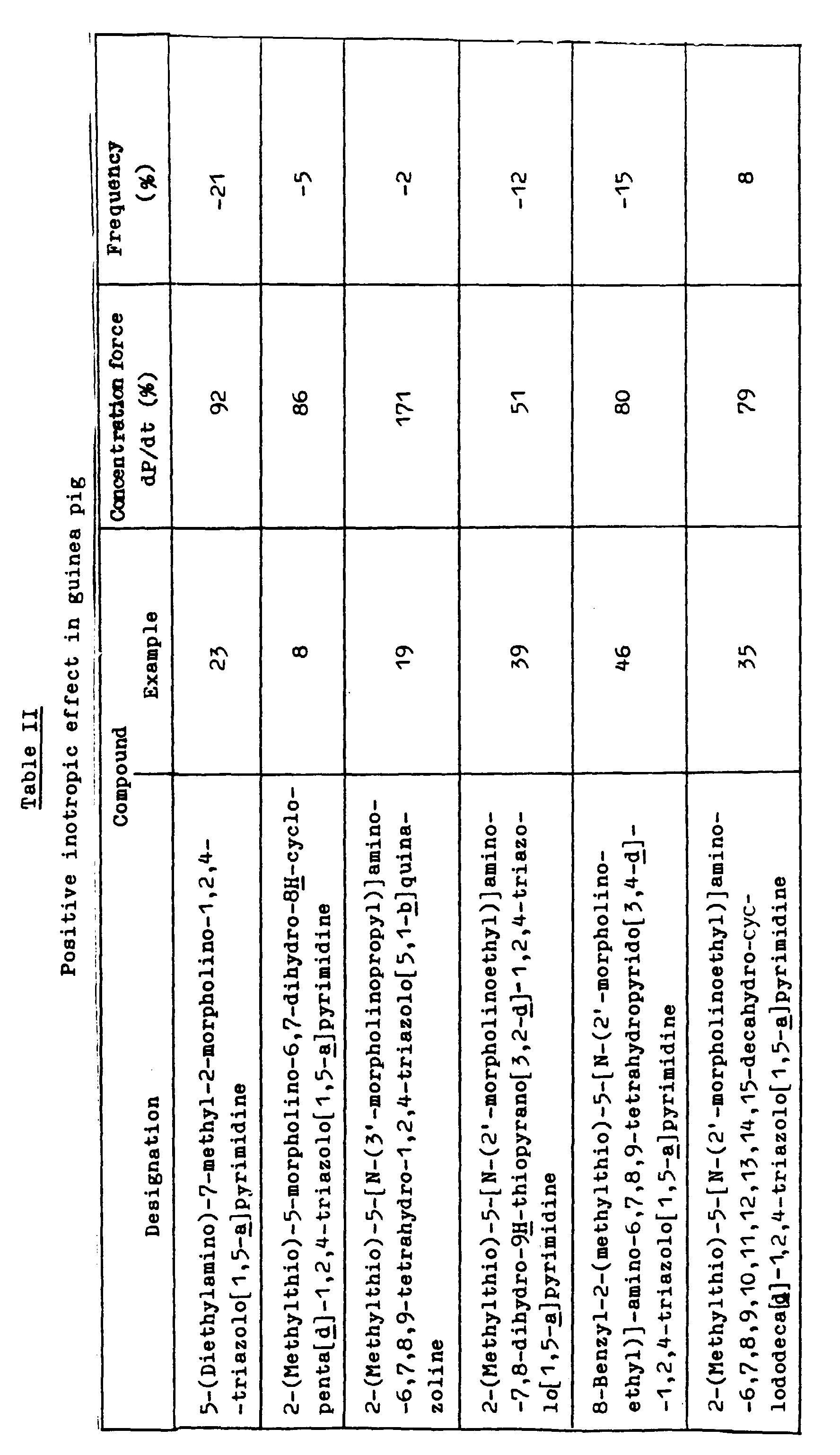

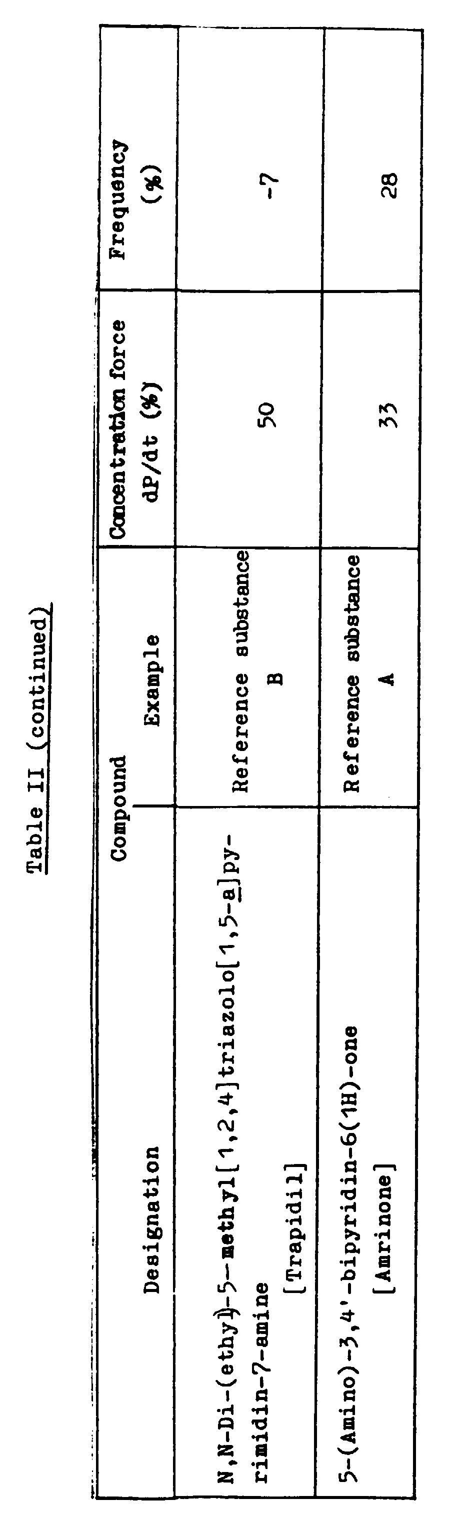

According to the above Table II the positive inotropic effect of the tested 1,2,4-triazolo[1,5-a]pyrimidine derivatives according to the invention considerably surpasses that of the reference substances (A and B). Besides, the former are less tachycardic.

### 3.a) Antianginal effect in rats

The experiments were carried out by the method of Leitold (Leitold, M. and Laufen, H.: Arzneimittel Forschung 33, 1 117 to 1 121 [1983]). Rats weighing 180 to 220 g were narcotized with chloralose-urethane (70/700 mg/kg i.p.). The ECG was registered with needle electrodes in standard II leading. The experimental coronaria insufficiency was induced with vasopressin (1 NE/kg i.v.). The height of T wave in ECG was measured before and after the administration of vasopressin in both the control and treated groups. The test compounds were administered intravenously 2 minutes prior to the treatment with vasopressin. The ED₅₀ values of the compounds are shown in the following Table IIIA.

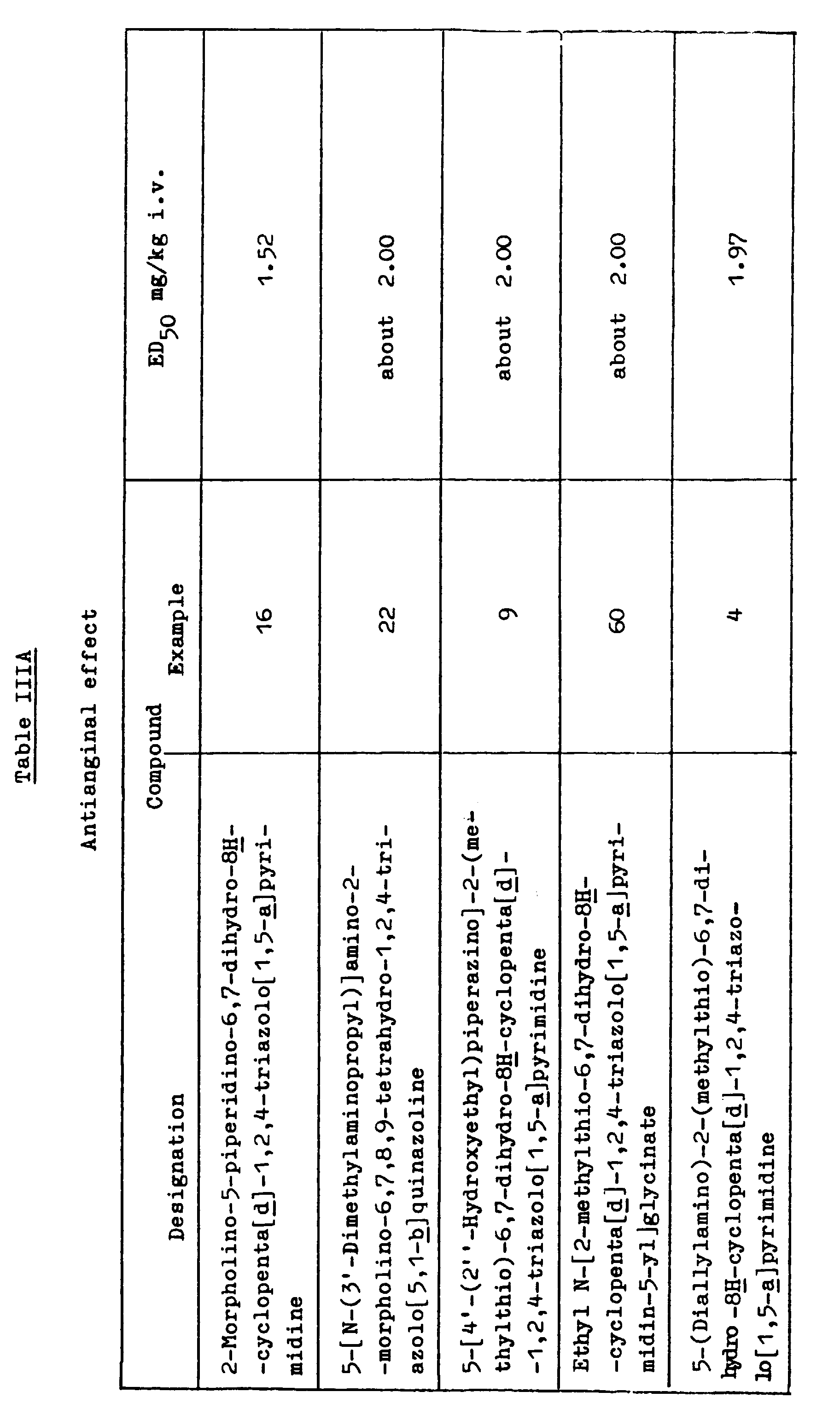

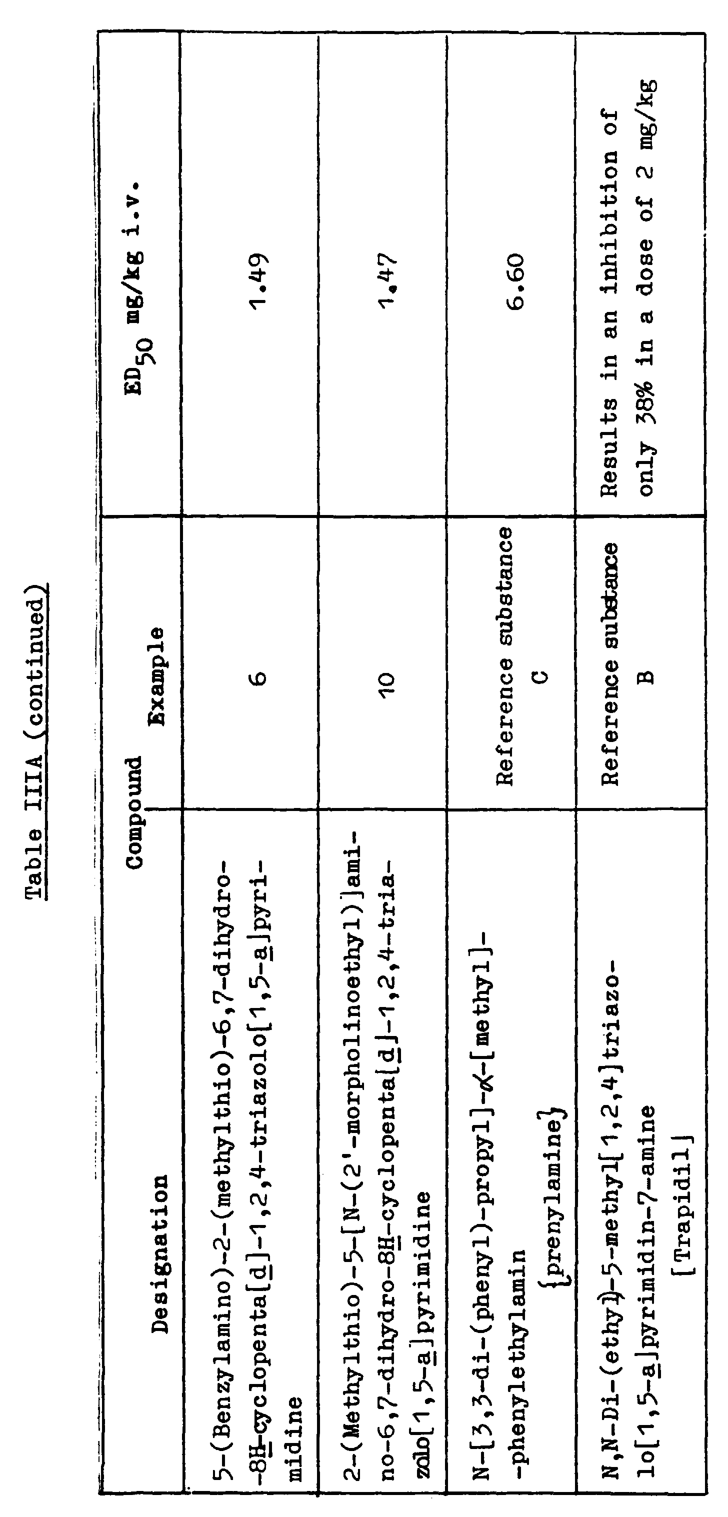

From the above Table IIIA it can be seen that the tested 1,2,4-triazolo[1,5-a]pyrimidine derivatives according to the invention are superior to the reference compounds (B and C), they are 3 to 4 times more effective than N-[3,3-di-(phenyl)-propyl]-α-[methyl]-phenylethylamin {prenylamine} 〈Reference substance C〉 and there is a similar superiority of the former as compared to N,N-di-(ethyl-5-methyl-[1,2,4]triazolo[1,5-a] pyrimidin-7-amine [trapidil] 〈Reference substance B〉.

### 3.b) Coronary flow test in dog

The experiments were carried out in young mongrel dogs of both sexes weighing 10 to 26 kg. The test compounds were administered in 1-5-10 mg/kg doses intravenously.

The animals were anaesthetized by (RS)-5-[ethyl]-5-[1'-(methyl)-butyl]-barbituric acid {pentobarbital} (Nembuta®, Serva) and artificially ventilated (type: RO-5). In open chest dogs blood flow in the left anterior descending coronary artery was measured continuously by an electromagnetic flowmeter (Godard-Statham, type: SP 2202). Mean arterial blood pressure was recorded on the carotis with a Statham gauge (type: p23Db). The changes of the myocardial contractile forces were followed isometrically with a microdynamometer (type: Experimetria, SG-01) affixed to the anterior wall of the left ventricle. Data were collected on the first derivate of the myocardial contractile force to follow the changes of the contraction and relaxation speed. The data were recorded by an apparatus of Medicor CH-61 type. The changes of the tissular flow in the left anterior coronary wall were recorded by the so-called thermoclearance method (Golenhofen 1964) with a Radelkis direct recording apparatus. The changes of the heart rate were also followed. The conductivity of the coronary artery (the ratio of the mean coronary flow and the mean blood pressure) was calculated. The mean values were evaluated statistically using Student's test as well as regression analysis and multiple t test. The data are shown in the following Table IIIB.

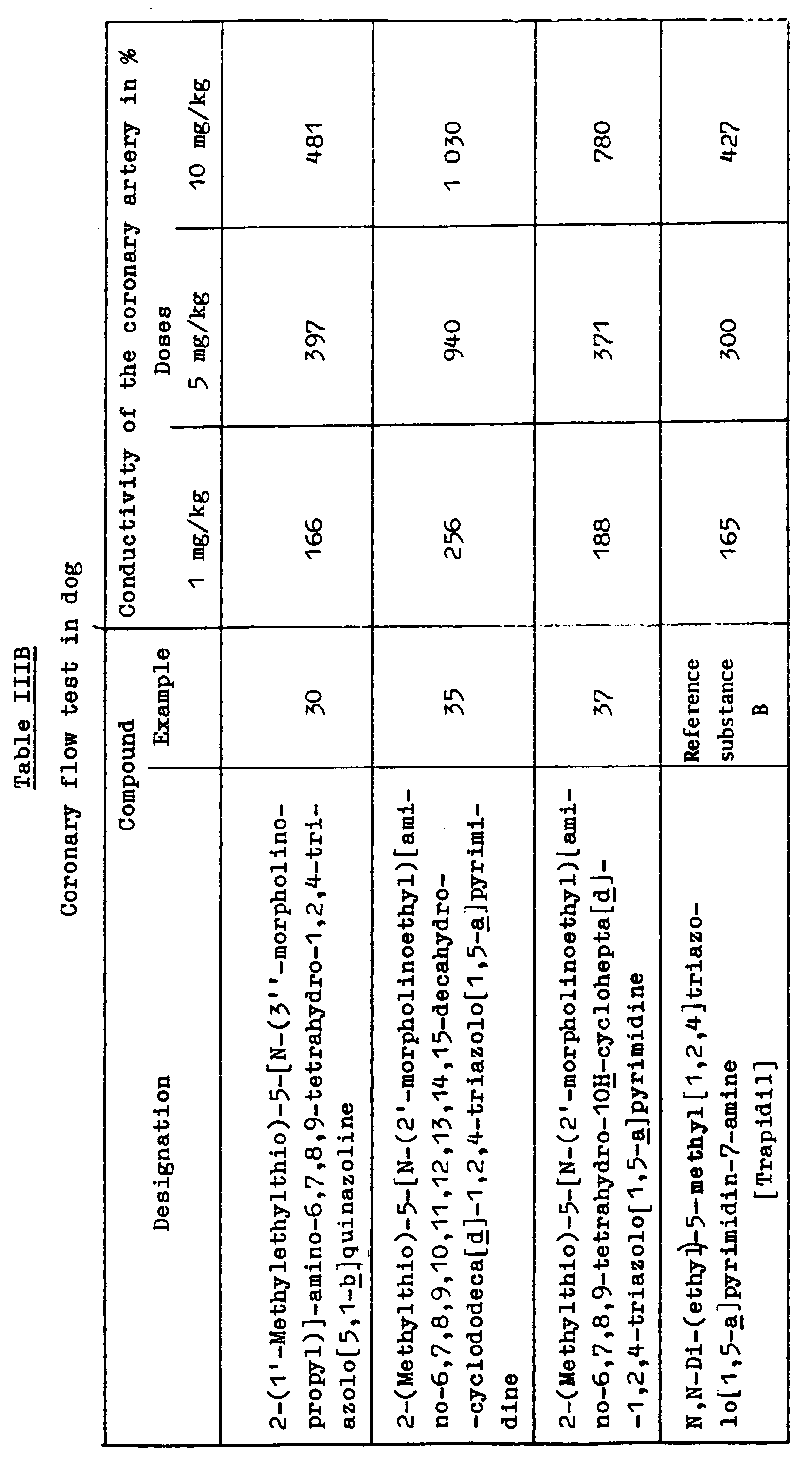

From the data of the above Table IIIB it can be seen that the tested 1,2,4-triazolo[1,5-a]pyrimidine derivatives according to the invention improve the coronary flow and they are more effective than the reference substance.

### 4. Antiinflammatory (Carrageen oedema inhibiting) effect

The experiments were carried out by the method of Winter et al. (Winter, C. A. et al.: Proc. Soc. Exp. Biol. Med. 111, 544 to 547 [1962]) in rats weighing 150 to 180 g. 0.1 ml of a 1 per cent carrageen suspension was injected subcutaneously into the plantar region of one of the hind paws. Rats were fasted for 12 hours and received drinking water ad libitum. One hour before treatment with the test compound the animals were hydrated orally with 30 ml/kg of tap water. The test compounds or the vehicle were administered p.o. in a volume of 10 ml/kg, then one hour later carrageen was applied. The volume of the treated paw was measured by a plethysmometer before and 3 hours after injection in such a way that displacement of the liquid arising from the volume alteration was indicated on a millimeter scale. The volume of the treated paws were compared with those of the control group. The dose resulting in an inhibition of 30% (ID₃₀) was determined by the aid of a line of regression. The results are given in the following Table IV.

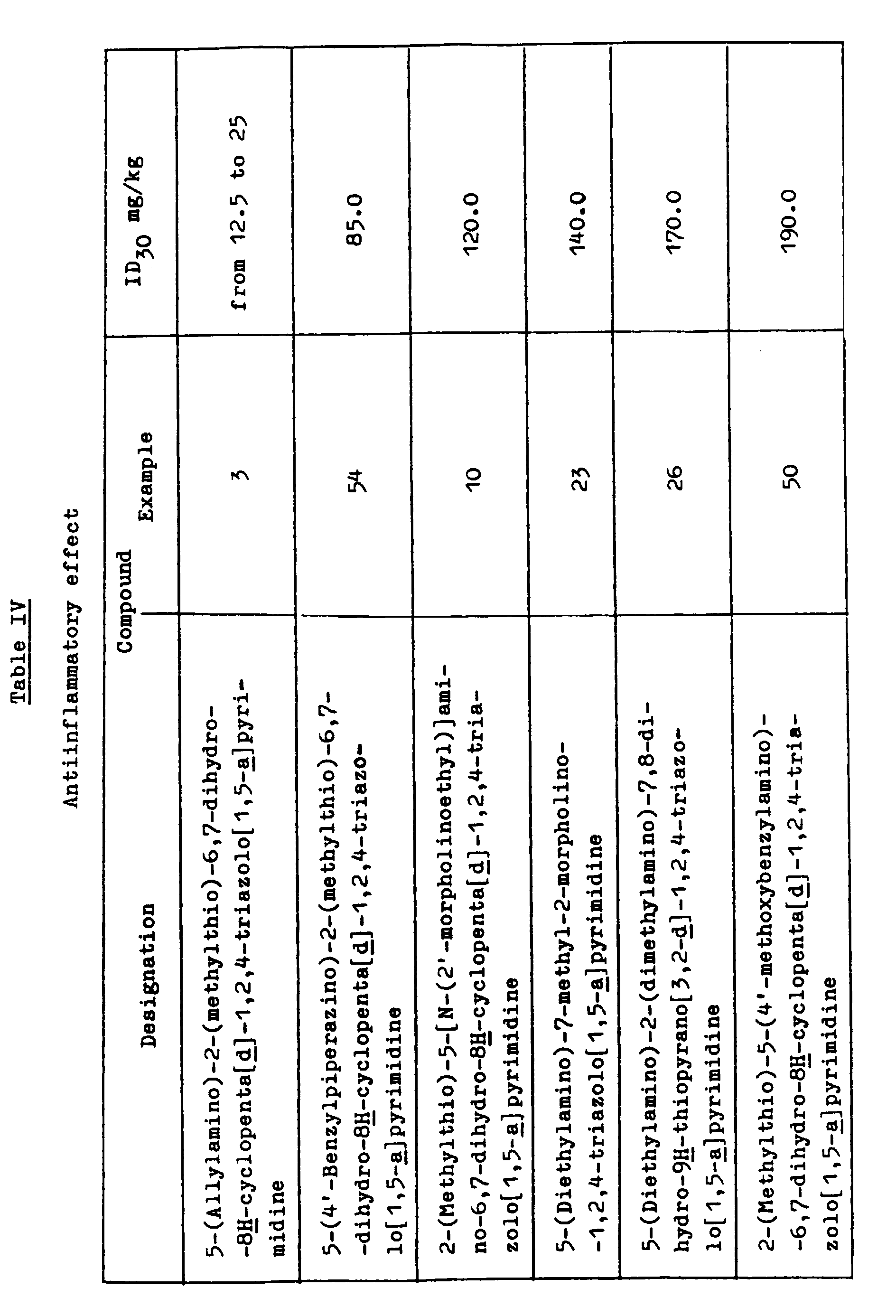

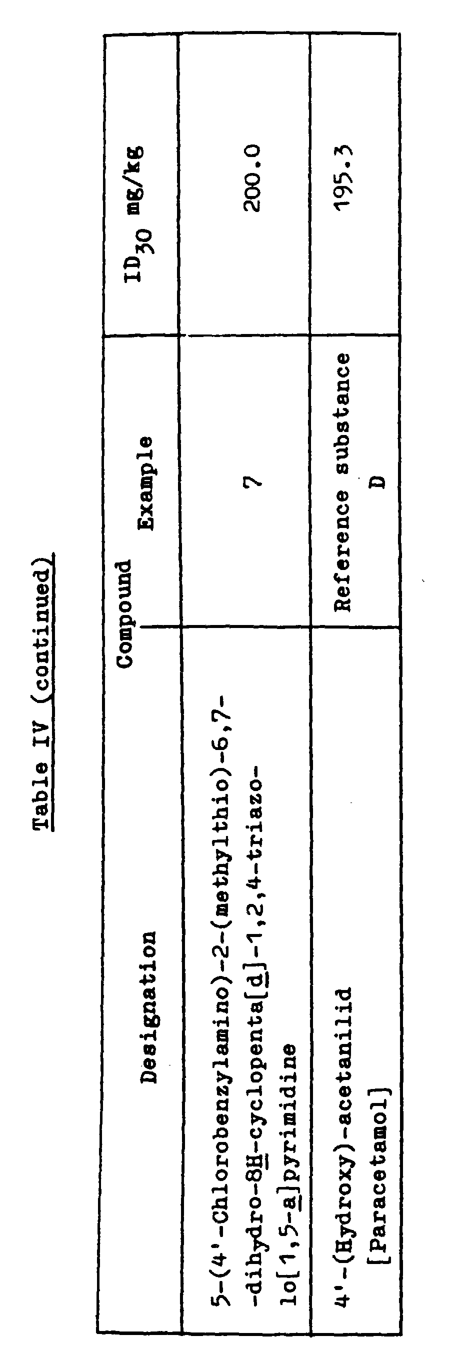

From the above Table IV it can be seen that one of the tested 1,2,4-triazolo[1,5-a]pyrimidine derivatives according to the invention is at least 8 times more effective than 4'-(hydroxy)-acetanilid [paracetamol] {Reference substance D} 3 further derivatives according to the invention are 1.4 to 2.3 times more effective than the said reference substance and 3 further derivatives according to the invention can be considered as effective as the said reference substance.

### 5. Motility inhibiting activity

The tests were performed according to the method of Borsy et al. (Arch. Int. Pharmacodyn. 124, 1 to 2 [1960]). Groups consisting of 3 mice each were treated orally with different doses of the compounds to be tested. Then the test animals were placed in a Dews equipment. In this equipment the number of interruptions of infrared beam within 30 minutes was counted. The inhibition is expressed as the percentage of the value obtained for the control animals. The reference 2-(methyl)-2-(propyl)-trimethylencarbamat [meprobamate] is inactive in a dose of 100 mg/kg. The results are given in the following Table V.

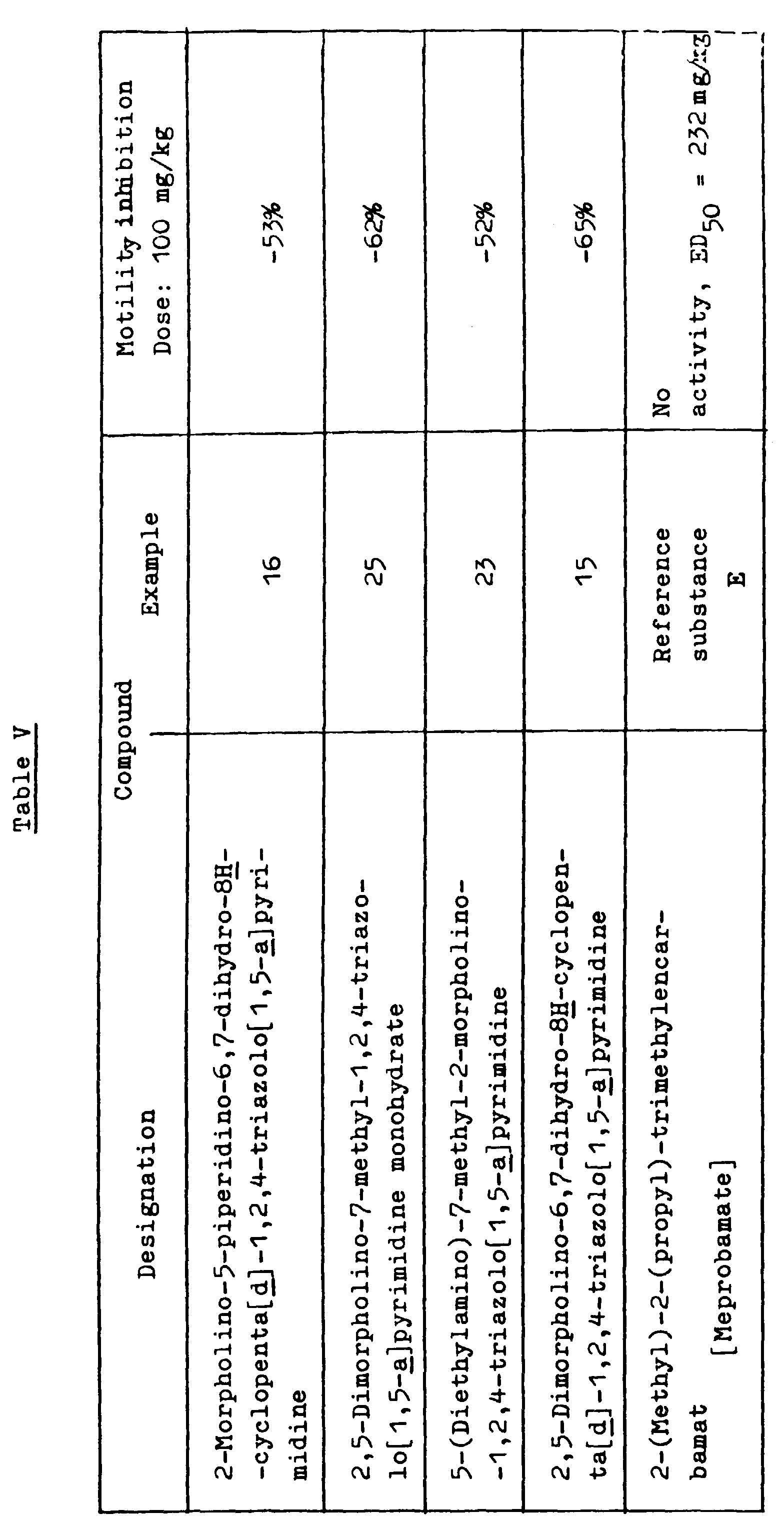

From the above Table V it can be established that the activity of the tested 1,2,4-triazolo[1,5-a]pyrimidine derivatives according to the invention surpasses that of the said reference substance (E).

### 6. Narcosis potentiating activity

Groups consisting of 6 mice each were orally treated with the compound to be examined. After one hour, 5-(1'-cyclohexenyl)-1,5-di-(methyl)-barbituric acid [hexobarbital] was injected intravenously at a dosage of 40 mg/kg. The control group was treated only with 5-(1'-cyclohexenyl)-1,5-di-(methyl)-barbituric acid [hexobarbital] to provoke sleep. The duration of sleep was recorded. If the duration of sleep of an animal exceeded that of the mean value of the control group by a factor of 2.5, it was considered as a positive reaction. The frequency of the positive reaction is expressed as the percentage of the value obtained for the control animals. The reference substance 2-(methyl)-2-(propyl)-trimethylencarbamat [meprobamate] is inactive in a dose of 100 mg/kg. The results are given in the following Table VI.

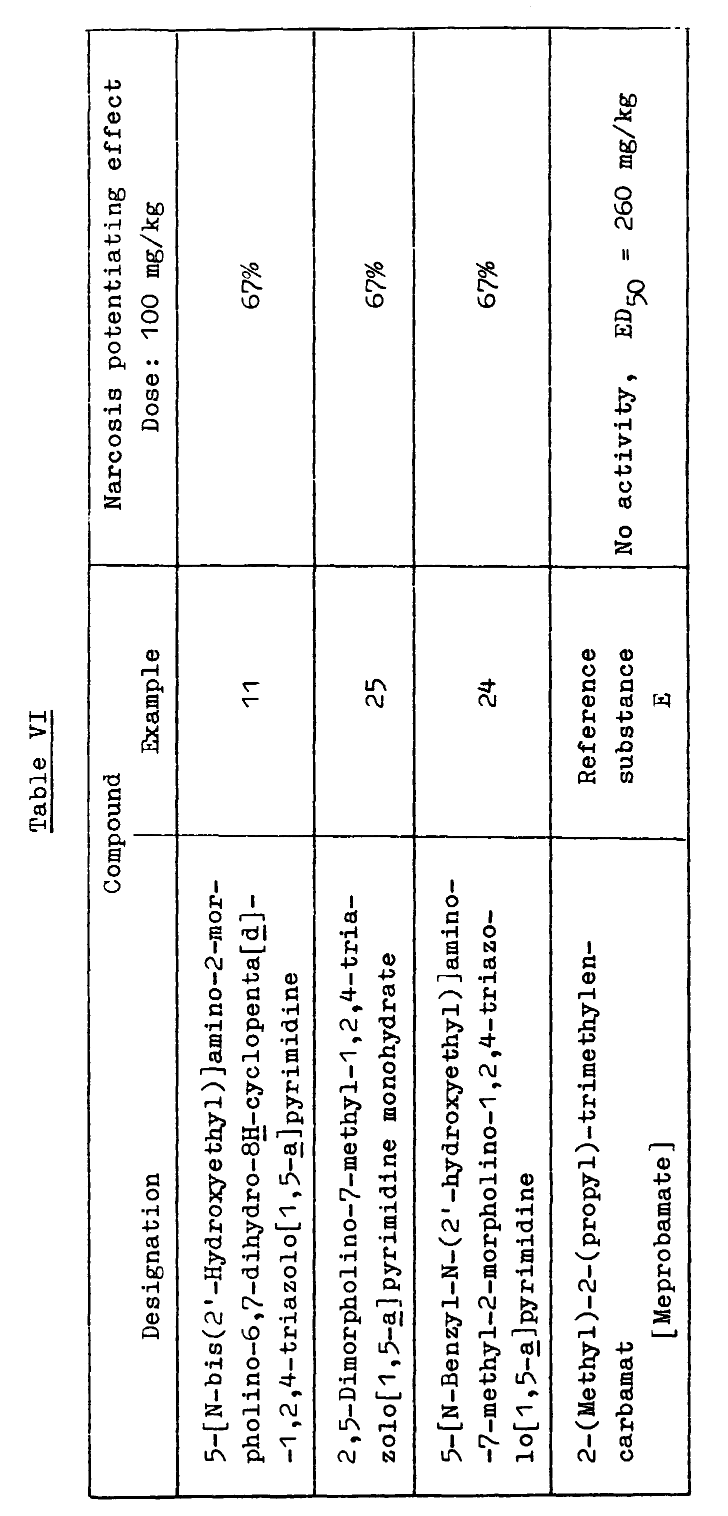

From the data of the above Table VI it can be established that the activity of the tested 1,2,4-triazolo[1,5-a] pyrimidine derivatives according to the invention surpasses that of the said reference Substance (E).

### 7. Spasmolytic activity on mice

The tests were carried out according to the modified method of Benzinger and Hane (Arch. Int. Pharmacodyn. 167, 245 to 249 [1959]. Mice of both sexes (NMRI breed, body weight 20 to 25 g) were treated orally with the compounds to be tested. One hour after the administration 6,7,8,9-tetra-(hydro)-5H-tetrazolo[1,5-a]azepine {pentetrazole} was given intraperitoneally in a dosis of 125 mg/kg. The tonic-extensoric spasms on the hind limbs were registered. As reference compound 3,5,5-tri-(methyl)-oxazolidine-2,4-dione [trimetadione] {reference substance F} was used. The results are set forth in the following Table VII.

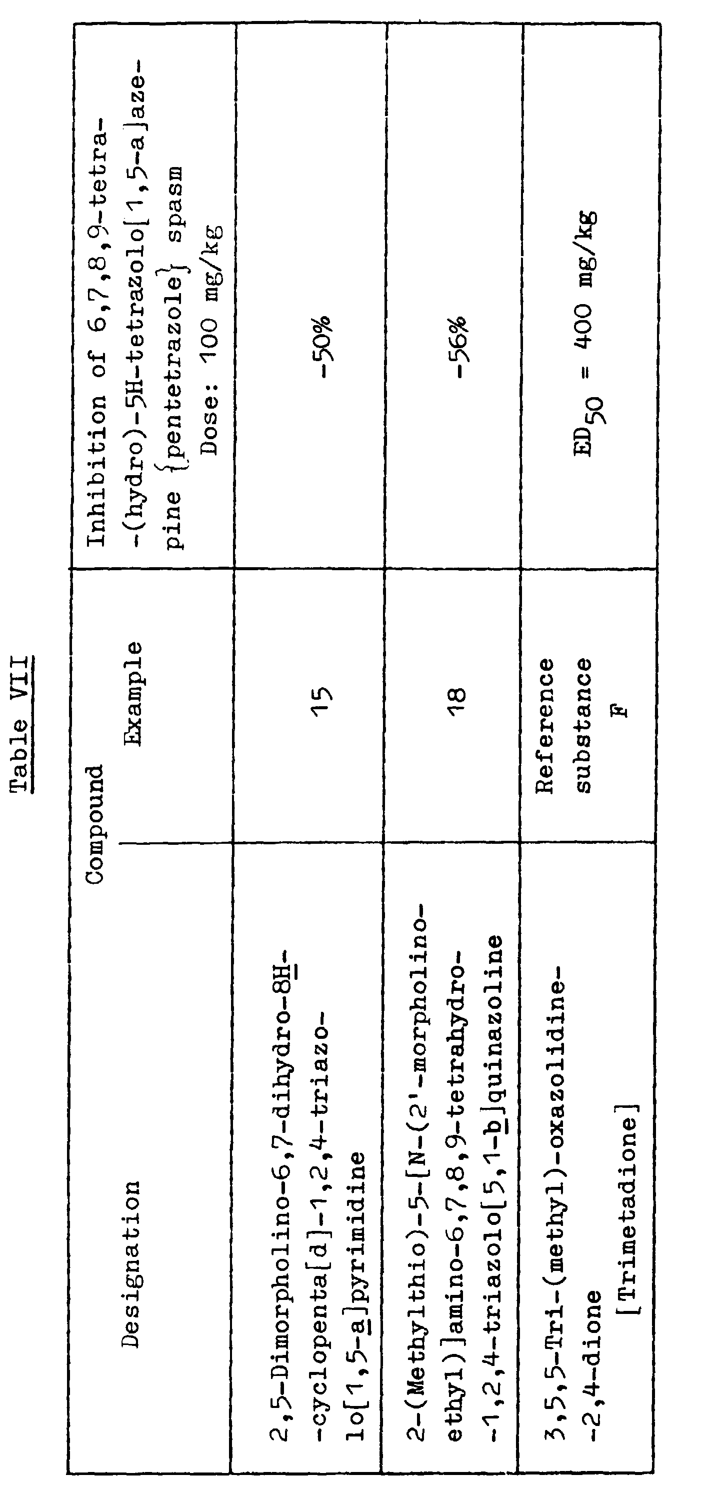

From the data of the above Table VII it can be established that the activity of the tested 1,2,4-triazolo[1,5-a] pyrimidine derivatives according to the invention surpasses that of the said reference compound (F).

### 8. Gastric acid secretion test

The tests were carried out according to the method of Shay (Shay, H. et al.: Gastroenterology 5, 45 [1945].) Rats of both sexes (Wistar breed, weighing 180 to 240 g) were fastened for 48 hours. On the day of the test the pylorus of the animals was bound under ether narcosis. The test compounds were administered orally 3 hours before the operation. 4 hours after the operation the animals were anaesthetized and the stomach was removed, the content thereof was centrifuged and the amount of the free acid was determined by titration, in the presence of Töpfer reagent. The inhibition in relation to the control is calculated in percentage. The results are shown in the following Table VIII.

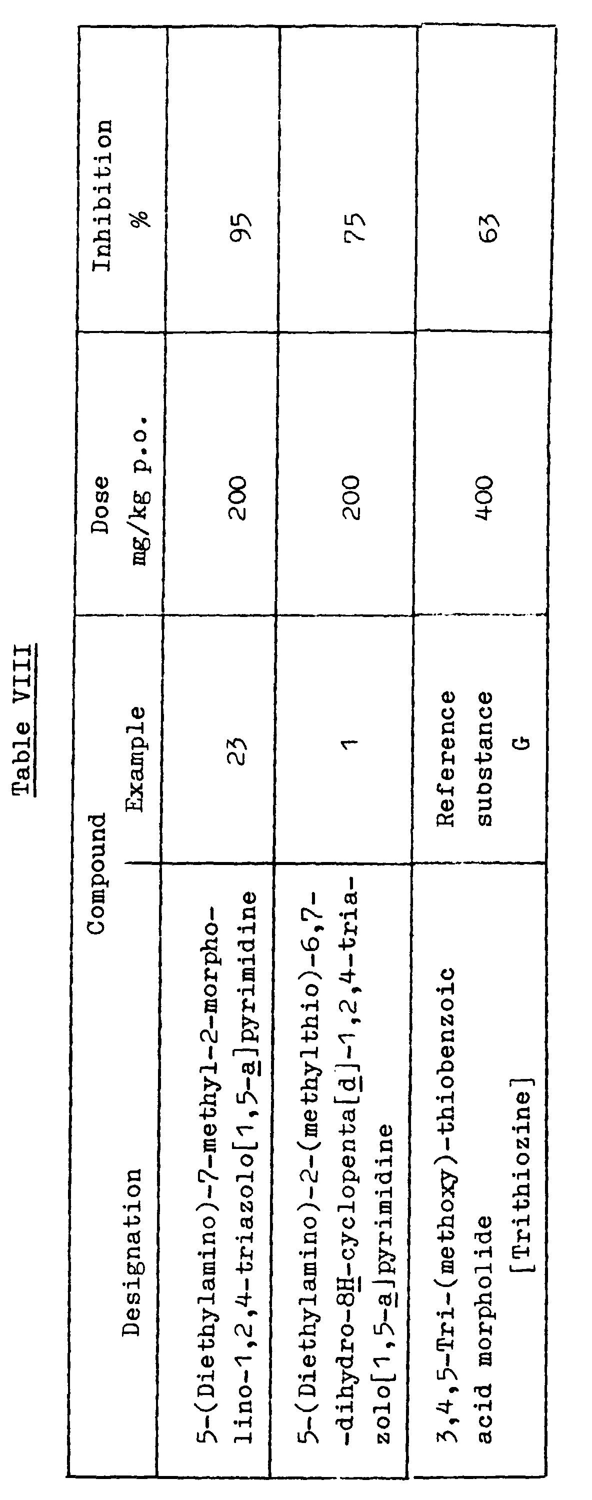

From the data of the above Table VIII it can be established that the tested 1,2,4-triazolo[1,5-a]pyrimidine derivatives according to the invention are more effective than the said reference substance (G).

The invention is further illustrated by the following Examples.

### Example 1

### 5-(Diethylamino)-2-(methylthio)-6,7-dihydro-8H-cyclopenta[d]-1,2,4-triazolo[1,5-a]pyrimidine

4.81 g (0.02 mole) of 5-chloro-2-(methylthio)-6,7-dihydro-8H-cyclopenta[d]-1,2,4-triazolo[1,5-a]pyrimidine are suspended in 30 ml of 2-propanol, 2.93 g (0.04 mole) of diethylamine are added to the suspension and the reaction mixture is boiled for 1.5 hours. The solution thus obtained is evaporated to dryness, the crystalline residue is suspended in 40 ml of water, filtered, washed with water, dried and recrystallized from an aqueous methanol solution (ratio of methanol : water = 1 : 1 [ml/ml]).
Yield: 5.32 g (96 %)
M.p.: 119-120.5 °C

According to the method of Example 1 the following derivatives are prepared by reacting an appropriate 1,2,4-triazolo[1,5-a]pyrimidine derivative of the general Formula II with an appropriate amine of the general Formula III:

### Example 2

### 5-[N-(3'-Hydroxypropyl)]amino-2-(methylthio)-6,7-dihydro-8H-cyclopenta[d]-1,2,4-triazolo[1,5-a]pyrimidine

Yield: 5.25 g (94 %)
M.p.: 170-171.5 °C (recrystallized from acetonitrile).

### Example 3

### 5-(Allylamino)-2-(methylthio)-6,7-dihydro-8H-cyclopenta[d]-1,2,4-triazolo[1,5-a]pyrimidine

Yield: 5.07 g (97 %)
M.p.: 166-167.5 °C (recrystallized from acetonitrile),

### Example 4

### 5-(Diallylamino)-2-(methylthio)-6,7-dihydro-8H-cyclopenta[d]-1,2,4-triazolo[1,5-a]pyrimidine

Yield: 5.55 g (92 %)
M.p.: 110-111.5 °C (recrystallized from acetonitrile).

### Example 5

### 5-(Cyclohexylamino)-2-(methylthio)-6,7-dihydro-8H-cyclopenta[d]-1,2,4-triazolo[1,5-a]pyrimidine

Yield: 5.97 g (98 %)
M.p.: 114-116 °C (recrystallized from acetonitrile),

### Example 6

### 5-(Benzylamino)-2-(methylthio)-6,7-dihydro-8H-cyclopenta[d]-1,2,4-triazolo[1,5-a]pyrimidine

Yield: 5.95 g (95.5 %)
M.p: 158-159.5 °C (recrystallized from acetonitrile).

### Example 7

### 5-(4'-Chlorobenzylamino)-2-(methylthio)-6,7-dihydro-8H-cyclopenta[d]-1,2,4-triazolo[1,5-a]pyrimidine

Yield: 6.71 g (97 %)
M.p: 162-164 °C (recrystallized from acetonitrile).

### Example 8

### 2-(Methylthio)-3-morpholino-6,7-dihydro-8H-cyclopenta[d]-1,2,4-triazolo[1,5-a]pyrimidine

Yield: 5.71 g (98 %)
M.p.: 162-163.5 °C (recrystallized from an aqueous methanol solution [ratio of methanol : water = 1 : 1 {ml/ml}]).

### Example 9

### 5-[4'-(2"-Hydroxyethyl)piperazino]-2-(methylthio)-6,7-dihydro-8H-cyclopenta[d]-1,2,4-triazolo[1,5-a] pyrimidine

Yield: 4.48 g (67 %)
M.p.. 142-144 °C (recrystallized from methanol).

### Example 10

### 2-(Methylthio)-5-[N-(2'-morpholinoethyl)]amino-6,7-dihydro-8H-cyclopenta[d]-1,2,4-triazolo[1,5-a] pyrimidine

Yield: 6.55 g (98 %)
M.p.: 210-212 °C (recrystallized from methanol).

### Example 11

### 5-[N-bis(2'-Hydroxyethyl)]amino-2-morpholino-6,7-dihydro-8H-cyclopenta[d]-1,2,4-triazolo[1,5-a] pyrimidine

Yield: 5.5 g (80 %)
M.p.: 167-169 °C (recrystallized from an aqueous methanol solution).

### Example 12

### 5-[N-(2'-Hydroxyethyl)]amino-2-morpholino-6,7-dihydro-8H-cyclopenta[d]-1,2,4-triazolo[1,5-a] pyrimidine

Yield: 4.93 g (81 %)
M.p.: 216-217.5 °C (recrystallized from methanol).

### Example 13

### 5-(Allylamino)-2-morpholino-6,7-dihydro-8H-cyclopenta[d]-1,2,4-triazolo[1,5-a]pyrimidine

Yield: 5.71 g (95 %)
M.p.: 165.5-167 °C (recrystallized from acetonitrile).

### Example 14

### 5-(Diallylamino)-2-morpholino-6,7-dihydro-8H-cyclopenta[d]-1,2,4-triazolo[1,5-a]pyrimidine

Yield: 6.60 g (97 %)
M.p.: 117-120 °C (recrystallized from cyclohexane).

### Example 15

### 2,5-Dimorpholino-6,7-dihydro-8H-cyclopenta[d]-1,2,4-triazolo[1,5-a]pyrimidine

Yield: 6.08 g (92 %)
M.p.: 211-213 °C (recrystallized from an aqueous isopropanol solution [ratio of isopropanol : water = 1 : 1 {ml/ml}]).

### Example 16

### 2-Morpholino-5-piperidino-6,7-dihydro-8H-cyclopenta[d]-1,2,4-triazolo[1,5-a]pyrimidine

Yield: 6.30 g (96 %)
M.p.: 207-208.5 °C (recrystallized from an aqueous methanol solution [ratio of methanol : water = 7 : 1 {ml/ml}]).

### Example 17

### 2-Morpholino-5-[N-(3'-morpholinopropyl)]amino-6,7-dihydro-8H-cyclopenta[d]-1,2,4-triazolo[1,5-a] pyrimidine

Yield: 5.58 g (72 %)
M.p.: 167-169 °C (recrystallized from methanol).

### Example 18

### 2-(Methylthio)-5-[N-(2'-morpholinoethyl)]amino-6,7,8,9--tetrahydro-1,2,4-triazolo[5,1-b]quinazoline

Yield: 5.85 g (85 %)
M.p.: 162-163 °C (recrystallized from isopropanol).

### Example 19

### 2-(Methylthio)-5-[N-(3'-morpholinopropyl)]amino-6,7,8,9-tetrahydro-1,2,4-triazolo[5,1-b]quinazoline

Yield: 6.13 g (84.5 %)
M.p.: 163-164 °C (recrystallized from methanol).

### Example 20

### 3-(4'-Methylpiperazino)-2-(methylthio)-6,7,8,9-tetrahydro-1,2,4-triazolo[5,1-b]quinazoline

Yield: 5.80 g (91 %)
M.p.: 174-175 °C (recrystallized from isopropanol).

### Example 21

### 5-(Benzylamino)-2-(morpholino)-6,7,8,9-tetrahydro-1,2,4-triazolo[5,1-b]quinazoline

Yield: 5.32 g (73 %)
M.p.: 191-193 °C (recrystallized from acetonitrile).

### Example 22

### 5-[N-(3'-Dimethylaminopropyl)]amino-2-morpholino-6,7,8,9-tetrahydro-1,2,4-triazolo[5,1-b]quinazoline

Yield: 4.39 g (61 %)
M.p.: 182-184 °C (recrystallized from an aqueous methanol solution [ratio of methanol : water = 1 : 3 {ml/ml}]).

### Example 23

### 5-(Diethylamino)-7-methyl-2-morpholino-1,2,4-triazolo[1,5-a]pyrimidine

Yield: 4.50 g (77.5 %)
M.p.: 127-129 °C (recrystallized from ethyl acetate).

### Example 24

### 5-[N-Benzyl-N-(2'-hydroxyethyl)]amino-7-methyl-2-morpholino-1,2,4-triazolo[1,5-a]pyrimidine

Yield: 5.34 g (72.5 %)
M.p.: 159-160.5 °C (recrystallized from methanol).

### Example 25

### 2,3-Dimorpholino-7-methyl-1,2,4-triazolo[1,5-a]pyrimidine monohydrate

Yield: 6.32 g (98 %)
M.p.: 188-190 °C (recrystallized from an aqueous isopropanol solution [ratio of isopropanol : water = 1 : 1 {ml/ml}]).

### Example 26

### 5-(Diethylamino)-2-(dimethylamino)-7,8-dihydro--9H-thiopyrano[3,2-d]-1,2,4-triazolo[1,5-a]pyrimidine

Yield: 5.43 g (88.5 %)
M.p.: 124-126 °C (recrystallized from ethyl acetate).

### Example 27

### 5-[N-(2'-Hydroxyethyl)]amino-2-(methylthio)-6,7-dihydro-8H-cyclopenta[d]-1,2,4-triazolo[1,5-a] pyrimidine

To a suspension of 4.81 g (0.02 mole) of 5-chloro-2-(methylthio)-6,7-dihydro-8H-cyclopenta[d]-1,2,4-triazolo[1,5-a]pyrimidine in 20 ml of 2-propanol 2.44 g (0.04 mole) of 2-aminoethanol are added and the reaction mixture is boiled for 1.5 hours. Then it is cooled, the cold suspension is diluted with water, stirred for a short time, finally the separated crystals are filtered and washed with water.
Yield: 5.04 g (95 %)
M.p.: 191-192 °C (recrystallized from methanol).

On proceeding according to the method of Example 27 the following derivatives are prepared by reacting an appropriate 1,2,4-triazolo[1,5-a]pyrimidine derivative of the general Formula II with an appropriate amine of the general Formula III:

### Example 28

### 5-(Diethylamino)-2-(methylthio)-6,7,8,9-tetrahydro-1,2,4-triazolo[5,1-b]quinazoline

Yield: 5.13 g (88 %)
M.p.: 121-122 °C (recrystallized from ethyl acetate).

### Example 29

### 2-(1'-Methylethylthio)-5-[N-(2"-morpholino-ethyl)]amino-6,7,8,9-tetrahydro-1,2,4-triazolo[5,1-b] quinazoline

Yield: 7.08 g (94 %)
M.p.: 190-191 °C (recrystallized from acetonitrile).

### Example 30

### 2-(1'-Methylethylthio)-5-[N-(3"-morpholinopropyl)]amino-6,7,8,9-tetrahydro-1,2,4-triazolo[5,1-b] quinazoline

Yield: 6.68 g (85.5 %)
M.p.: 140-141 °C (recrystallized from ethyl acetate).

### Example 31

### 2-(Ethylthio)-5-[N-(2"-morpholinoethyl)]amino-6,7,8,9-tetrahydro-1,2,4-triazolo[5,1-b] quinazoline

Yield: 6.31 g (87 %)
M.p.: 156-158 °C (recrystallized from acetonitrile).

### Example 32

### 2-(Ethylthio)-5-[N-(3"-morpholinopropyl)]amino-6,7,8,9-tetrahydro-1,2,4-triazolo[5,1-b] quinazoline

Yield: 6.78 g (90 %)
M.p.: 154-155 °C (recrystallized from ethyl acetate).

### Example 33

### 5-(Diethylamino)-7-methyl-2-morpholino-1,2,4-triazolo[1,5-a]pyrimidine

Yield: 4.36 g (75 %)
M.p.: 127-128 °C (recrystallized from ethyl acetate)

### Example 34

### 2-(Methylthio)-5-[N-(3'-morpholinopropyl)]amino-6,7,8,9-tetrahydro-1,2,4-triazolo[5,1-b] quinazoline

Yield: 6.24 g (86 %)
M.p.: 163.5-165 °C (recrystallized from isopropanol).

### Example 35

### 2-(Methylthio)-5-[N-(2'-morpholinoethyl)]amino-6,7,8,9,10,11,12,13,14,15-decahydro-cyclododeca[d] -1,2,4-triazolo[1,5-a]pyrimidine

To a suspension of 3.39 g (0.01 mole) of 5-chloro-2-(methylthio)-6,7,8,9,10,11,12,13,14,15-decahydro-cyclododeca[d]-1,2,4-triazolo[1,5-a]pyrimidine in 10.0 ml of 2-propanol 1.11 g (0.011 mole) of triethylamine and 1.43 g (0.011 mole) of 2-aminoethylmorpholine are successively added, and the reaction mixture is boiled for 2 hours. The 2-propanol is then removed in vacuo, the residue is suspended in water, then the crystals are filtered and washed with water.
Yield: 3.80 g (87.5 %)
M.p.: 134-136 °C (recrystallized from ethyl acetate).

On proceeding according to the method of Example 35 the following derivatives are prepared by reacting the appropriate 1,2,4-triazolo[1,5-a]pyrimidine derivative of the general Formula II with the appropriate amine of the general Formula III:

### Example 36

### 2-Morpholino-5-[N-(3'-morpholinopropyl)]amino-6,7,8,9-tetrahydro-1,2,4-triazolo[5,1-b]quinazoline

Yield: 3.81 g (95 %)
M.p.: 163-165 °C (recrystallized from acetonitrile).

### Example 37

### 2-(Methylthio)-5-[N-(2'-morpholinoethyl)]amino-6,7,8,9-tetrahydro-10H-cyclohepta[d]-1,2,4-triazolo [1,5-a]pyrimidine

4.84 g (0.018 mole) of 5-chloro-2-(methylthio)-6,7,8,9-tetrahydro-10H-cyclohepta[d]-1,2,4-triazolo[1,5-a]pyrimidine are suspended in 15 ml of 2-propanol, 2.02 g (0.02 mole) of triethylamine and 2.60 g (0.02 mole) of 2-aminoethylmorpholine are successively added to the suspension and it is boiled for 1 hour. The cold suspension is diluted with water, the separated crystals are filtered and washed with water.
Yield: 6.28 g (96 %)
M.p.: 173-174 °C (recrystallized from acetonitrile)

On proceeding according to the above Example the following derivatives are prepared by reacting an appropriate 1,2,4-triazolo[1,5-a]pyrimidine derivative of the general Formula II with an appropriate amine of the general Formula III:

### Example 38

### 2-(Methylthio)-5-[N-(3'-morpholinopropyl)]amino-6,7,8,9-tetrahydro-10H-cyclohepta[d]-1,2,4-triazolo [1,5-a]pyrimidine

Yield: 6.47 g (95 %)
M.p.: 150.5-151 °C (recrystallized from acetonitrile).

### Example 39

### 2-(Methylthio)-5-[N-(2'-morpholinoethyl)]amino-7,8-dihydro-9H-thiopyrano[3,2-d]-1,2,4-triazolo [1,5-a]pyrimidine

Yield: 5.48 g (83 %)
M.p.: 123-124.5 °C (recrystallized from acetonitrile).

### Example 40

### 2-(Methylthio)-5-[N-(2'-piperidinoethyl)]amino-6,7,8,9-tetrahydro-1,2,4-triazolo[5,1-b]quinazoline

Yield: 5.68 g (91 %)
M.p.: 140-141 °C (recrystallized from isopropanol).

### Example 41

### 2-(Methylthio)-5-[N-(2'-pyrrolidinoethyl)]amino--6,7,8,9-tetrahydro-1,2,4-triazolo[5,1-b]quinazoline

Yield: 5.51 g (92 %)
M.p.: 130-132 °C (recrystallized from ethyl acetate).

### Example 42

### 5-(4'-Hydroxybutylamino)-2-(methylthio)-6,7-dihydro--8H-cyclopenta[d]-1,2,4-triazolo[1,5-a]pyrimidine

Yield: 3.67 g (69.5 %)
M.p.: 140-142 °C (recrystallized from an aqueous acetone solution [ratio of acetone : water = 5 : 1 {ml/ml}]).

### Example 43

### 7-Benzyl-2-(methylthio)-5-[N-(3'-morpholinopropyl)]amino-6,7,8,9-tetrahydropyrido[4,3-d]-1,2,4-triazolo [1,5-a]pyrimidine

To a suspension of 2.42 g (0.007 mole) of 7-benzyl-5-chloro-2-(methylthio)-6,7,8,9-tetrahydropyrido(4,3-d]-1,2,4-triazolo[1,5-a]pyrimidine in l0 ml of 2-propanol 2.02 g (0.014 mole) of 3-aminopropylmorpholine are added and the reaction mixture is boiled for 1 hour. Then it is cooled, the separated crystals are filtered and washed with 2-propanol and water.
Yield: 3.00 g (94.5 %)
M.p.: 166-168 °C (decomp.) (recrystallized from acetonitrile).

According to the method of Example 43 the following derivatives are prepared by reacting an appropriate 1,2,4-triazolo[1,5-a]pyrimidine derivative of the general Formula II with an appropriate amine of the general Formula III:

### Example 44

### 2-(Methylthio)-5-morpholino-6,7,8,9-tetrahydro-1,2,4-triazolo[5,1-b]quinazoline

Yield: 2.08 g (97 %)
M.p.: 185-186 °C (recrystallized from acetonitrile).

### Example 45

### 2-(Methylthio)-5-piperidino-6,7,8,9-tetrahydro-1,2,4-triazolo[5,1-b]quinazoline

Yield: 2.08 g (98 %)
M.p.: 187-188 °C (recrystallized from acetonitrile).

### Example 46

### 8-Benzyl-2-(methylthio)-5-[N-(2'-morpholinoethyl)]-amino-6,7,8,9-tetrahydropyrido[3,4-d]-1,2,4-triazolo [1,5-a]pyrimidine

To a suspension of 4.17 g (0.012 mole) of 8-benzyl-5-chloro-2-(methylthio)-6,7,8,9-tetrahydropyrido[3,4-d]-1,2,4-triazolo[1,5-a]pyrimidine in 12 ml of 2-propanol 1.215 g (0.012 mole) of triethylamine and 1.56 g (0.012 mole) of 2--aminoethylmorpholine are successively added, and the reaction mixture is boiled for 1.5 hours. The separated crystals are filtered and washed with cold 2-propanol and water.
Yield: 4.64 g (88 %)
M.p.: 185-188 °C (decomp.) (recrystallized from acetonitrile).

On proceeding according to the method of Example 46 the following derivatives are prepared by reacting an appropriate 1,2,4-triazolo[1,5-a]pyrimidine derivative of the general Formula II with an appropriate amine of the general Formula III:

### Example 47

### 7-Benzyl-2-(methylthio)-5-[N-(2'-morpholinoethyl)]amino-6,7,8,9-tetrahydropyrido[4,3-d]-1,2,4-triazolo [1,5-a]pyrimidine

Yield: 4.17 g (79 %)
M.p.: 151-153 °C (decomp.) (recrystallized from acetonitrile).

### Example 48

### 5-(1'-Adamantylamino)-2-(methylthio)-6,7-dihydro-8H-cyclopenta[d]-1,2,4-triazolo[1,5-a]pyrimidine

Yield: 3.41 g (80 %)
M.p.: 205-206.5 °C (recrystallized from acetonitrile).

### Example 49

### 5-{N-[2'-(1"H-Indol-3"-yl)ethyl]amino}-2-(methylthio)-6,7-dihydro-8H-cyclopenta[d]-1,2,4-triazolo[1,5-a] pyrimidine

Yield: 4.15 g (95 %)
M.p.: 245.5-247.5 °C (recrystallized from dimethylformamide).

### Example 50

### 2-(Methylthio)-5-(4'-methoxybenzylamino)-6,7-dihydro--8H-cyclopenta[d]-1,2,4-triazolo[1,5-a]pyrimidine

To a suspension of 5.05 g (0.021 mole) of 5-chloro-2-(methylthio)-6,7-dihydro-8H-cyclopenta[d]-1,2,4-triazolo[1,5-a]pyrimidine in 30 ml of 2-propanol 6.04 g (0.044 mole) of 4-methoxybenzylamine are added, and the reaction mixture is boiled for 1.5 hours. Then the solvent is removed in vacuo, the residue is taken up in chloroform and washed twice with water. The chloroform phase is dried over anhydrous Na₂SO₄ and evaporated in vacuo to constant weight. The oily product is suspended in diethyl ether, then the crystalline product thus obtained is filtered off and washed with diethyl ether.
Yield: 6.35 g (88.5 %)
M.p.: 130.5-132 °C (recrystallized from acetonitrile).

### Example 51

### 7-Benzyl-5-(diethylamino)-2-(methylthio)-6,7,8,9-tetrahydropyrido[4,3-d]-1,2,4-triazolo[1,5-a] pyrimidine

0.69 g (0.002 mole) of 7-benzyl-5-chloro-2-(methylthio)-6,7,8,9-tetrahydropyrido[4,3-d]-1,2,4-triazolo[1,5-a]pyrimidine is suspended in 3.0 ml of 2-propanol. 0.44 g (0.006 mole) of diethylamine are added to the suspension and the reaction mixture is boiled for half an hour. The solution thus obtained is evaporated to dryness in vacuo, the residue is dissolved in chloroform and the solution is extracted twice with ether. The chloroform phase is dried over anhydrous Na₂SO₄, evaporated to constant weight in vacuo, the oily product is suspended in ethyl acetate, filtered and washed with cold ethyl acetate.
Yield: 0.53 g (69 %)
M.p.: 111-113 °C (recrystallized from ethyl acetate).

### Example 52

### 2-(Dimethylamino)-5-[N-(2'-morpholinoethyl)]amino-7,8-dihydro-9H-thiopyrano[3,2-d]-1,2,4-triazolo [1,5-a]pyrimidine

To a suspension of 5.40 g (0.02 mole) of 2-(dimethylamino)-5-chloro-7,8-dihydro-9H-thiopyrano[3,2-d]-1,2,4-triazolo[1,5-a]pyrimidine in 20 ml of 2-propanol 2.125 g (0.021 mole) of triethylamine and 2.73 g (0.021 mole) of 2-aminoethylmorpholine are successively added, and the reaction mixture is boiled for one hour. Upon cooling triethylamine hydrochloride gets separated, which is filtered and washed with 2-propanol. The filtrate is evaporated to dryness in vacuo and the residue is dissolved in chloroform. The solution thus obtained is extracted twice with water, dried over anhydrous Na₂SO₄ and evaporated in vacuo. The oily product is suspended in ethyl acetate, the crystalline product thus obtained is filtered off and washed with ethyl acetate.
Yield: 5.42 g (74.5 %)
M.p.: 110-113 °C (recrystallized from ethyl acetate),

On proceeding according to the method of Example 52 the following derivative are prepared by reacting the appropriate 1,2,4-triazolo[1,5-a]pyrimidine derivative of the general Formula II with the appropriate amine of the general Formula III:

### Example 53

### 2-(Dimethylamino)-5-[N-(3'-morpholinopropyl)]amino-7,8-dihydro-9H-thiopyrano[3,2-d]-1,2,4-triazolo[1,5-a] pyrimidine

Yield: 5.35 g (71 %)
M.p.: 86-89 °C (recrystallized from ethyl acetate).

### Example 54

### 5-(4'-Benzylpiperazino)-2-(methylthio)-6,7-dihydro--8H-cyclopenta[d]-1,2,4-triazolo[1,5-a]pyrimidine

9.62 g (0.04 mole) of 5-chloro-2-(methylthio)-6,7-di-hydro-8H-cyclopenta[d]-1,2,4-triazolo[1,5-a]pyrimidine are suspended in 160 ml of 2-propanol. Then 14.17 g (0.14 mole) of triethylamine and 11.47 g (0.046 mole) of N-benzylpiperazine dihydrochloride are successively added to the suspension and it is stirred on a water bath of 30 °C for 8 hours. The separated crystals are filtered and washed with 2-propanol and plenty of water.
Yield: 13.65 g (90 %)
M.p.: 151-153 °C (recrystallized from acetone).

### Example 55

### 5-[N-(3'-Hydroxypropyl)]amino-2-morpholino-6,7-dihydro--8H-cyclopenta[d]-1,2,4-triazolo[1,5-a]pyrimidine

To a suspension of 5.59 g (0.02 mole) of 5-chloro-2-morpholino-6,7-dihydro-8H-cyclopenta[d]-1,2,4-triazolo-[1,5-a]pyrimidine in 40 ml of isopropanol 3.15 g (0.042 mole) of 3-amino-1-propanol are added, and the mixture is boiled for 1 hour. The solvent is removed in vacuo, the residue is dissolved in chloroform, treated with a slight amount of silica gel, filtered and evaporated to dryness in vacuo.
Yield: 5.69 g (89.5 %)
M.p.: 185-187 °C (recrystallized from acetonitrile).

### Example 56

### 5-(tert.-Butylamino)-2-(methylthio)-6,7-dihydro-8H-cyclopenta[d]-1,2,4-triazolo[1,5-a]pyrimidine

To a suspension of 1.20 g (0.005 mole) of 5-chloro-2-(methylthio)-6,7-dihydro-8H-cyclopenta[d]-1,2,4-triazolo[1,5-a]pyrimidine in 10 ml of 2-propanol 3.08 g (0.042 mole) of tert.-butylamine are added and the mixture is stirred on an oil bath of 50 °C for 10 hours. The solution thus obtained is evaporated in vacuo, the residue is suspended in water, filtered and washed with water.
Yield: 1.20 g (86 %)
M.p.: 121-122.5 °C (recrystallized from ethyl acetate).

### Example 57

### 5-(tert.-Butylamino)-2-(methylthio)-6,7-dihydro-8H-cyclopenta[d]-1,2,4-triazolo[1,5-a]pyrimidine

6.02 g (0.025 mole) of 5-chloro-2-(methylthio)-6,7-dihydro-8H-cyclopenta[d]-1,2,4-triazolo[1,5-a]pyrimidine are suspended in 20 ml of methanol. 7.31 g (0.1 mole) of tert.-butylamine are added to the suspension and the reaction mixture is stirred at 25 °C for 10 hours. The soluton is evaporated to dryness in vacuo and the residue is suspended in water, then the separated crystals are filtered and washed with water.
Yield: 2.76 g (40 %)
M.p.: 120-121.5 °C (recrystallized from a mixture of ethyl acetate and cyclohexane in a ratio of 1 : 1 [ml/ml]).

### Example 58

### (2-Morpholino-6,7-dihydro-8H-cyclopenta[d]-1,2,4-triazolo[1,5-a]pyrimidin-5-yl)thioglycolic acid ethylester

To a suspension of 9.80 g (0.035 mole) of 5-chloro-2-morpholino-6,7-dihydro-8H-cyclopenta[d]-1,2,4-triazolo[1,5-a]pyrimidine in 70 ml of ethanol 3.90 g (0.0385 mole) of triethylamine and 4.21 g (0.035 mole) of ethyl thioglycolate are subsequently added, and the suspension thickening in a short time is stirred at 30 °C for 2.5 hours. The separated crystals are filtered and washed with an aqueous ethanol solution.
Yield: 11.35 g (89 %)
M.p.: 123.5-125.5 °C (recrystallized from an aqueous ethanol solution [ratio of ethanol : water = 3 : 5 {ml/ml}]).

### Example 59

### (2-Methylthio-6,7-dihydro-8H-cyclopenta[d]-1,2,4-triazolo[1,5-a]pyrimidin-5-yl)thioglycolic acid ethylester

One proceeds according to Example 58 except that the appropriate 1,2,4-triazolo[1,5-a]pyrimidine derivative is used as starting substance and the reaction mixture is stirred at 50°C for 1 hour.
Yield: 9.54 g (84 %)
M.p.: 108-109 °C (recrystallized from ethanol).

### Example 60

### Ethyl N-[2-methylthio-6,7-dihydro-8H-cyclopenta[d]-1,2,4-triazolo[1,5-a]pyrimidin-5-yl]glycinate

17.30 g (0.071 mole) of 5-chloro-2-(methylthio)-6,7-dihydro-8H-cyclopenta[d]-1,2,4-triazolo[1,5-a]pyrimidine and 14.0 g (0.1 mole) of glycine ethyl ester hydrochloride are suspended in 80 ml of ethanol, then 18.20 g (0.18 mole) of triethylamine are dropped to it. The reaction mixture is stirred at a temperature of 30-35 °C for 6 hours. The thick suspension is diluted with 250 ml of water, then the separated white crystals are filtered and washed with water.
Yield: 21.67 g (98 %)
M.p.: 128-129 °C (recrystallized from an aqueous ethanol solution [ratio of ethanol : water = 1 : 1 {ml/ml}]).

### Example 61

### Ethyl N-[2-methylthio-6,7-dihydro-8H-cyclopenta[d]-1,2,4-triazolo[1,5-a]pyrimidin-5-yl]glycinate

One proceeds according to Example 60 except that 1.2 mole of glycine ethyl ester hydrochloride are used, calculated on 1 mole of chloro derivative, and the reaction mixture is stirred at 30-35 °C for 24 hours. The solvent is removed in vacuo, then the residue is suspended in water, filtered and washed with water.
Yield: 10.94 g (89 %)
M.p.: 128-129 °C (recrystallized from an aqueous ethanol solution [ratio of ethanol : water = 1 : 1 {ml/ml}]).

### Example 62

### Ethyl N-[2-morpholino-6,7-dihydro-8H-cyclopenta[d]-1,2,4-triazolo[1,5-a]pyrimidin-5-yl]glycinate

To a suspension of 9,80 g (0.035 mole) of 5-chloro-2-morpholino-6,7-dihydro-8H-cyclopenta[d]-1,2,4-triazolo[1,5-a]pyrimidine in 50 ml of ethanol 19.54 g (0.14 mole) of glycine ethyl ester hydrochloride are added. Then 17.71 g (0.175 mole) of triethylamine are dropped to it, and the mixture is stirred on a water bath of 40 °C for 20 hours. The solvent is removed in vacuo, the residual crystalline product is suspended in water, filtered and washed with water. The residue is dissolved in a slight amount of chloroform containing 1 % by volume of methanol, treated with a slight amount of silica gel, filtered and evaporated to dryness in vacuo. Thus ethyl N-[2-morpholino-6,7-dihydro-8H-cyclopenta[d]-1,2,4-triazolo[1,5-a]pyrimidin-5-yl]glycinate dihydrate is obtained (m.p.: 78-82 °C), which is dried in vacuo at a temperature of 60 °C to get the corresponding ethyl ester.
Yield: 7.20 g (61.5 %)
M.p.: 145-147 °C (recrystallized from an aqueous ethanol solution [ratio of ethanol : water = 1 : 1 {ml/ml}]).

### Example 63

### 2-(Methylthio)-5-morpholino-6,7,8,9-tetrahydro-1,2,4-triazolo[5,1-b]quinazoline

0.51 g (0.0017 mole) of 5-bromo-2-(methylthio)-6,7,8,9-tetrahydro-1,2,4-triazolo(5,1-b]quinazoline is suspended in 2 ml of 2-propanol, 0.30 g (0.0034 mole) of morpholine is added and the reaction mixture is boiled for half an hour. Then the suspension is cooled and the separated crystals are washed successively with 2-propanol, water and 2-propanol again.
Yield: 0.49 g (94 %)
M.p.: 182-184 °C (recrystallized from acetonitrile).

### Example 64

### 2-(Methylthio)-5-[N-(2'-morpholinoethyl)]amino-6,7,8,9-tetrahydro-1,2,4-triazolo[5,1-b]quinazoline

9.45 g (0.04 mole) of 2-(methylthio)-6,7,8,9-tetrahydro-1,2,4-triazolo[5,1-b]quinazolin-5(10H)-one are suspended in 16.14 g (0.1 mole) of hexamethyldisilazane, 13.02 g (0.1 mole) of 2-aminoethylmorpholine and 0.76 g (0.004 mole) of p-toluenesulfonic acid monohydrate are successively added to the suspension and the reaction mixture is stirred for 6 hours on a water bath of 170-175 °C while the hexamethyldisiloxane being formed as a side-product is distilled off. The brown solution is cooled to 90 °C, the thick suspension is diluted with 2-propanol and allowed to cool to room temperature. The separated crystals are filtered and washed with 2-propanol.
Yield: 12.87 g (92 %)
M.p.: 164-165 °C (recrystallized from isopropanol).

### Example 65

### 2-[3'-(Dimethylamino)propylamino]-5-[N-(2'-morpholinoethyl)]amino-6,7,8,9-tetrahydro-1,2,4-triazolo[5,1-b] quinazoline

One proceeds according to Example 64 except that the reaction is carried out at a temperature between 150 and 155 °C.
Yield: 9.49 g (78.5 %)
M.p.: 174-176 °C (recrystallized from acetonitrile).

### Example 66

### 2-[3'-(Dimethylamino)propylamino]-5-[N-(3"-morpholinopropyl)]amino-6,7,8,9-tetrahydro-1,2,4-triazolo[5,1-b] quinazoline

One proceeds according to Example 64 except that the reaction is carried out at a temperature between 150 and 155 °C.
Yield: 7.70 g (61.5 %)
M.p.: 159-161 °C (recrystallized from acetonitrile).

### Example 67

### 2-(Methylsulfonyl)-5-[N-(2'-morpholinoethyl)[amino-6,7,8,9-tetrahydro-1,2,4-triazolo[5,1-b] quinazoline

To a suspension of 5.37 g (0.02 mole) of 2-(methylsulfonyl)-6,7,8,9-tetrahydro-1,2,4-triazolo[5,1-b]quinazoline-5(10H)-one in 16.14 g (0.1 mole) of hexamethyldisilazane 13.02 g (0.1 mole) of 2-aminoethylmorpholine and 0.38 g (0.002 mole) of p-toluenesulfonic acid monohydrate are added, and the reaction mixture is stirred on an oil bath of 170 to 175 °C for 16 hours. Upon cooling the brown solution separates into two phases. The upper phase is decanted, the lower thick oily phase is taken up in chloroform and extracted with water. The chloroform solution dried over anhydrous Na₂SO₄ is treated with silica gel, evaporated to dryness in vacuo and the residual brown oily product is recrystallized from 15 ml of 2-propanol.
Yield: 3.73 g (49 %)
M.p.: 162-164 °C (recrystallized from isopropanol).

### Example 68

### 2-(Methylsulfonyl)-5-[N-(2'-morpholinoethyl)]amino-6,7,8,9-tetrahydro-1,2,4-triazolo[5,1-b] quinazoline

0.57 g (0.002 mole) of 5-chloro-2-(methylsulfonyl)-6,7,8,9-tetrahydro-1,2,4-triazolo[5,1-b]quinazoline is suspended in 5 ml of 2-propanol. 0.22 g (0.0022 mole) of triethylamine and 0.29 g (0.0022 mole) of 2-aminoethylmorpholine are successively added to the suspension and it is boiled at 80 °C for half an hour. The solution thus obtained is evaporated to dryness, the residue is suspended in water, filtered and washed with 2-propanol.
Yield: 0.31 (40.5 %)
M.p.: 161-164 °C (recrystallized from isopropanol).

### Example 69

### {2-[2'-Methylthio-6',7'-dihydro-8'H-cyclopenta[d]-1,2,4-triazolo[1',5'-a]pyrimidin-5'-yl]amino}acetic acid morpholide

1.20 g (0.005 mole) of 5-chloro-(methylthio)-6,7-dihydro-8H-cyclopenta[d]-1,2,4-triazolo[1,5-a]pyrimidine are suspended in 5 ml of 2-propanol, 0.90 g (0.005 mole) of 4-glycyl-morpholine hydrochloride and 1.01 g (0.01 mole) of triethylamine are successively added to the suspension and it is boiled for 2 hours. The separated crystals are filtered and washed with 2-propanol and water.
Yield: 1.45 g (83 %)
M.p.: 232-233.5 °C (recrystallized from methanol).

### Example 70

### 2-Methylthio-5-morpholino-6,7,8,9-tetrahydro-1,2,4-triazolo[5,1-b]quinazoline

To a suspension of 0.51 g (0.002 mole) of 5-chloro-2-(methylthio)-6,7,8,9-tetrahydro-1,2,4-triazolo[5,1-b]quinazoline in 2 ml of acetonitrile 0.35 g (0.004 mole) of morpholine is added, and the reaction mixture is boiled for 1 hour. Then it is cooled, 2 ml of water are added, the separated crystals are filtered and washed successively with a slight amount of water and acetonitrile.
Yield: 0.50 g (82 %)
M.p.: 182-184.5 °C (recrystallized from acetone).

### Example 71

### 1-[2'-{2"-Methylthio-6",7"-dihydro-8"H-cyclopenta[d]-1",2",4"-triazolo[1",5"-a]pyrimidin-5"-yl}amino]acetyl-4-(2‴-hydroxyethyl)piperazine

One proceeds according to Example 70 using the appropriate amine of the general Formula III except that the crystals are not washed with water.
Yield: 1.37 g (70 %)
M.p.: 203-204.5 °C (recrystallized from acetonitril).

### Example 72

### 2-Methylthio-5-morpholino-6,7,8,9-tetrahydro-1,2,4-triazolo[5,1-b]quinazoline

One proceeds according to Example 70 except that instead of acetonitrile 2 ml of dimethylformamide are used as solvent.
Yield: 0.39 g (64 %)
M.p.: 181-184 °C (recrystallized from acetonitrile).

### Example 73

### 2-Methylthio-5-morpholino-6,7,8,9-tetrahydro-1,2,4-triazolo[5,1-b]quinazoline

One proceeds according to Example 70 except that instead of acetonitrile 2 ml of tetrahydrofuran are used as solvent and the reaction mixture is boiled for 3 hours instead of for 1 hour.
Yield: 0.42 g (69 %)
M.p.: 181-183 °C (recrystallized from acetonitrile).

### Example 74

### 2-Methylthio-5-morpholino-6,7,8,9-tetrahydro-1,2,4-triazolo[5,1-b]quinazoline

One proceeds according to Example 70 except that instead of acetonitrile 2 ml of ethylene glycol are used as solvent and the reaction mixture is boiled for 10 minutes instead of for 1 hour.
Yield: 0.58 g (95 %)
M.p.: 180-182 °C (recrystallized from acetonitrile).

### Example 75

### 2-Methylthio-5-morpholino-6,7,8,9-tetrahydro-1,2,4- triazolo[5,1-b]quinazoline

One proceeds according to Example 70 except that the reaction is carried out in 1 ml (1.00 g ≈ 0.0115 mole) of morpholine, and the reaction mixture is boiled for 2 hours instead of for 1 hour.
Yield: 0.50 g (82 %)
M.p.: 180-182.5 °C (recrystallized from acetonitrile).

### Example 76

### 2-Methylthio-5-morpholino-6,7,8,9-tetrahydro-1,2,4-triazolo[5,1-b]quinazoline

To a solution of 0.51 g (0.002 mole) of 5-chloro-2-methylthio-6,7,8,9-triazolo[5,1-b]quinazoline in 2 ml of benzene 0.35 g (0.004 mole) of morpholine is added, and the reaction mixture is boiled for 1.5 hours. Then the solvent is removed in vacuo, the crystalline residue is suspended in water, filtered and washed successively with a slight amount of water and acetonitrile.
Yield: 0.53 g (87 %)
M.p.: 182-184 °C (recrystallized from acetonitrile).

### Example 77

### 2-Methylthio-5-morpholino-6,7,8,9-tetrahydro-1,2,4-triazolo[5,1-b]quinazoline

One proceeds according to Example 76 except that instead of benzene 2 ml of chloroform are used as solvent and the reaction mixture is boiled for 2.5 hours instead of for 1.5 hours.
Yield: 0.55 g (90 %)
M.p.: 182-183.5 °C (recrystallized from acetonitrile).

### Example 78

### 2-Methylthio-5-morpholino-6,7,8,9-tetrahydro-1,2,4-triazolo[5,1-b]quinazoline

To a solution of 0.51 g (0.002 mole) of 5-chloro-2--methylthio-6,7,8,9-tetrahydro-1,2,4-triazolo(5,1-b]quinazoline in 2 ml of xylene 0.35 g (0.004 mole) of morpholine is added, and the reaction mixture is boiled for 1 hour. Then it is cooled and diluted with diethyl ether. The separated crystals are filtered and washed successively with diethyl ether, a slight amount of water and acetonitrile.
Yield: 0.51 g (83.5 %)
M.p.: 181-183.5 °C (recrystallized from acetonitrile).

### Example 79

### 2-Methylthio-3-morpholino-6,7,8,9-tetrahydro-1,2,4-triazolo[5,1-b]quinazoline

One proceeds according to Example 78 except that instead of xylene 2 ml of chlorobenzene are used as solvent.
Yield: 0.39 g (64 %)
M.p.: 181-183 °C (recrystallized from acetonitrile).

### Example 80

### 5-[N-(2'-Morpholinoethyl)]amino-2-diallylamino-6,7-dihydro-8H-cyclopenta[d]-1,2,4-triazolo[1,5-a]pyrimidine

A mixture of 0.814 g (0.003 mole) of 2-diallylamino-6,7-dihydro-8H-cyclopenta[d]-1,2,4-triazolo(1,5-a]pyrimidin--5[9H]-one, 3.87 g (0.024 mole ≈ 5.0 ml) of hexamethyl-disilazane, 3.12 g (0.024 mole ≈ 3.12 ml) of 2-morpholinoethylamine and 0.19 g (0.001 mole) of p-toluenesulfonic acid monohydrate is stirred at 160 °C for 3 hours. Then it is cooled to 90 °C and 20 ml of methanol are dropped to it. The mixture is boiled for further 1 hour and evaporated to dryness in vacuo. To the residual brown oil (about 4 g) 20 ml of ether are added, then the separated crystals are filtered and washed with water.
Yield: 0.84 g (73 %)
M.p.: 131.5-133.5 °C (after recrystallization from a mixture of cyclohexane and ethyl acetate in a ratio of 1 : 1 [ml/ml]).

### Example 81

### 5-[N-(2'-Morpholinoethyl)]amino-2-diallylamino-6,7-dihydro-8H-cyclopenta[d]-1,2,4-triazolo[1,5-a] pyrimidine

To a solution of 1.60 g (0.00466 mole) of 2-diallylamino-5-trimethylsilyloxy-6,7-dihydro-8H-cyclopenta[d]-1,2,4-triazolo[1,5-a]-pyrimidine in 1.55 g (0.0096 mole ≈ 2.0 ml) of hexamethyldisilazane 3.0 g (0.023 mole) of 2-morpholino-ethylamine and 0.13 g (0.001 mole) of anhydrous ammonium sulfate are added, and the reaction mixture is stirred at 160 °C for 3 hours. Then it is cooled to 90 °C and 10 ml of methanol are cautiously dropped to it. The mixture is evaporated to dryness and the residue is suspended with water, then the separated crystals are filtered and washed with water.
Yield: 1.50 g (83.8 %)
M.p.: 132-133.5 °C (recrystallized from a mixture of cyclohexane and ethyl acetate in a ratio of 1:1 [ml/ml]).

### Example 82

### 5-[N-(2'-Morpholinoethyl)]amino-2-diallylamino-6,7-dihydro-8H-cyclopenta[d]-1,2,4-triazolo[1,5-a] -pyrimidine

To a solution of 0.81 g (0.0028 mole) of 2-diallylamino-5-chloro-6,7-dihydro-8H-cyclopenta[d]-1,2,4-triazolo[1,5-a]pyrimidine in 3 ml of acetonitrile 0.81 g (0.0062 mole) of 2-morpholinoethylamine is added, and the reaction mixture is boiled for 1 hour under stirring. Then it is cooled and 3 ml of water are added. The separated crystals are filtered and washed with a slight amount of a 1:1 (by volume) mixture of acetonitrile and water.
Yield: 0.45 g (42 %)
M.p.: 131-133 °C (recrystallized from a mixture of cyclohexane and ethyl acetate in a ratio of 1 : 1 [ml/ml]).

### Example 83

### 2-Methylsulphinyl-5-[N-(2'-morpholinoethyl)]amino-6,7,8,9-tetrahydro-1,2,4-triazolo[5,1-b]quinazoline

To a solution of 3.65 g (0.0135 mole) of 5-chloro-2-methylsulphinyl-6,7,8,9-tetrahydro-1,2,4-triazolo[5,1-b]-quinazoline in 15 ml of acetonitrile 3.91 g (0.03 mole) of 2-morpholinoethylamine are added, and the reaction mixture is boiled for 1 hour under stirring. The solution is evaporated to dryness in vacuo and the residual oil is dissolved in 25 ml of water. The aqueous solution is extracted with chloroform, the chloroform phase is washed with water, dried over anhydrous sodium sulfate and evaporated to dryness in vacuo. The residual 4.8 g of oily product gets crystalline upon adding 20 ml of ethyl acetate. The crystals are filtered and washed with a slight amount of ethyl acetate.
Yield: 4.23 g (86 %)
M.p.: 141-143 °C(recrystallized from ethyl acetate).

### Example 84

### 2-Diallylamino-5-[4-ethoxycarbonylpiperazino]-6,7-dihydro-8H-cyclopenta[d]-1,2,4-triazolo[1,5-a] pyrimidine

To a solution of 3.45 g (0.012 mole) of 2-diallylamino-5-chloro-6,7-dihydro-8H-cyclopenta[d]-1,2,4-triazolo[1,5-a]pyrimidine in l0 ml of acetonitrile 1.45 g (0.0143 mole ≈ 2.0 ml) of triethylamine and 2.26 g (0.0143 mole ≈ 2.1 ml) of ethoxycarbonylpiperazine are added, and the reaction mixture is stirred at room temperature for 5 hours. Then further 1.08 g (0.0068 mole ≈ 1.0 ml) of ethoxycarbonylpiperazine are added to the mixture, which is stirred for a further hour and allowed to stand overnight. 10 ml of water are dropped to it under stirring, then the separated crystalline product is filtered and washed with water.
Yield: 2.68 g (54.7 %)
M.p.: 125-128 °C (after recrystallization from ethyl acetate).

### Example 85

### 2-Diallylamino-5-[4-ethoxycarbonylpiperazino]-6,7-dihydro-8H-cyclopenta[d]-1,2,4-triazolo[1,5-a]pyrimidine maleate

2.06 g (0.005 mole) of the compound prepared according to Example 84 and 0.81 g (0.007 mole) of maleic acid are dissolved in 25 ml of ethyl acetate at 30 °C. 30 ml of diethyl ether are added to the solution at the same temperature, and the mixture is allowed to crystallize on standing. Then it is cooled to a temperature between 0 °C and 5 °C, the separated crystals are filtered and washed with a slight amount of a 1:1 (by volume) mixture of ether and ethyl acetate.
Yield: 2.40 g (90.9 %)
M.p.: 87-92 °C (recrystallized from a mixture of ethyl acetate and ether in a ratio of 1:1 [ml/ml]).

### Example 86

### 5-[N-Ethyl-N-(2'-hydroxyethyl)]amino-2-methylthio-6,7,8,9-tetrahydro-1,2,4-triazolo[5,1-b]quinazoline

To a solution of 5.1 g (0.02 mole) of 5-chloro-2-methylthio-6,7,8,9-tetrahydro-1,2,4-triazolo[5,1-b]quinazoline in 20 ml of acetonitrile 2.23 g (0.022 mole ≈ 3.07 ml) of triethylamine and 3.92 g (0.044 mole ≈ 4.3 ml) of 2-ethylaminoethanol are added, and the reaction mixture is boiled for 4 hours under stirring. Then 80 ml of water are added to the solution while it is still warm. After cooling the separated product is filtered and washed with water and a slight amount of ethyl acetate.
Yield: 3.40 g (55.3 %)
M.p.: 124-126 °C (recrystallized from acetonitrile).

### Example 87

### 5-[N-(n-Butyl)-N-(2-hydroxyethyl)]amino-2-morpholino-6,7,8,9-tetrahydro-1,2,4-triazolo[5,1-b]quinazoline

To a solution of 1.20 g (0.0041 mole) of 5-chloro-2--morpholino-6,7,8,9-tetrahydro-1,2,4-triazolo[5,1-b]quinazoline in 5 ml of acetonitrile 1.17 g (0.01 mole ≈ 1.31 ml) of 2-(n-butylamino)ethanol are added and the reaction mixture is boiled for 4 hours under stirring. The solution thus obtained is evaporated to dryness in vacuo. The residue is dissolved in a mixture of 20 ml of chloroform and 20 ml of water. The phases are separated, the chloroform phase is dried and evaporated to dryness in vacuo. Thus 1.56 g of yellow oily product is obtained, which is purified by chromatography on a Kieselgel 60H layer (eluent: a mixture of benzene and chloroform) and recrystallized from a mixture of ether and acetone in a ratio of 25:3 [ml/ml].
Yield: 0.81 g (55.1 %)
M.p.: 90-92 °C.

### Example 88

### 2-Methylthio-5-morpholino-6,7,8,9,10,11-hexahydrocycloocta[d]-1,2,4-triazolo[1,5-a]pyrimidine

To a solution of 0.10 g (0.00035 mole) of 5-chloro-2-methylthio-6,7,8,9,10,11-hexahydro-cycloocta[d]-1,2,4-triazolo[1,5-a]pyrimidine in 2 ml of acetonitrile 0.13 g (0.0015 mole ≈ 0.13 ml) of morpholine is added, and the reaction mixture is boiled for 2 hours. Upon adding 6 ml of water to the hot solution crystals get separated, which are cooled and washed with water and a slight amount of icy acetonitrile.
Yield: 0.068 g (58.1 %)
M.p.: 172.5-173.5 °C (after recrystallization from ethyl acetate).

### Example 89

### 2-Methylthio-5-morpholino-6,7,8,9,10,11,12,13,14,15-decahydro-cyclododeca[d]-1,2,4-triazolo[1,5-a] pyrimidine

To a solution of 0.13 g (0.00038 mole) of 5-chloro-2-methylthio-6,7,8,9,10,11,12,13,14,15-decahydro-cyclododeca[d]-1,2,4-triazolo[1,5-a]pyrimidine in 2 ml of acetonitrile 0.13 g (0.0015 mole ≈ 0.13 ml) of morpholine is added, and the reaction mixture is boiled for 2 hours under stirring. Upon dropping 6 ml of water to the solution thus obtained crystals get separated. After cooling the product is filtered and washed with water and a slight amount of icy acetonitrile.
Yield: 0.123 g (83.1 %)
M.p.: 151-153 °C (after recrystallization from acetonitrile),

### Example 90

### 5-Benzylamino-2-(4'-methylpiperazino)-6,7-dihydro-8H-cyclopenta[d]-1,2,4-triazolo[1,5-a]pyrimidine

A mixture of 1.34 g (0.0049 mole) of 2-(4'-methylpiperazino)-6,7-dihydro-8H-cyclopenta[d]-1,2,4-triazolo[1,5-a]pyrimidin-5(9H)-one, 8.07 g (0.05 mole ≈ 10.4 ml) of hexamethyldisilazane, 5.36 g (0.05 mole ≈ 5.46 ml) of benzylamine and 0.1 g (0.001 mole) of p-toluenesulfonic acid monohydrate are stirred on an oil bath of 160-165 °C for 12 hours. After cooling 20 ml of methanol are added to the reaction mixture which is boiled further for 1 hour, then evaporated to dryness in vacuo. 4 ml of ethyl acetate are added to the residual brown oil (about 3 g). The separated crystals are filtered and washed with a slight amount of cold ethyl acetate.
Yield: 1.37 g (77.4 %)
M.p.: 195-197 °C (after recrystallization from acetonitrile).

### Example 91

### 5-[N-(3',4'-Dihydroxyphenylethyl)amino]-2-ethylamino-6,7-dihydro-8H-cyclopenta[d]-1,2,4-triazolo[1,5-a] pyrimidine

A mixture of 2.85 g (0.012 mole) of 2-ethylamino-5-chloro-6,7-dihydro-8H-cyclopenta[d]-1,2,4-triazolo[1,5-a]pyrimidine, 20 ml of isopropanol, 2.53 g (0.025 mole ≈ 3.5 ml) of triethylamine and 2.81 g (0.012 mole) of 2-(3',4'-dihydroxyphenyl)ethylamine hydrobromide is stirred on an oil bath of 100 °C for 1 hour. Upon cooling the solution gets crystalline. The separated crystals are filtered and washed successively with water, isopropanol and ether.
Yield: 3.73 g (87.7 %)
M.p.: 254-258 °C (decomp.) (after recrystallizaton from acetic acid).

### Example 92

### 5-Diisopropylamino-7-methyl-2-morpholino-1,2,4-triazolo[1,5-a]pyrimidine

To a solution of 2.54 g (0.01 mole) of 5-chloro-7--methyl-2-morpholino-1,2,4-triazolo[1,5-a]pyrimidine in 10 ml of dimethylformamide 2.02 g (0.02 mole ≈ 2.8 ml) of diisopropylamine are added, and the reaction mixture is boiled for 24 hours under stirring. The solution thus obtained is evaporated to dryness in vacuo and the residue is recrystallized from 20 ml of water.
Yield: 1.5 g (47.1 %)
M.p.: 134-136 °C.

### Example 93

### 2-Morpholino-5-[N-(3'-morpholinopropyl)amino]-6,8-dihydro-9H-thiopyrano[4,3-d]-1,2,4-triazolo[1,5-a] pyrimidine

To a solution of 2.43 g (0.0078 mole) of 5-chloro-2-morpholino-6,8-dihydro-9H-thiopyrano[4,3-d]-1,2,4-triazolo[1,5-a]pyrimidine in 15 ml of acetonitrile 1.44 g (0.01 mole ≈ 1.46 ml) of 3-morpholinopropylamine and 1.01g (0.01 mole ≈ 1.39 ml) of triethylamine are added, and the reaction mixture is boiled for 1 hour under stirring. Then it is cooled and the separated product is filtered and washed with water and acetonitrile.
Yield: 3.02 g (92.3 %)
M.p.: 173-176 °C (after recrystallization from acetonitrile).

### Example 94

### 2-Ethylamino-5-morpholino-6,7-dihydro-8H-cyclopenta[d]--1,2,4-triazolo[1,5-a]pyrimidine

To a solution of 0.96 g (0.004 mole) of 2-ethylamino-5-chloro-6,7-dihydro-8H-cyclopenta[d]-1,2,4-triazolo[1,5-a]pyrimidine in 5 ml of acetonitrile 0.87 g (0.01 mole ≈ 0.87 ml) of morpholine is added, and the reaction mixture is boiled for 1.5 hours under stirring. Then it is cooled and 10 ml of water are added. The separated product is filtered and washed with water and icy acetonitrile.
Yield: 1.04 g (90 %)
M.p.: 278-283 °C (decomp.) [recrystallized from n-butanol].

### Example 95

### 2,5-Dimorpholino-6,7,8,9,10,11-hexahydro-cycloocta[d]-1,2,4-triazolo[1,5-a]pyrimidine

A mixture of 0.30 g (0.00093 mole) of 5-chloro-2-morpholino-6,7,8,9,10,11-hexahydro-cycloocta[d]-1,2,4-triazolo[1,5-a]pyrimidine, 3 ml of acetonitrile and 0.26 g (0.003 mole ≈ 0.26 ml) of morpholine is boiled for 2 hours under stirring. The warm reaction mixture is diluted with 3 ml of water and cooled. The separated crystals are filtered and washed with water and icy acetonitrile.
Yield: 0.28 g (80.0 %)
M.p.: 227-229 °C (after recrystallization from acetonitrile).

### Example 96

### 2,5-Dimorpholino-6,7,8,9,10,11,12,13,14,15-decahydro-cyclododeca[d]-1,2,4-triazolo[1,5-a]pyrimidine

To a solution of 0.30 g (0.0008 mole) of 5-chloro-2--morpholino-6,7,8,9,10,11,12,13,14,15-decahydro-cyclododeca[d]-1,2,4-triazolo[1,5-a]pyrimidine in 2.0 ml of acetonitrile 0.26 g (0.003 mole ≈ 0.26 ml) of morpholine is added, and the reaction mixture is boiled for 2 hours. 3 ml of water are added to the warm solution. The crystals separated upon cooling are filtered and washed with water and icy acetonitrile.
Yield: 0.29 g (85.3 %)
M.p.: 188-190 °C (after recrystallization from acetonitrile)

### Example 97

### 5-(4'-Methylpiperazino)-2-morpholino-6,7,8,9-tetrahydro--1,2,4-triazolo[5,1-b]quinazoline

A mixture of 8.81 g (0.03 mole) of 5-chloro-2-morpholino-6,7,8,9-tetrahydro-1,2,4-triazolo[5,1-b]quinazoline, 6.01 g (0.06 mole) of N-methylpiperazine and 30 ml of isopropanol is boiled for 1 hour under stirring, then it is evaporated to dryness in vacuo. The residue is triturated with 50 ml of water, the crystalline product is filtered and washed with water.
Yield: 9.7 g (90.5 %)
M.p.: 196-197 °C (after recrystallization from acetonitrile).

### Example 98

### 5-Morpholino-2-(n-octylamino)-6,7-dihydro-8H-cyclopenta[d]-1,2,4-triazolo[1,5-a]pyrimidine

To a solution of 1.20 g (0.0037 mole) of 5-chloro-2-(n-octylamino)-6,7-dihydro-8H-cyclopenta[d]-1,2,4-triazolo[1,5-a]pyrimidine in 5 ml of acetonitrile 0.96 g (0.011 mole) of morpholine are added, and the reaction mixture is boiled for 1.5 hours under stirring. To the warm solution 5 ml of water are added. The crystals separated upon cooling are filtered and washed with water.
Yield: 1.23 g (89.1 %)
M.p.: 179-181 °C (after recrystallization from acetonitrile).

### Example 99

### 5-[N-(2'-Morpholinoethyl)amino]-2-(n-hexylthio)-6,7,8,9-tetrahydro-1,2,4-triazolo[5,1-b]quinazoline

To a solution of 6.73 g (0.0207 mole) of 2-(n-hexyl-thio)-5-chloro-6,7,8,9-tetrahydro-1,2,4-triazolo[5,1-b]quinazoline in 30 ml of acetonitrile 2.23 g (0.022 mole ≈ 3.07 ml) of triethylamine and 2.86 g (0.022 mole ≈ 2.9 ml) of 2-morpholinoethylamine are added, and the reaction mixture is boiled for 1.5 hours under stirring. 30 ml of water are added to the warm mixture. The crystals separated upon cooling are filtered and washed with water.
Yield: 7.78 g (89.8 %)
m.p.: 107-108 °C (after recrystallization from ethyl acetate).

### Example 100

### 2-(Allylthio)-5-[N-(2'-morpholinoethyl)amino]-6,7,8,9-tetrahydro-1,2,4-triazolo[5,1-b]quinazoline

To a solution of 5.0 g (0.0178 mole) of 2-allylthio-5-chloro-6,7,8,9-tetrahydro-1,2,4-triazolo[5,1-b]quinazoline in 15 ml of acetonitrile 2.02 g (0.02 mole ≈ 2.79 ml) of triethylamine and 2.60 g (0.02 mole ≈ 2.60 ml) of 2-morpholinoethylamine are added. The reaction mixture is boiled for 1 hour under stirring and then 30 ml of water are added. The crystals separated upon cooling are filtered and washed with water.
Yield: 5.80 g (87 %)
M.p.: 129.5-130.5 °C (after recrystallization from acetonitrile).

### Example 101

### 2-Benzylthio-5-[N-(2'-morpholinoethyl)amino]-6,7,8,9-tetrahydro-1,2,4-triazolo[5,1-b]quinazoline

To a solution of 2.81 g (0.0085 mole) of 2-benzylthio-5-chloro-6,7,8,9-tetrahydro-1,2,4-triazolo[5,1-b]quinazoline in 10 ml of acetonitrile 1.01 g (0.01 mole ≈ 1.39 ml) of triethylamine and 1.30 g (0.01 mole ≈ 1.30 ml) of 2-morpholinoethylamine are added, and the reaction mixture is boiled for 1.5 hours under stirring. 10 ml of water are added to the warm solution, then the mixture is cooled, and the separated crystals are filtered and washed with water.
Yield: 3.23 g (89.5 %)
M.p.: 139-140 °C (after recrystallization from acetonitrile).

### Example 102

### 2-Benzylthio-5-[N-(3'-morpholinopropyl)amino]-6,7,8,9-tetrahydro-1,2,4-triazolo[5,1-b]quinazoline

To a solution of 2.33 g (0.007 mole) of 2-benzylthio-5-chloro-6,7,8,9-tetrahydro-1,2,4-triazolo[5,1-b]quinazoline in 8 ml of acetonitrile 0.91 g (0.009 mole ≈ 1.25 ml) of triethylamine and 1.30 g (0.009 mole ≈ 1.32 ml) of 3-morpholinopropylamine are added, and the reaction mixture is boiled for 2 hours under stirring. 8 ml of water are added to the warm solution, then the mixture is cooled, and the acetonitrile is distilled off in vacuo. The residual aqueous solution is extracted with 10 ml of chloroform, the chloroform phase is dried over sodium sulfate and evaporated to dryness in vacuo. The oily product thus obtained gets crystalline upon adding a slight amount of ether. The separated crystals are filtered and washed with ether.
Yield: 2.63 g (86.6 %)
M.p.: 114.5-116 °C (after recrystallization from ethyl acetate).

### Example 103

### 2-Methylthio-5-piperazino-6,7,8,9-tetrahydro-1,2,4-triazolo[5,1-b]quinazoline

To a solution of 0.64 g (0.0025 mole) of 5-chloro-2-methylthio-6,7,8,9-tetrahydro-1,2,4-triazolo[5,1-b]quinazoline in 10 ml of acetonitrile 0.43 g (0.005 mole) of anhydrous piperazine is added, and the reaction mixture is boiled for 1.5 hours under stirring. Then it is cooled, the separated crystals are filtered and washed four times with 5 ml of acetonitrile each. The acetonitrile filtrates are combined and evaporated to dryness in vacuo. The residual crystals are suspended in a slight amount of water, filtered again and washed with water and ether.
Yield: 0.55 g (72.4 %)
M.p.: 171-172.5 °C (after recrystallization from ethyl acetate).

### Example 104

### 5-[N-(2'-Morpholinoethyl)amino]-2-[4"-nitrobenzylthio]-6,7,8,9-tetrahydro-1,2,4-triazolo[5,1-b]quinazoline

A mixture of 0.92 g (0.00245 mole) of 5-chloro-2-(4-nitrobenzylthio)-6,7,8,9-tetrahydro-1,2,4-triazolo[5,1-b]quinazoline, 5 ml of dichloroethane, 0.28 g (0.0027 mole ≈ 0.38 ml) of triethylamine and 0.35 g (0.0027 mole ≈ 0.35 ml) of 2-morpholinoethylamine is boiled for 1 hour under stirring. Then it is cooled, 5 ml of water are added, the phases are separated, the dichloroethane phase is dried over anhydrous sodium sulfate and evaporated to dryness in vacuo.
Yield: 0.98 g (85.2 %)
M.p.: 165-167 °C (after recrystallization from acetonitrile).

### Example 105

### 2-(4'-Chlorobenzylthio)-5-[N-(2'-morpholinoethyl)amino)-6,7,8,9-tetrahydro[5,1-b]quinazoline

A mixture of 0.80 g (0.0022 mole) of 5-chloro-2-(4'-chlorobenzylthio)-6,7,8,9-tetrahydro-1,2,4-triazolo[5,1-b]quinazoline, 5 ml of dichloroethane, 0.25 g (0.0025 mole ≈ 0.35 ml) of triethylamine and 0.325 g (0.0025 mole ≈ 0.33 ml) of 2-morpholinoethylamine is boiled for 4 hours under stirring. Then it is cooled, 5 ml of dichloroethane and 10 ml of water are added, the phases are separated, the organic phase is extracted with a saturated sodium chloride solution, dried over anhydrous sodium sulfate and evaporated to dryness in vacuo.
Yield: 0.77 g (76.2 %)
M.p.: 136-137.5 °C (after recrystallization from acetonitrile).

### Example 106

### 5-[N-(2'-Morpholinoethyl)amino]-2-(2"-nitro-4"-trifluoromethylphenylthio)-6,7,8,9-tetrahydro-1,2,4-triazolo [5,1-b]quinazoline

To a solution of 0.95 g (0.0022 mole) of 5-chloro-2-(2'-nitro-4'-trifluoromethylphenylthio)-6,7,8,9-tetrahydro-1,2,4'-triazolo[5,1-b]quinazoline in 10 ml of acetonitrile 0.24 g (0.0024 mole ≈ 0.33 ml) of triethylamine and 0.31 g (0.0024 mole ≈ 0.31 ml) of 2-morpholinoethylamine are added, and the reaction mixture is boiled for 2 hours under stirring. The solution thus obtained is evaporated to dryness in vacuo, the residue is dissolved in a mixture of 10 ml of water and 15 ml of chloroform, the aqueous phase is extracted with 10 ml of chloroform, the chloroform phases are combined, extracted with water, dried over anhydrous sodium sulfate and evaporated to dryness in vacuo. To the residual oily product a slight amount of diisopropyl ether and a few drops of ethyl acetate are added. The separated crystals are filtered and washed with diisopropyl ether.
Yield: 0.42 g (36.5 %)
M.p.: 124-126 °C (from a mixture of ethyl acetate and isopropanol in a ratio of 1:1 [ml/ml]).

### Example 107

### 2-Benzylamino-5-[N-(2'-morpholinoethyl)amino]-6,7,8,9-tetrahydro-1,2,4-triazolo[5,1-b]quinazoline

A mixture of 3.14 g (0.01 mole) of 2-benzylamino-5-chloro-6,7,8,9-tetrahydro-1,2,4-triazolo[5,1-b]quinazoline, 25 ml of isopropanol, 1.11 g (0.011 mole ≈ 1.53 ml) of triethylamine and 1.43 g (0.011 mole ≈ 1.43 ml) of 2-morpholinoethylamine is stirred on an oil bath of 100 °C for half an hour. 25 ml of water are added to the warm reaction mixture, then it is stirred until it gets cool. The separated product is filtered and washed with water and a slight amount of isopropanol.
Yield: 3.88 g (95.2 %)
M.p.: 192-194 °C (after recrystallization from methanol).

### Example 108

### 2-(Dimethylamino)-5-[N-(2'-hydroxyethyl)amino]-7,8-dihydro-9H-thiopyrano[3,2-d]-1,2,4-triazolo[1,5-a] pyrimidine

A mixture of 1.35 g (0.005 mole) of 2-dimethylamino-5-chloro-7,8-dihydro-9H-thiopyrano[3,2-d]-1,2,4-triazolo[1,5-a]pyrimidine, 5 ml of isopropanol and 0.61 g (0.01 mole) of ethanolamine is boiled for half an hour under stirring. 10 ml of water are added to the warm solution. The mixture is cooled, then the separated product is filtered and washed with water and cold isopropanol.
Yield: 1.27 g (86.4 %)
M.p.: 187-189 °C (after recrystallization from acetonitrile).

### Example 109

### 5-Amino-2-methylthio-6,7-dihydro-8H-cyclopenta[d]-1,2,4-triazolo[1,5-a]pyrimidine

A mixture of 1.45 g (0.006 mole) of 5-chloro-2-methylthio-6,7-dihydro-8H-cyclopenta[d]-1,2,4-triazolo[1,5-a]pyrimidine, 10 ml of ethanol and 2.5 ml (0.03 mole) of concentrated ammonium hydroxide is stirred at room temperature for 2 hours. The separated crystalline product is filtered and washed with cold ethanol.
Yield: 0.8 g (60 %)
M.p.: 305-307 °C (decomp.) [recrystallized from dimethylformamide].

### Example 110

### 2-Methylthio-5-morpholino-6,7,8,9-tetrahydro-1,2,4-triazolo[5,1-b]quinazoline

A mixture of 0.098 g (0.00025 mole) of 5-(4'-methylphenylsulfonyloxy)-2-methylthio-6,7,8,9-tetrahydro-1,2,4--triazolo[5,1-b]quinazoline, 2 ml of acetonitrile and 0.13 g (0.0015 mole) of morpholine is boiled for 2 hours under stirring. Then it is cooled and evaporated to dryness in vacuo. The residue is dissolved in a mixture of 10 ml of chloroform and 10 ml of water, the phases are separated, the chloroform phase is extracted with 5 ml of 1N sodium hydroxide solution and water, dried and evaporated.
Yield: 0.029 g (38%)
M.p.: 183-185 °C (after recrystallization from acetonitrile).

### Example 111

### 2-Methylthio-5-morpholino-6,7,8,9-tetrahydro-1,2,4-triazolo[5,1-b]quinazoline

A mixture of 0.03 g (0.0001 mole) of 5-(methanesulfonyloxy)-2-methylthio-6,7,8,9-tetrahydro-1,2,4-triazolo[5,1-b]quinazoline, 1 ml of acetonitrile and 0.085 g (0.00075 mole) of morpholine is boiled for 1 hour under stirring. Then it is cooled and evaporated to dryness in vacuo. The residue is dissolved in a mixture of 5 ml of chloroform and 5 ml of water, the phases are separated, the chloroform phase is extracted with 1 ml of 1N sodium hydroxide solution and water, dried and evaporated.
Yield: 0.01 g (36 %)
M.p.: 182-185 °C (after recrystallization from acetonitrile).

### Example 112

### 5-[(4'-Hydroxypiperidino)]-2-methylthio-6,7,8,9-tetrahydro-1,2,4-triazolo[5,1-b]quinazoline

To a solution of 5.1 g (0.02 mole) of 5-chloro-2-methylthio-6,7,8,9-tetrahydro-1,2,4-triazolo[5,1-b]quinazoline in 30 ml of acetonitrile 6.47 g (0.064 mole) of 4-hydroxypiperidine are added, and the reaction mixture is stirred at room temperature for 10 hours. Then 30 ml of water are added, the mixture is stirred for a further hour, then the separated product is filtered and washed with water and cold acetonitrile.
Yield: 5.71 g (89.2 %)
M.p.: 223-224.5 °C (after recrystallization from acetonitrile).

### Example 113

### 2-(3'-Dimethylaminopropylamino)-5-morpholino-6,7--dihydro-8H-cyclopenta[d]-1,2,4-triazolo[1,5-a] pyrimidine

To a solution of 1.77 g (0.006 mole) of 2-(3'-dimethylaminopropylamino)-5-chloro-6,7-dihydro-8H-cyclopenta[d]-1,2,4-triazolo[1,5-a]pyrimidine in 15 ml of chloroform 1.92 g (0.022 mole ≈ 1.92 ml) of morpholine are added, and the reaction mixture is boiled for 1.5 hours under stirring. Then it is cooled, extracted with 20 ml of water, dried and evaporated to dryness in vacuo.
Yield: 1.3 g (62.8 %)
M.p.: 210-213 °C (after recrystallization from acetonitrile).

### Example 114

### 5-(2',3'-Dichloro-6'-aminobenzylamino)-2-morpholino-6,7-dihydro-8H-cyclopenta[d]-1,2,4-triazolo[1,5-a] pyrimidine

A mixture of 1.38 g (0.005 mole) of 5-chloro-2-morpholino-6,7-dihydro-8H-cyclopenta[d]-1,2,4-triazolo[1,5-a]pyrimidine, 8 ml of isopropanol, 1.13 g (0.005 mole) of 2,3-dichloro-6-aminobenzylamine hydrochloride and 1.5 ml (0.015 mole) of triethylamine is boiled for 1.5 hours under stirring. Then it is cooled and evaporated to dryness in vacuo. The residue is suspended in 20 ml of water, filtered and washed with water.
Yield 2.14 g (98.5 %)
M.p.: 255-258 °C (recrystallized from dimethylformamide).

### Example 115

### 5-(2'-Methylbenzylamino)-2-morpholino-6,7-dihydro-8H-cyclopenta[d]-1,2,4-triazolo[1,5-a]pyrimidine

To a solution of 2.78 g (0.01 mole) of 5-chloro-2-morpholino-6,7-dihydro-8H-cyclopenta[d]-1,2,4-triazolo[1,5-a]pyrimidine in 15 ml of chloroform 1.34 g (0.011 mole) of 2-methylbenzylamine and 1.0 g (0.01 mole ≈ 1.5 ml) of triethylamine are added, and the reaction mixture is boiled for 4 hours. Then it is cooled, and the separated product is filtered and washed with water and cold isopropanol.
Yield: 2.93 g (80.5 %)
M.p.: 97-99 °C (recrystallized from isopropanol).

### Example 116

### 5-Morpholino-2-(4'-methylpiperazino)-6,7-dihydro-8H-cyclopenta[d]-1,2,4-triazolo[1,5-a]pyrimidine

To a solution of 2.93 g (0.01 mole) of 5-chloro-2-(4'-methylpiperazino)-6,7-dihydro-8H-cyclopenta[d]-1,2,4-triazolo[1,5-a]pyrimidine in 15 ml of chloroform 1.92 g (0.022 mole ≈ 1.92 ml) of morpholine are added, and the reaction mixture is boiled for 4 hours. Then it is cooled and evaporated to dryness in vacuo. The residue is suspended in 50 ml of water, filtered and washed with water and isopropanol.
Yield: 2.16 g (63.0 %)
M.p.: 165-167 °C (recrystallized from isopropanol).

### Example 117

### 5-Morpholino-2-piperidino-6,7-dihydro-8H-cyclopenta[d]-1,2,4-triazolo[1,5-a]pyrimidine

To a solution of 2.78 g (0.01 mole) of 5-chloro-2-piperidino-6,7-dihydro-8H-cyclopenta[d]-1,2,4-triazolo[1,5-a]pyrimidine in 15 ml of chloroform 1.92 g (0.022 mole ≈ 1.92 ml) of morpholine are added, and the reaction mixture is boiled for 2 hours under stirring. Then it is cooled and evaporated to dryness in vacuo. The residue is suspended in 50 ml of water, filtered and washed with water and a slight amount of isopropanol.
Yield: 3.24 g (98.8 %)
M.p.: 187-189 °C (recrystallized from isopropanol).

### Example 118

### 3-[N-Methyl-N-(2'-morpholino-6',7'-dihydro-8'H-cyclopenta[d]-1',2',4'-triazolo[1',5'-a]pyrimidin-5'-yl)-amino]-propyl amine and N-{3-[N'-(2"-morpholino-6",7"-dihydro-8"H-cyclopenta[d]-1,2,4-triazolo[1,5-a]pyrimidin-5"-yl)-amino] -propyl}-N-methyl-amine

A mixture of 5.54 g (0.02 mole) of 5-chloro-2-morpholino-6,7-dihydro-8H-cyclopenta[d]-1,2,4-triazolo[1,5-a]pyrimidine, 30 ml of isopropanol and 3.52 g (0.04 mole ≈ 2.95 ml) of 3-methylaminopropylamine is boiled for 1.5 hours under stirring. Then it is cooled, 40 ml of water are added and the solution is neutralized with solid sodium hydrogen carbonate. The separated product, which is a mixture of the two title compounds, is filtered. Thus 4.52 g (68.2 %) of a crystalline product are obtained, which is recrystallized twice from isopropanol to yield pure 3-[N-methyl-N-(2'-morpholino-6',7'-dihydro-8'H-cyclopenta[d]-1',2',4'-triazolo[1',5'-a]pyrimidin-5'-yl)amino]-propylamine melting at 178-180 °C (recrystallized from isopropanol).

The mother liquors are evaporated to dryness and the residue is subjected to chromatography on a Kieselgel H column (eluent: a 1:2 mixture (by volume) of benzene and ethyl acetate) to obtain N-{3-(N'-(2"-morpholino-6",7"-dihydro-8"H-cyclopenta[d]-1",2",4"-triazolo[1",5"-a]pyrimidin-5"-yl)-amino]-propyl}-N-methylamine. It has been recrystallized from isopropanol.

### Example 119

### [N-(2-Methylthio-6,7-dihydro-8H-cyclopenta[d]-1,2,4-triazolo[1,5-a]pyrimidin-5-yl)]-aminoacetic acid

0.28 g (0.012 mole) of sodium metal is dissolved in 10 ml of abs. ethanol. 0.45 g (0.006 mole) of glycine and 1.45 g (0.006 mole) of 5-chloro-2-methylthio-6,7-dihydro-8H-cyclopenta[d]-1,2,4-triazolo[1,5-a]pyrimidine are added and the reaction mixture is stirred at 40 °C for 5 hours. Then it is cooled and evaporated to dryness. The residue is dissolved in 15 ml of benzene and extracted with 15 ml of water. The aqueous phase is treated with charcoal and acidified with glacial acetic acid, then the separated product is filtered and washed with water and a slight amount of isopropanol.
Yield: 1.10 g (65.6 %)
M.p.: 262-267 °C (decomp.) [after purification by clarification with charcoal].

## Claims (Claims for the following Contracting State(s): AT, BE, CH, DE, FR, IT, LI, LU, MC, NL, PT, SE)

1. 1,2,4-Triazolo[1,5-a]pyrimidine derivatives of the general formula 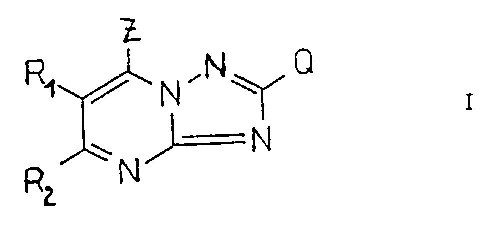 wherein
Q represents a morpholino, piperazino or piperidino group, optionally carrying a C₁₋₄ alkyl or benzyl substituent; or a group of the formula
S(O)ₚ-R₃ A,
wherein
p stands for 0, 1 or 2 and
R₃ denotes straight or branched chained C₁₋₈ alkyl, C₂₋₆ alkenyl or phenyl-(C₁₋₄ alkyl), which latter may optionally carry one or more halogen or nitro substituent(s), or phenyl, optionally substituted by one or more nitro and/or trifluoromethyl;
or a group of the formula 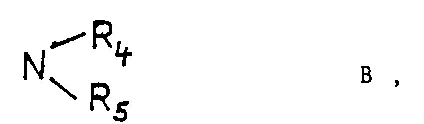 wherein
R₄ and R₅ independently each represent hydrogen, straight or branched chained C₁₋₁₂ alkyl, C₂₋₆ alkenyl, phenyl-(C₁₋₄ alkyl) including such having more than 1 phenyl group or di-(C₁₋₄ alkyl)-amino-(C₁₋₆ alkyl),
R₁ and R₂ independently each represent hydrogen or straight or branched chained C₁₋₄ alkyl or
R₁ and R₂ together form a group of the formula 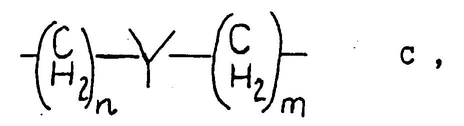 wherein
Y stands for a CH₂ group, a sulfur atom or a group of the formula 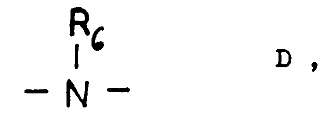
in which latter
R₆ denotes hydrogen or phenyl-(C₁₋₄ alkyl),
n and m each represent 0, 1, 2, 3, 4 or 5
and
Z represents a group of the formula 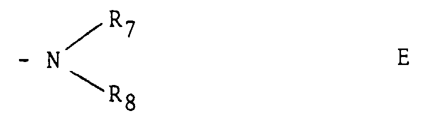 in which latter
R₇ and R₈ independently each represent hydrogen, straight or branched chained C₁₋₁₂ alkyl, optionally carrying one or more substituents(s) selected from the group consisting of hydroxy, carboxy, amino, morpholinocarbonyl, [4-(2'-{hydroxy}-ethyl)-piperazin--1'-yl]-carbonyl and (C₁₋₄ alkoxy)-carbonyl or optionally carrying a morpholino, piperazino or piperidino group which optionally carry a C₁₋₄ alkyl or benzyl substituent; C₂₋₆ alkenyl, C₃₋₈ cycloalkyl, adamantyl or phenyl-(C₁₋₄ alkyl), which latter may optionally carry one or more substituent(s) selected from the group consisting of halogen, hydroxy, C₁₋₄ alkyl, C₁₋₄ alkoxy, amino and nitro; (C₁₋₄ alkyl)-amino-(C₁₋₈ alkyl) and di-(C₁₋₄ alkyl)-amino-(C₁₋₈ alkyl);
or
R₇ and R₈ together form a group of the formula 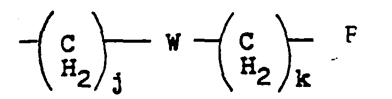 in which latter
j and k independently each represent 1, 2 or 3
and
W stands for oxygen or a 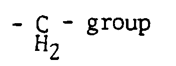
or a 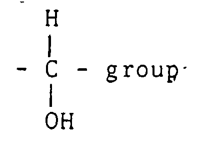 or a group of the formula 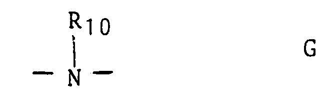 in which latter
R₁₀ denotes hydrogen, C₁₋₄ alkoxycarbonyl or C₁₋₄ alkyl, which latter may optionally carry one or more substituent(s) selected from the group consisting of hydroxy, (C₁₋₄-alkoxy)-carbonyl and phenyl;
or
Z stands for a group of the formula
-S-R₉ H
wherein
R₉ represents C₁₋₄ alkyl, optionally substituted by a (C₁₋₄ alkoxy)-carbonyl group,
with the provisos that
a) if Q represents a group of the formula A
R₁ and R₂ are other than hydrogen or C₁₋₄ alkyl
and
b) if Q represents a morpholino group,
Z represents formula E
with
R₇ = R₈ = hydrogen
and
R₁ stands for hydrogen,
R₂ is other than hydrogen
and their salts.

2. 1,2,4-Triazolo[1,5-a]pyrimidine derivatives according to claim 1, characterized in that the halogen(s) which may be carried by the phenyl-(C₁₋₄ alkyl) group which may be represented by R₃ is/are chlorine and/or bromine.

3. 1,2,4-Triazolo[1,5-a] pyrimidine derivatives according to claim 1 or 2, characterized in that the C₁₋₄ alkyl substituent which may be carried by the morpholino, piperazino or piperidino group which may be represented by Q and/or the alkyl part(s) of the phenyl-(C₁₋₄ alkyl) group(s) which may be represented by R₃ and/or R₄ and/or R₅ and/or R₆ and/or R₇ and/or R₈ and/or the C₁₋₄ alkyl group(s) of the di-(C₁₋₄ alkyl)-amino part(s) of the di-(C₁₋₄ alkyl)-amino-(C₁₋₆ alkyl group(s) which may be represented by R₄ and/or R₅ and/or the C₁₋₄ alkyl group(s) of the (C₁₋₄ alkyl)-amino parts of the (C₁₋₄ alkyl)-amino-(C₁₋₈ alkyl) groups(s) and/or of the di-(C₁₋₄ alkyl)-amino-(C₁₋₈ alkyl) group(s) which may be represented by R₇ and/or R₈ and/or the C₁₋₄ alkyl group which may be represented by R₉ or R₁₀ and/or the alkoxy part of the C₁₋₄ alkoxycarbonyl group which may be represented by R₁₀ and/or the C₁₋₄ alkyl group which may be represented by R₁ and/or R₂ is/are such one(s) having 1 or 2 carbon atom(s); and/or the C₁₋₈ alkyl group which may be represented by R₃ is such one having from 1 to 6, particularly from 1 to 3, carbon atom(s); and/or the C₂₋₆ alkenyl group(s) which may be represented by R₃ or R₄ and/or R₅ and/or R₇ and/or R₈ is/are such one(s) having from 2 to 4, particularly 3, carbon atoms, and/or the C₁₋₁₂ alkyl group which may be represented by R₄ and/or R₅ and/or R₇ and/or R₈ is/are such one[s] having from 1 to 8, particularly 1 to 6, carbon atom(s); and/or the C₁₋₆ alkyl group(s) of the amino-(C₁₋₆ alkyl) part(s) of the di-(C₁₋₄ alkyl)-amino-(C₁₋₆ alkyl) group(s) which may be represented by R₄ and/or R₅ is/are such one(s) having from 1 to 4 carbon atom(s); and/or the C₁₋₈ alkyl group(s) of the amino-(C₁₋₈ alkyl) part(s) of the (C₁₋₄ alkyl)-amino-(C₁₋₈ alkyl) and/or di-(C₁₋₄ alkyl)amino-(C₁₋₈ alkyl) group(s) which may be represented by R₇ and/or R₈ is/are such one(s) having from 1 to 6 carbon atom(s); and/or the C₃₋₈ cycloalkyl group(s) which may be represented by R₇ and/or R₈ is/are such one(s) having 5 or 6 carbon atom(s).

4. 1,2,4-Triazolo[1,5-a]pyrimidine derivatives according to claims 1 to 3, characterized in that
Q represents a morpholino, piperazino or piperidino group or a (C₁₋₆ alkyl)-thio group,
R₁ and R₂ independently each represent hydrogen or straight or branched chained C₁₋₄ alkyl or together form a 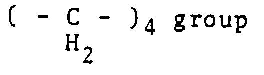 and
Z stands for a group of formula E, wherein R₇ and R₈ independently each represent straight or branched chained C₁₋₆ alkyl, which may optionally carry a morpholino, piperazino or piperidino group.

5. 2-(Methylthio)-5-{N-[3'-(morpholino)-propyl]}-amino-6,7,8,9-tetrahydro-1,2,4-triazolo[5,1-b]quinazoline, 5-(diethylamino)-7-methyl-2-morpholino-1,2,4-triazolo[1,5-a]pyrimidine, 2-[1'-(methyl)-ethyl-thio]-5-{N-[3'-(morpholino)-propyl]}-amino-6,7,8,9-tetrahydro-1,2,4-triazolo[5,1-b]quinazoline, 2-(ethylthio)-5-{N-[2'-(morpholino)-ethyl]}-amino-6,7,8,9-tetrahydro-1,2,4-triazolo[5,1-b]quinazoline and 2-(methylthio)-5-{N-[2'-(morpholino)-ethyl]}-amino-6,7,8, 9,10,11,12,13,14,15-decahydro-cyclododeca[d]-1,2,4-triazolo[1,5-a]pyrimidine and salts thereof.

6. A process for preparing the compounds according to claims 1 to 5, characterized by
reacting 1,2,4-triazolo[1,5-a]pyrimidine derivatives of the general formula 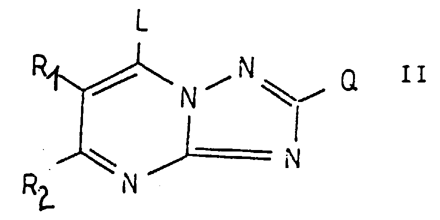 wherein
Q, R₁ and R₂ are as stated in claims 1 to 4,
and
L represents a leaving group, selected from halogen, tri-(C₁₋₄ alkyl)-silyloxy, C₁₋₄ alkylsulfonyloxy, arylsulfonyloxy or C₁₋₄ alkylarylsulfonyloxy,
with an amine or thiol of the general formula
H-Z III
wherein
Z is as stated in claims 1 to 4,
and optionally in a manner known per se converting a 1,2,4-triazolo[1,5-a]pyrimidine derivative of the formula I thus obtained into a salt or converting a salt of the 1,2,4-triazolo[1,5-a] pyrimidine derivative of the general formula I thus obtained into the free base of formula I or into another salt.

7. A process as claimed in claim 6, characterized by carrying out the reaction of the 1,2,4-triazolo[1,5-a]pyrimidine derivative of the general formula II with an excess of the amine of the general formula III.

8. A process as claimed in claim 6, characterized by carrying out the reaction of the 1,2,4-triazolo[1,5-a]pyrimidine derivative of the general formula II, wherein L represents halogen, C₁₋₄ alkylsulfonyloxy, arylsulfonyloxy or C₁₋₄ alkylarylsulfonyloxy, with the amine or thiol of the formula III in a protic, polar aprotic or apolar aprotic solvent, in the presence of a basic acid-binding agent.

9. Medicaments, characterized in that they contain as [an] active ingredient(s) at least one compound according to claims 1 to 5, suitably in admixture with at least one inert solid and/or liquid pharmaceutical carrier and/or diluent and/or additive.

10. Use of the compounds according to claims 1 to 5 for preparing medicaments, particularly such having positive inotropic, antianginal, antiinflammatory and ulcus and gastric acid secretion inhibiting effects.

## Claims (Claims for the following Contracting State(s): ES, GR)

1. A process for preparing 1,2,4-triazolo[1,5-a]pyrimidine derivatives of the general formula 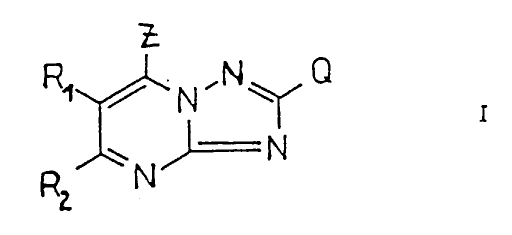 wherein
Q represents a morpholino, piperazino or piperidine group, optionally carrying a C₁₋₄ alkyl or benzyl substituent; or a group of the formula
S(O)ₚ-R₃ A,
wherein
p stands for 0, 1 or 2
and
R₃ denotes straight or branched chained C₁₋₈ alkyl, C₂₋₆ alkenyl or phenyl-(C₁₋₄ alkyl), which latter may optionally carry one or more halogen or nitro substituent(s), or phenyl, optionally substituted by one or more nitro and/or trifluoromethyl;
or a group of the formula 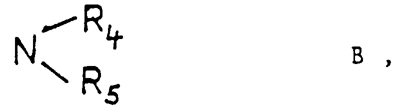 wherein
R₄ and R₅ independently each represent hydrogen, straight or branched chained C₁₋₁₂ alkyl, C₂₋₆ alkenyl, phenyl--(C₁₋₄ alkyl) including such having more than 1 phenyl group or di-(C₁₋₄ alkyl)-amino-(C₁₋₆ alkyl),
R₁ and R₂ independently each represent hydrogen or straight or branched chained C₁₋₄ alkyl or
R₁ and R₂ together form a group of the formula 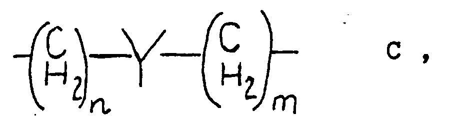 wherein
Y stands for a CH₂ group, a sulfur atom or a group of the formula 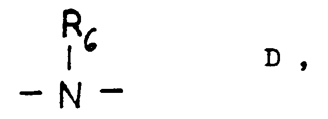
in which latter
R₆ denotes hydrogen or phenyl-(C₁₋₄ alkyl),
nand m each represent 0, 1, 2, 3, 4 or 5
and
Z represents a group of the formula 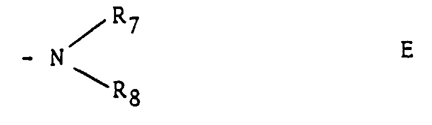 in which latter
R₇ and R₈ independently each represent hydrogen, straight or branched chained C₁₋₁₂ alkyl, optionally carrying one or more substituents(s) selected from the group consisting of hydroxy, carboxy, amino, morpholinocarbonyl, [4-(2'-{hydroxy}-ethyl)-piperazin-1-yl]-carbonyl and (C₁₋₄ alkoxy)-carbonyl or optionally carrying a morpholino, piperazino or piperidino group which optionally carry a C₁₋₄ alkyl or benzyl substituent; C₂₋₆ alkenyl, C₃₋₈ cycloalkyl, adamantyl or phenyl-(C₁₋₄ alkyl), which latter may optionally carry one or more substituent(s) selected from the group consisting of halogen, hydroxy, C₁₋₄ alkyl, C₁₋₄ alkoxy, amino and nitro; (C₁₋₄ alkyl)-amino-(C₁₋₈ alkyl) and di-(C₁₋₄ alkyl)-amino-(C₁₋₈ alkyl);
or
R₇ and R₈ together form a group of the formula 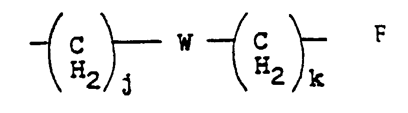 in which latter
j and k independently each represent 1, 2 or 3
and
W stands for oxygen or a 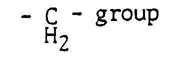
or a 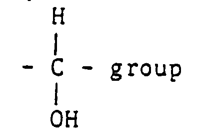 or a group of the formula 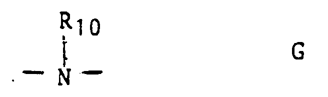 in which latter
R₁₀ denotes hydrogen, C₁₋₄ alkoxycarbonyl or C₁₋₄ alkyl, which latter may optionally carry one or more substituent(s) selected from the group consisting of hydroxy, (C₁₋₄-alkoxy)-carbonyl and phenyl;
or
Z stands for a group of the formula
-S-R₉ H
wherein
R₉ represents C₁₋₄ alkyl, optionally substituted by a (C₁₋₄ alkoxy)-carbonyl group,
with the provisos that
a) if Q represents a group of the formula A
R₁ and R₂ are other than hydrogen or C₁₋₄ alkyl,
b) if Q represents a morpholino group,
Z represents formula E
with
R₇ = R₈ = hydrogen
and
R₁ stands for hydrogen,
R₂ is other than hydrogen
and their salts, characterized by
reacting 1,2,4-triazolo[1,5-a]pyrimidine derivatives of the general formula 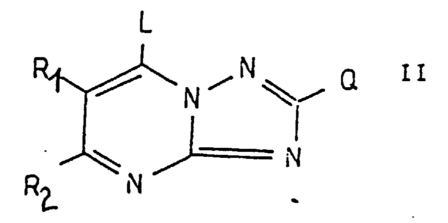 wherein
Q, R₁ and R₂ are as stated above,
and
L represents a leaving group, preferably halogen, tri-(C₁₋₄ alkyl)-silyloxy, C₁₋₄ alkylsulfonyloxy, arylsulfonyloxy or C₁₋₄ alkylarylsulfonyloxy,
with an amine or thiol of the general formula
H-Z III
wherein
Z is as stated in claims 1 to 4,
and optionally in a manner known per se converting a 1,2,4-triazolo[1,5-a]pyrimidine derivative of the formula I thus obtained into a salt or converting a salt of the 1,2,4-triazolo[1,5-a]pyrimidine derivative of the general formula I thus obtained into the free base of formula I or into another salt.

2. A process according to claim 1, characterized in that 1,2,4-triazolo[1,5-a]pyrimidine derivatives are prepared, in which the halogen(s) which may be carried by the phenyl-(C₁₋₄ alkyl) group which may be represented by R₃ is/are chlorine and/or bromine.

3. A process according to claim 1 or 2, characterized in that 1,2,4-triazolo[1,5-a]pyrimidine derivatives are prepared, in which the C₁₋₄ alkyl substituent which may be carried by the morpholino, piperazino or piperidino group which may be represented by Q and/or the alkyl part(s) of the phenyl-(C₁₋₄ alkyl) group(s) which may be represented by R₃ and/or R₄ and/or R₅ and/or R₆ and/or R₇ and/or R₈ and/or the C₁₋₄ alkyl group(s) Of the di-(C₁₋₄ alkyl)-amino part(s) of the di-(C₁₋₄ alkyl)-amino-(C₁₋₆ alkyl group(s) which may be represented by R₄ and/or R₅ and/or the C₁₋₄ alkyl group(s) of the (C₁₋₄ alkyl)-amino parts of the (C₁₋₄ alkyl)-amino-(C₁₋₈ alkyl) group(s) and/or of the di-(C₁₋₄ alkyl)-amino-(C₁₋₈ alkyl) group(s) which may be represented by R₇ and/or Rₒ and/or the C₁₋₄ alkyl group which may be represented by R₉ or R₁₀ and/or the alkoxy part of the C₁₋₄ alkoxycarbonyl group which may be represented by R₁₀ and/or the C₁₋₄ alkyl group which may be represented by R₁ and/or R₂ is/are such one(s) having 1 or 2 carbon atom(s); and/or the C₁₋₈ alkyl group which may be represented by R₃ is such one having from 1 to 6, particularly from 1 to 3, carbon atom(s); and/or the C₂₋₆ alkenyl group(s) which may be represented by R₃ or R₄ and/or R₅ and/or R₇ and/or R₈ is/are such one(s) having from 2 to 4, particularly 3, carbon atoms, and/or the C₁₋₁₂ alkyl group which may be represented by R₄ and/or R₅ and/or R₇ and/or R₈ is/are such one[s] having from 1 to 8, particularly 1 to 6, carbon atom(s); and/or the C₁₋₆ alkyl group(s) of the amino-(C₁₋₆ alkyl) part(s) of the di-(C₁₋₄ alkyl)-amino-(C₁₋₆ alkyl) group(s) which may be represented by R₄ and/or R₅ is/are such one(s) having from 1 to 4 carbon atom(s); and/or the C₁₋₈ alkyl group(s) of the amino-(C₁₋₈ alkyl) part(s) of the (C₁₋₄ alkyl)-amino-(C₁₋₈ alkyl) and/or di-(C₁₋₄ alkyl)-amino-(C₁₋₈ alkyl) group(s) which may be represented by R₇ and/or R₈ is/are such one(s) having from 1 to 6 carbon atom(s);
and/or the C₃₋₈ cycloalkyl group(s) which may be represented by R₇ and/or R₈ is/are such one(s) having 5 or 6 carbon atom(s).

4. A process according to claims 1 to 3, characterized in that 1,2,4-triazolo[1,5-a]pyrimidine derivatives are prepared, in which
Q represents a morpholino, piperazino or piperidino group or a (C₁₋₆ alkyl)-thio group,
R₁ and R₂ independently each represent hydrogen or straight or branched chained C₁₋₄ alkyl or together form a 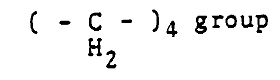 and
Z stands for a group of formula E, wherein R₇ and R₈ independently each represent straight or branched chained C₁₋₆ alkyl, which may optionally carry a morpholino, piperazino or piperidino group.

5. A process according to claims 1 to 4, characterized in that 2-(methylthio)-5-{N-[3'-(morpholino)-propyl]}-amino-6,7,8,9-tetrahydro-1,2,4-triazolo[5,1-b]quinazoline, 5-(diethylamino)-7-methyl-2-morpholino-1,2,4-triazolo[1,5-a]pyrimidine, 2-[1'-(methyl)-ethyl-thio]-5-{N-[3'-(morpholino)-propyl]}-amino-6,7,8,9-tetrahydro-1,2,4-triazolo[5,1-b]quinazoline, 2-(ethylthio)-5-{N-[2'-(morpholino)-ethyl]}-amino-6,7,8,9-tetrahydro-1,2,4-triazolo[5,1-b]quinazoline and 2-(methylthio)-5-{N-[2'-(morpholino)-ethyl]}-amino-6,7,8, 9,10,11,12,13,14,15-decahydro-cyclododeca[d]-1,2,4-triazolo[1,5-a]pyrimidine and salts thereof are prepared.

6. A process as claimed in claims 1 to 5, characterized by carrying out the reaction of the 1,2,4-triazolo[1,5-a]pyrimidine derivative of the general formula II with an excess of the amine of the general formula III.

7. A process as claimed in claims 1 to 6, characterized by carrying out the reaction of the 1,2,4-triazolo[1,5-a]pyrimidine derivative of the general formula II, wherein L represent halogen, C₁₋₄ alkylsulfonyloxy, arylsulfonyloxy or C₁₋₄ alkylarylsulfonyloxy, with the amine or thiol of the formula III in a protic, polar aprotic or apolar aprotic solvent, in the presence of a basic acid-binding agent.

## Patentansprüche (Patentansprüche für folgende(n) Vertragsstaat(en): AT, BE, CH, DE, FR, IT, LI, LU, MC, NL, PT, SE)

1. 1,2,4-Triazolo[1,5-a]pyrimidin-Derivate der allgemeinen Formel (I) 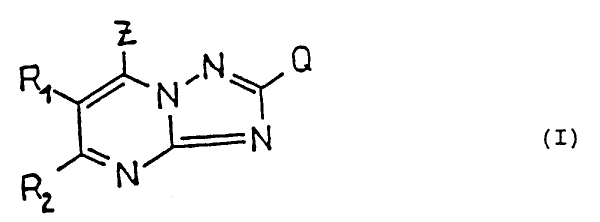 worin
Q Morpholino-, Piperazino- or Piperidinogruppe bedeutet, die gegebenenfalls einen C₁₋₄ Alkyl- oder Benzyl-Substituenten trägt;
oder eine Gruppe der Formel A bedeutet,
S(O)ₚ - R₃ (A)
worin
p 0, 1 oder 2 ist und
R₃ für eine gerade oer abgezweigte C₁₋₈ Alkylgruppe, C₂₋₆ Alkenylgruppe oder Phenyl-(C₁₋₄ alkyl)-gruppe steht, wobei die letztere gegebenenfalls einen oder mehrere Halogen- oder Nitrosubstituenten tragen kann, oder für eine gegebenenfalls durch eine oder mehrere Stickstoffafatom(e) und/oder Trifluormethylgruppe(n) substituierte Phenylgruppe bedeutet;
oder für eine Gruppe der Formel B steht, 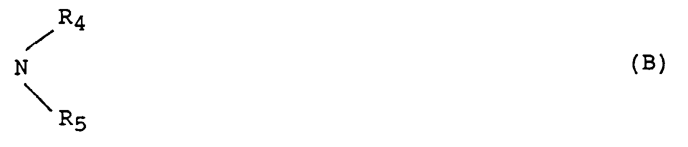 worin
R₄ und R₅ unabhängig voneinander Wasserstoff, gerade oder abgezweigte C₁₋₁₂ Alkylgruppe, C₂₋₆ Alkenylgruppe, Phenyl-(C₁₋₄ alkyl)gruppe inbegriffen solche, die mehr als eine Phenylgruppe enthalten, oder Di-(C₁₋₄ alkyl)-amino-(C₁₋₆ alkyl)-gruppe bedeuten,
R₁ und R₂ unabhängig voneinander Wasserstoff oder gerade oder abgezweigte C₁₋₄ Alkylgruppe bedeuten oder
R₁ und R₂ zusammen eine Gruppe der Formel C bedeuten
- (CH₂)ₙ - Y - (CH₂)ₘ - (C)
wobei in der letzteren Gruppe
Y für eine CH₂-Gruppe, ein Schwefelatom oder eine Gruppe der Formel D 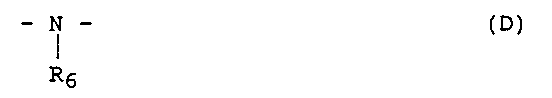 bedeutet, worin
R₆ Wasserstoff oder eine Phenyl-(C₁₋₄ alkyl)-gruppe bedeutet,
n und m 0, 1, 2, 3, 4 oder 5 bedeuten und
Z für eine Gruppe der Formel E 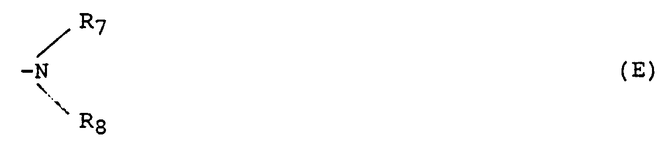 steht, und in der letzteren
R₇ und R⁸ unabhängig voneinander Wasserstoff, gerade oder abgezweigte C₁₋₁₂ Alkylgruppe bedeuten, die gegebenenfalls eine oder mehrere Substituenten gewählt aus der Gruppe Hydroxyl, Carboxyl, Amino, Morpholinocarbonyl, [4-(2'-{Hydroxy}-ethyl)-piperazin-1'-yl]-carbonyl und (C₁₋₄ Alkoxy)-carbonyl tragen oder gegebenenfalls eine Morpholino-, Piperazino- oder Piperidinogruppe haben, die gegebenenfalls durch eine C₁₋₄ Alkyl- oder Benzylgruppe substituiert ist; C₂₋₆ Alkenylgruppe, C₃₋₈ Cycloalkylgruppe, Adamantylgruppe oder Phenyl-(C₁₋₄ alkyl)gruppe, wobei die letztere gegebenenfalls einen oder mehreren Substituenten gewählt aus der Gruppe bestehend aus Halogen, Hydroxyl, C₁₋₄ Alkyl, C₁₋₄ Alkoxy, Amino und Nitro tragen kann; (C₁₋₄ Alkyl)-amino-(C₁₋₈ alkyl) und Di-(C₁₋₄ alkyl)-amino-(C₁₋₈ alkyl);
oder
R₇ und R₈ zusammen eine Gruppe der Formel F
- (CH₂)ⱼ - W - (CH₂)ₖ - (F)
bilden
- in der letzteren
j und k unabhänhig voneinander 1, 2 oder 3 bedeuten und
W für Säuerstoff oder eine -CH₂-Gruppe oder eine 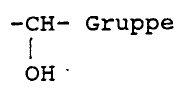 oder eine Gruppe der Formel G 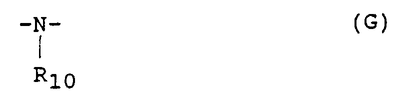 steht, und in der letzteren
R₁₀ für Wasserstoff, C₁₋₄ Alkoxycarbonyl oder C₁₋₄ Alkyl steht, und die letztere gegebenenfalls einen oder mehreren Substituenten gewählt aus der Gruppe bestehend aus Hydroxyl, (C₁₋₄ Alkyloxy)-carbonyl und Phenyl tragen kann;
oder
Z für eine Gruppe der Formel H
- S - R₉ (H)
steht, worin
R₉ eine C₁₋₄ Alkylgruppe bedeutet, die gegebenenfalls durch eine (C₁₋₄ Alkoxy)-carbonyl-gruppe substituiert ist, mit der Beschränkung, dass
a) falls Q eine Gruppe der Formel A bedeutet,
R₁ und R₂ Wasserstoff oder eine von C₁₋₄ Alkyl abweichende Bedeutung haben,
und
b) falls Q eine Morpholinogruppe bedeutet,
Z steht für eine Gruppe der Formel E,
worin
R₇ = R₈ = Wasserstoff
und
R₁ für Wasserstoff steht, dann
R₂ eine von Wasserstoff abweichende Bedeutung hat,
und ihre Salze.

2. 1,2,4-Triazolo[1,5-a]pyrimidin-Derivate nach Patentanspruch 1, dadurch gekennzeichnet, dass das/die Halogen(e), das/die durch die Phenyl-(C₁₋₄ alkyl)-gruppe der Formel R₃ getragen werden kann/können, Chlor und/oder Brom ist/sind.

3. 1,2,4-Triazolo[1,5-a]pyrimidin-Derivate nach Patentanspruch 1 oder 2, dadurch gekennzeichnet, dass der C₁₋₄ Alkyl-Substituent, der durch die Morpholino-, Piperazino- oder Piperidinogruppe der Formel Q getragen werden kann und/oder der/die Alkylteil(e) der Phenyl-(C₁₋₄ alkyl)gruppe(n) der Formel R₃ und/oder R₄ und/oder R₅ und/oder R₆ und/oder R₇ und/oder R₈ und/oder die C₁₋₄ Alkylgruppe(n) des Di-(C₁₋₄ alkyl)-amino-Teile(s) der Di-(C₁₋₄ alkyl)-amino(C₁₋₆ alkyl)-Gruppe(n) der Formel R₄ und/oder R₅ und/oder die C₁₋₄ Alkylgruppe(n) der (C₁₋₄ Alkyl)-amino-Teile der (C₁₋₄ Alkyl)-amino-(C₁₋₈ alkyl)-gruppe(n) und/oder der di-(C₁₋₄ Alkyl)-amino-(C₁₋₈ alkyl)-gruppe(n) der Formel R₇ und/oder R₈ und/oder die C₁₋₄ Alkylgruppe der Formel R₉ oder R₁₀ und/oder der Alkoxyteil der C₁₋₄ Alkoxycarbonylgruppe der Formel R₁₀ und/oder die C₁₋₄ Alkylgruppe der Formel R₁ und/oder R₂ 1 oder 2 Kohlenstoffatom(e) aufweist/aufweisen; und/oder die C₁₋₈ Alkylgruppe der Formel R₃ 1 bis 6, insbesondere 1 bis 3, Kohlenstoffatom(e) aufweist; und/oder die C₂₋₆ Alkenylgruppe(n) der Formel R₃ oder R₄ und/oder R₅ und/oder R₇ und/oder R₈ 2 bis 4, insbesondere 3, Kohlenstoffatome aufweist/aufweisen; und/oder die C₁₋₁₂ Alkylgruppe der Formel R₄ und/oder R₅ und/oder R₇ und/oder R₈ 1 bis 8, insbesondere 1 bis 6, Kohlenstoffatom(e) aufweist; und/oder die C₁₋₆ Alkylgruppe(n) des/der Amino-(C₁₋₆ alkyl)-Teile(s) der Di-(C₁₋₄ alkyl)-amino-(C₁₋₆ alkyl)-gruppe(n) der Formel R₄ und/oder R₅ 1 bis 4 Kohlenstoffatom(e) aufweist/aufweisen; und/oder die C₁₋₈ Alkylgruppe(n) des/der Amino-(C₁₋₈ alkyl)-Teile(s) der (C₁₋₄ Alkyl)-amino-(C₁₋₈ alkyl)- und/oder Di(C₁₋₄ alkyl)-amino-(C₁₋₈ alkyl)-gruppe(n) der Formel R₇ und/oder R₈ 1 bis 6 Kohlenstoffatom(e) aufweist/aufweisen; und/oder die C₃₋₈ Cycloalkylgruppe(n) der Formel R₇ und/oder R₈ 5 oder 6 Kohlenstoffatomen hat/haben.

4. 1,2,4-TriazoLo[1,5-a]pyrimidin-Derivate nach den Patentansprüchen 1 bis 3, dadurch gekennzeichnet, dass
Q eine Morpholino-, Piperazino- oder Piperidinogruppe oder eine (C₁₋₆ Alkyl)-thio-gruppe bedeutet,
R₁ und R₂ unabhängig voneinander Wasserstoffatom oder eine gerade oder abgezweigte C₁₋₄ Alkylgruppe bedeuten oder zusammen eine (-CH₂-)₄ Gruppe bilden und
Z für eine Gruppe der Formel E steht, worin R₇ und R₈ unabhängig voneinander eine gerade oder abgezweigte C₁₋₆ Alkylgruppe bedeuten, die gegebenenfalls eine Morpholino-, Piperazino- oder Piperidinogruppe tragen können.

5. 2-(Methylthio)-5-{N-[3'-(morpholino)-propyl]}-amino-6,7,8,9-tetrahydro-1,2,4-triazolo[5,1-b]chinazolin, 5-(Diethylamino)-7-methyl-2-morpholino-1,2,4-triazolo[1,5-a]pyrimidin, 2-[1'-(Methyl)-ethyl-thio]-5-{N-[3'-(morpholino)-propyl]}-amino-6,7,8,9-tetrahydro-1,2,4-triazolo[5,1--b]chinazolin, 2-(Ethylthio-5-{N-[2'-(morpholino)-ethyl]}-amino-6,7,8,9-tetrahydro-1,2,4-triazolo[5,1-b]chinazolin und 2-(Methylthio)-5-{N-[2'-(morpholino)-ethyl]}-amino-6,7,8,9,10,11,12,13,14,15-dekahydro-cyclododeca[d]-1,2,4-triazolo[1,5-a]pyrimidin und die Salze derselben.

6. Verfahren zur Herstellung der Verbindungen nach den Patentansprüchen 1 bis 5, dadurch gekennzeichnet, dass 1,2,4-Triazolo[1,5-a]pyrimidin-Derivate der allgemeinen Formel (II), 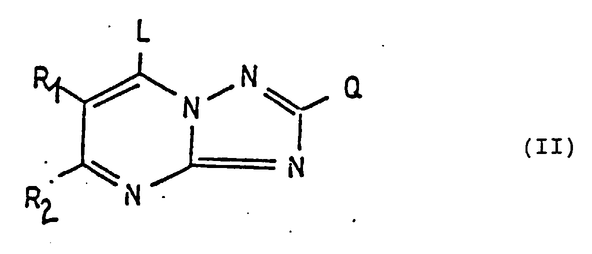 worin
Q, R₁ und R₂ die in den Patentansprüchen 1 bis 4 angegebenen Bedeutungen haben und
L eine austretende Gruppe gewählt aus der Gruppe bestehend aus Halogen, Tri-(C₁₋₄ alkyl)-sylyloxy, C₁₋₄ Alkylsulfonyloxy, Arylsulfonyloxy oder C₁₋₄ Alkylarylsulfonyloxy, bedeutet,
mit einem Amin oder Thiol der allgemeinen Formel (III),
H - Z (III)
worin
Z die in den Patentansprüchen 1 bis 4 angegebene Bedeutung hat,
und gegebenenfalls das derart erhaltene 1,2,4-Triazolo[1,5-a]pyrimidin-Derivat der Formel (I) in ein Salz, oder ein Salz des derart erhaltenen 1,2,4-Triazolo[1,5-a]pyrimidin-Derivates der allgemeinen Formel (I) in eine freie Base der Formel (I) oder in ein anderes Salz übergeführt wird.

7. Verfahren nach Patentanspruch 6, dadurch gekennzeichnet, dass die Umsetzung der 1,2,4-Triazolo[1,5-a]pyrimidin-Derivate der allgemeinen Formel (II) mit einem Überschuss des Amins der allgemeinen Formel (III) durchgeführt wird.

8. Verfahren nach Patentanspruch 6, dadurch gekennzeichnet, dass die Umsetzung des 1,2,4-Triazolo[1,5-a]pyrimidin-Derivates der allgemeinen Formel (II), worin L Halogen, C₁₋₄ Alkylsulfonyloxy, Arylsulfonyloxy oder C₁₋₄ Alkylarylsulfonyloxy bedeutet, mit dem Amin oder Thiol der Formel (III) in einem protischen, polar-aprotischen oder apolar-aprotischen Lösungsmittel, in Gegenwart eines basischen säurebindenden Mittels durchgeführt wird.

9. Arzneimittel, dadurch gekennzeichnet, dass sie als aktive(n) Komponente(n) mindestens eine Verbindung nach den Patentansprüchen 1 bis 5, zweckmässig mit mindestens einem inerten festen und/oder flüssigen pharmazeutischen Trägerstoff und/oder Verdünnungsmittel und/oder Zusatzstoff vermischt, enthalten.

10. Verwendung der Verbindungen nach den Patentansprüchen 1 bis 5 zur Herstellung von Arzneimitteln, insbesondere für solche, die positive inotropische, antianginale, entzündungshemmende und Ulcus- und Magensäuresekretion-inhibierende Wirkung aufweisen.

## Patentansprüche (Patentansprüche für folgende(n) Vertragsstaat(en): ES, GR)

1. Verfahren zur Herstellung von 1,2,4-Triazolo[1,5-a]pyrimidin-Derivaten der allgemeinen Formel (I) 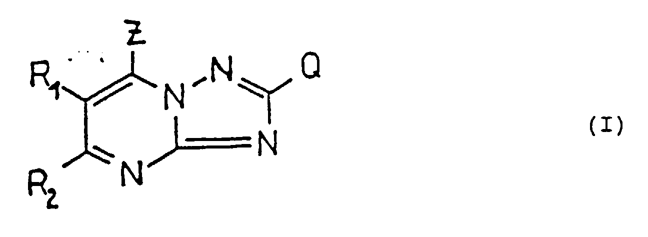 worin
Q Morpholino-, Piperazino- or Piperidinylgruppe bedeutet, die gegebenenfalls einen C₁₋₄ Alkyl- oder Benzyl-Substituenten trägt;
oder eine Gruppe der Formel A bedeutet,
S(O)ₚ - R₃ (A)
worin
p 0, 1 oder 2 ist und
R₃ für eine gerade oer abgezweigte C₁₋₈ Alkylgruppe, C₂₋₆ Alkenylgruppe oder Phenyl-(C₁₋₄ alkyl)-gruppe steht, wobei die letztere gegebenenfalls eine oder mehrere Halogen- oder Nitrosubstituenten oder eine gegebenenfalls durch eine oder mehrere Stickstoffafatom(e) und/oder Trifluormethylgruppe(n) substituierte Phenylgruppe bedeutet;
oder für eine Gruppe der Formel B steht, 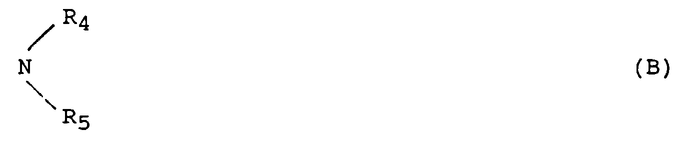 worin
R₄ und R₅ unabhängig voneinander Wasserstoff, gerade oder abgezweigte C₁₋₁₂ Alkylgruppe, C₂₋₆ Alkenylgruppe, Phenyl-(C₁₋₄ alkyl)gruppe inbegriffen solche, die mehr als eine Phenylgruppe enthalten, oder Di-(C₁₋₄ alkyl)-amino-(C₁₋₆ alkyl)-gruppe bedeuten,
R₁ und R₂ unabhängig voneinander Wasserstoff oder gerade oder abgezweigte C₁₋₄ Alkylgruppe bedeuten oder
R₁ und R₂ zusammen eine Gruppe der Formel C bedeuten
-(CH₂)ₙ - Y - (CH₂)ₘ - (C)
wobei in der letzteren Gruppe
Y für eine CH₂-Gruppe, ein Schwefelatom oder eine Gruppe der Formel D 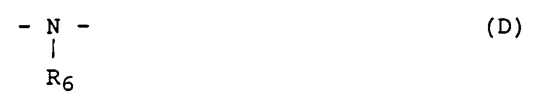 bedeutet, worin
R₆ Wasserstoff oder eine Phenyl-(C₁₋₄ alkyl)-gruppe bedeutet,
n und m 0, 1, 2, 3, 4 oder 5 bedeuten und
Z für eine Gruppe der Formel E 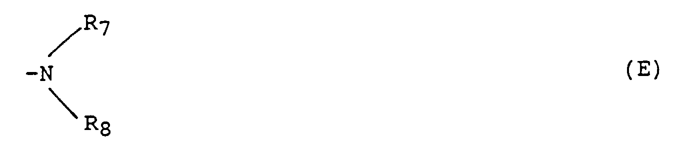 steht, und in den letzteren
R₇ und R⁸ unabhängig voneinander Wasserstoff, gerade oder abgezweigte C₁₋₁₂ Alkylgruppe bedeuten, die gegebenenfalls eine oder mehrere Substituenten gewählt aus der Gruppe Hydroxyl, Carboxyl, Amino, Morpholinocarbonyl, [4-(2'-{Hydroxy}-ethyl)-piperazin-1'-yl]-carbonyl und (C₁₋₄ Alkoxy)-carbonyl tragen oder gegebenenfalls eine Morpholino-, Piperazino- oder Piperidinogruppe haben, die gegebenenfalls durch eine C₁₋₄ Alkyl- oder Benzylgruppe substituiert ist; C₂₋₆ Alkenylgruppe, C₃₋₈ Cycloalkylgruppe, Adamantylgruppe oder Phenyl-(C₁₋₄ alkyl)gruppe, wobei die letztere gegebenenfalls einen oder mehreren Substituenten gewählt aus der Gruppe bestehend aus Halogen, Hydroxyl, C₁₋₄ Alkyl, C₁₋₄ Alkoxy, Amino und Nitro tragen kann; (C₁₋₄ Alkyl)-amino-C(₁₋₈ alkyl) und Di-(C₁₋₄ alkyl)-amino-(C₁₋₈ alkyl);
oder
R₇ und R₈ zusammen eine Gruppe der Formel F
- (CH₂)ⱼ - W - (CH₂)ₖ - (F)
bilden
- in der letzteren
j und k unabhänhig voneinander 1, 2 oder 3 bedeuten und
W für Säuerstoff oder eine -CH₂-Gruppe oder eine 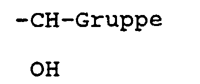 oder eine Gruppe der Formel G 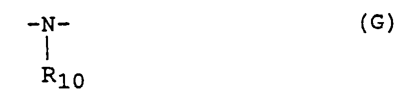 steht, und in der letzteren
R₁₀ für Wasserstoff, C₁₋₄ Alkoxycarbonyl oder C₁₋₄ Alkyl steht, und die letztere gegebenenfalls einen oder mehreren Substituenten gewählt aus der Gruppe bestehend aus Hydroxyl, (C₁₋₄ Alkyloxy)-carbonyl und Phenyl tragen kann;
oder
Z für eine Gruppe der Formel H
- S - R₉ (H)
steht, worin
R₉ eine C₁₋₄ Alkylgruppe bedeutet, die gegebenenfalls durch eine (C₁₋₄ Alkoxy)-carbonyl-gruppe substituiert ist, mit der Beschränkung, dass
a) falls Q eine Gruppe der Formel A bedeutet,
R₁ und R₂ Wasserstoff oder eine von C₁₋₄ Alkyl abweichende Bedeutung haben,
und
b) falls Q eine Morpholinogruppe bedeutet,
Z steht für eine Gruppe der Formel E,
worin
R₇ = R₈ = Wasserstoff
und
R₁ für Wasserstoff steht, dann
R₂ eine von Wasserstoff abweichende Bedeutung hat,
und ihren Salzen, dadurch gekennzeichnet, dass 1,2,4-Triazolo[1,5-a]pyrimidin-Derivate der allgemeinen Formel (II), 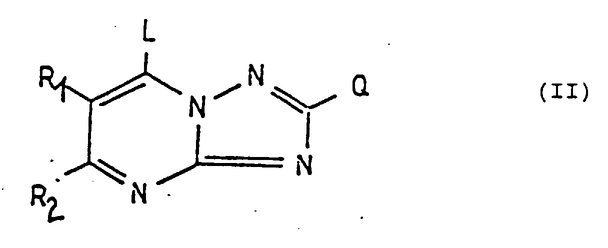 worin
Q, R₁ und R₂ die den in Patentansprüchen 1 bis 4 angegebenen Bedeutungen haben
und
L eine austretende Gruppe bedeutet, die aus der Gruppe bestehend aus Halogen, Tri-(C₁₋₄ Alkyl)-sylyloxy, C₁₋₄ Alkylsulfonyloxy, Arylsulfonyloxy oder C₁₋₄ Alkyl-arylsulfonyloxy gewählet ist,
mit einem Amin oder Thiol der allgemeinen Formel (III),
H - Z (III)
worin
Z die in den Patentansprüchen 1 bis 4 angegebene Bedeutung hat,
umgesetzt werden,
und gegebenenfalls ein derart erhaltenes 1,2,4-Triazolo[1,5-a]pyrimidin-Derivat der allgemeinen Formel (I) in ein Salz, oder ein Salz des derart erhaltenes des 1,2,4-Triazolo[1,5-a]pyrimidin-Derivates der allgemeinen Formel (I) in eine freie Base der allgemeinen Formel (I) oder in ein anderes Salz übergeführt wird.

2. Verfahren nach Patentanspruch 1, dadurch gekennzeichnet, dass 1,2,4-Triazolo[1,5-a]pyrimidin-Derivate hergestellt werden, in welchen das/die Halogen(e), das/die die durch die Phenyl-(C₁₋₄alkyl)-gruppe der Formel R₃ getragen werden können, Chlor und/oder Brom ist/sind.

3. Verfahren nach Patentanspruch 1 oder 2, dadurch gekennzeichnet, dass 1,2,4-Triazolo[1,5-a]pyrimidin-Derivate hergestellt werden, in welchen der C₁₋₄ Alkyl Substituent, der durch die Morpholino-, Piperazino- oder Piperidinogruppe der Formel Q und/oder der/die Alkylteil(e) der Phenyl-(C₁₋₄ alkyl)-gruppe(n) der Formel R₃ und/oder R₄ und/oder R₅ und/oder R₆ und/oder R₇ und/oder R₈ und/oder die C₁₋₄ Alkyl-gruppe(n) des/der Di-(C₁₋₄ Alkyl)-amino-Teile(s) der Di-(C₁₋₄ alkyl)-amino-(C₁₋₆ alkyl)-gruppe(n) der Formel R₄ und/oder R₅ und/oder die C₁₋₄ Alkylgruppe(n) des (C₁₋₄ Alkyl)-amino-Teiles der (C₁₋₄ Alkyl)-amino-(C₁₋₈ alkyl)-gruppe(n) und/oder der Di-(C₁₋₄ Alkyl)-amino-(C₁₋₈ alkyl)-gruppe(n) der Formel R₇ und/oder R₈ und/oder die C₁₋₄ Alkylgruppe der Formel R₉ oder R₁₀ und/oder der Alkoxyteil der C₁₋₄ Alkoxycarbonyl-Gruppe der Formel R₁₀ und/oder die C₁₋₄ Alkylgruppe der Formel R₁ und/oder R₂ 1 oder 2 Kohlenstoffatom(e) aufweist/aufweisen; und/oder die C₁₋₈ Alkylgruppe der Formel R₃ 1 bis 6, insbesondere 1 bis 3, Kohlenstoffatom(e) aufweist; und/oder die C₂₋₆ Alkenylgruppe(n) der Formel R₃ und/oder R₄ und/oder R₅ und/oder R₇ und/oder R₈ 2 bis 4, insbesondere 3, Kohlenstoffatome aufweist/aufweisen, und/oder die C₁₋₁₂ Alkylgruppe der Formel R₄ und/oder R₅ und/oder R₇ und/oder R₈ 1 bis 8, insbesondere 1 bis 6, Kohlenstoffatome aufweist; und/oder die C₁₋₆ Alkylgruppe(n) des/der Amino(C₁₋₆ alkyl)-Teile(s) der Di-(C₁₋₄ alkyl)-amino-(C₁₋₆ alkyl)gruppe(n) der Formel R₄ und/oder R₅ 1 bis 4 Kohlenstoffatom(e) aufweist/aufweisen; und/oder die c₁₋₈ Alkylgruppe(n) des/der (Amino-(C₁₋₈ alkyl)-Teile(s) der C₁₋₄ Alkyl)-amino(C₁₋₈ alkyl)- und/oder di-(C₁₋₄ Alkyl)-amino-(C₁₋₈ alkyl)Gruppe(n) der Formel R₇ und/oder R₈ 1 bis 6 Kohlenstoffatom(e) aufweist/aufweisen; und/oder die C₃₋₈ Cycloalkylgruppe(n) der Formel R₇ und/oder R₈ 5 oder 6 Kohlenstoffatome hat/haben.

4. Verfahren nach den Patentansprüchen 1 bis 3, dadurch gekennzeichnet, dass 1,2,4-Triazolo[1,5-a]pyrimidin-Derivate hergestellt werden, worin
Q eine Morpholino-, Piperazino- oder Piperidinogruppe oder eine (C₁₋₆ Alkyl)-thio-gruppe bedeutet,
R₁ und R₂ unabhängig voneinander Wasserstoffatom oder eine gerade oder abgezweigte C₁₋₄ Alkylgruppe bedeuten oder zusammen eine (-CH₂-)₄ Gruppe bilden und
Z für eine Gruppe der Formel E steht, worin R₇ und R₈ unabhängig voneinander eine gerade oder abgezweigte C₁₋₆ Alkylgruppe bedeuten, die gegebenenfalls eine Morpholino-, Piperazino- oder Piperidinogruppe tragen kann.

5. Verfahren nach den Patentansprüchen 1 bis 4, dadurch gekennzeichnet, dass 2-(Methylthio)-5-{N-[3'-(morpholino)--propyl]}-amino-6,7,8,9-tetrahydro-1,2,4-triazolo[5,1-b]chinazolin, 5-(Di-ethylamino)-7-methyl-2-morpholino-1,2,4-triazolo[1,5-a]pyrimidin, 2-[1'-(Methyl)-ethyl-thio]-5-{N-[3'-(morpholino)-propyl]}-amino-6,7,8,9-tetrahydro-1,2,4-triazolo[5,1-b]chinazolin, 2-(Ethylthio-5-{N-[2'-(morpholino)-ethyl]}-amino-6,7,8,9-tetrahydro-1,2,4-triazolo[5,1-b]chinazolin und 2-(Methylthio)-5-{N-[2'-(morpholino)-ethyl]}-amino-6,7,8,9,10,11,12,13,14,15-dekahydro-cyclododeca[d]-1,2,4-triazolo[1,5-a]pyrimidin und deren Salze hergestellt werden.

6. Verfahren nach den Patentansprüchen 1 bis 5, dadurch gekennzeichnet, dass die Umsetzung des 1,2,4-Triazolo[1,5--a]pyrimidin-Derivates der allgemeinen Formel (II) mit einem Überschuss des Amins der allgemeinen Formel (III) durchgeführt wird.

7. Verfahren nach den Patentansprüchen 1 bis 6, dadurch gekennzeichnet, dass die Umsetzung des 1,2,4-Triazolo[1,5-a]pyrimidin-Derivates der allgemeinen Formel (II), worin L Halogen, C₁₋₆ Alkylsulfonyloxy, Arylsulfonyloxy oder C₁₋₄ Alkylarylsulfonyloxy bedetutet, mit dem Amin oder Thiol der Formel (III) in einem protischen, polar-aproti'schen oder apolar-aprotischen Lösungsmittel, in Gegenwart eines basischen Säurebindemittels durchgeführt wird.

## Revendications (Revendications pour l'(les) Etat(s) contractant(s) suivant(s): AT, BE, CH, DE, FR, IT, LI, LU, MC, NL, PT, SE)

1. Dérivés de 1,2,4 - triazolo[1,5 - a]pyrimidine de formule générale (1) 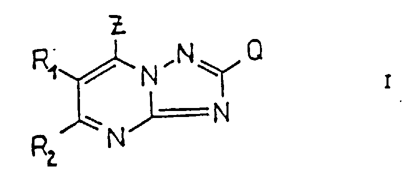 dans laquelle
Q représente un groupe morpholino, pipérazino ou pipéridinyle, qui porte éventuellement un substituant alkyle en C 1-4 ou benzyle;
ou un groupe de formule A
S(O)ₚ-R3
dans laquelle
p est 0,1 ou 2
et
R₃ représente un reste alkyle linéaire ou ramifié,
ayant de 1 à 8 atomes de carbone, alkényle ayant de 2 à 6 atomes de carbone ou phényl(alkyl en C 1-4), ce dernier pouvant porter éventuellement un ou plusieurs substituant(s) halogène ou nitro ou phényle éventuellement substitué par un ou plusieurs atome(s) d'azote et/ou groupe(s) trifluorométhyle;
ou un groupe de la formule B, 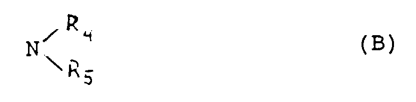 dans laquelle
R₄ et R₅ représentent indépendamment l'un de l'autre de l'hydrogène, un reste alkyle linéaire ou ramifié ayant de 1 à 12 atomes de carbone, alkényle ayant de 2 à 6 atomes de carbone, phényl-(alkyl en C 1-4) comportant ceux qui ont plus d'un groupe phényle, ou di-(alkyl en C 1-4) -amino-(alkyl en C 1-6),
R₁ et R₂ représentent indépendamment l'un de l'autre un atome d'hydrogène ou un reste alkyle linéaire ou ramifié, ayant de 1 à 4 atomes de carbone ou
R₁ et R₂ forment ensemble un groupe de formule C,
- (CH₂) - Y - (CH₂) - (C)
où dans ce dernier
Y signifie un groupe CH₂, un atome de soufre ou un groupe de formule D 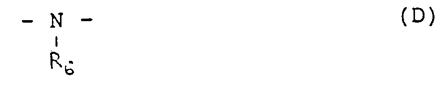 dans laquelle
R₆ représente un atome d'hydrogène ou un reste phényl-(alkyl en C 1-4),
n et m représentent chacun 0, 1, 2, 3, 4 ou 5
et
Z signifie un groupe de formule E 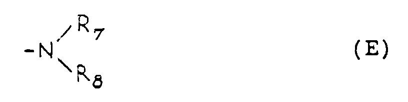 et dans la dernière
R₇ et R₈ signifient indépendamment l'un de l'autre de l'hydrogène, un alkyle linéaire ou ramifié ayant de 1 à 12 atomes de carbone, qui éventuellement peut porter un ou plusieurs substituant (s) choisi(s) dans le groupe hydroxyle, carboxyle, amino, morpholinocarbonyle, [4-(2'-(hydroxy)-éthyl)-pipérazine-1'-yle]-carbonyle et (alkyl en C 1-4)-carbonyle, ou ils ont un groupe morpholino, pipérazino ou pipéridino pouvant être substitué par un groupe alkyle ayant de 1 à 4 atomes de carbone ou benzyle; un groupe alkényle ayant de 2 à 6 atomes de carbone, un groupe cycloalkyle ayant de 3 à 8 atomes de carbone, un groupe adamantyle ou un groupe phényl-(alkyl en C 1-4), dans lequel ce dernier peut éventuellement porter un ou plusieurs substituant(s) choisi(s) dans le groupe halogène, hydroxyle, alkyle ayant de 1 à 4 atomes de carbone, alkoxy ayant de 1 à 4 atomes de carbone, amino et nitro;
(alkyl en C 1-4)-amine-(alkyl en C 1-8) et di-(alkyl en C 1-4)-amino-(alkyl en C 1-8); ou
R₇ et R₈ signifient ensemble un groupe de formule F
- (CH₂)j - W - (CH₂)k - (F)
dans laquelle
j et k signifient indépendammant l'un de l'autre 1, 2 ou 3 et
W signifie de l'oxygène ou un groupe -CH₂- ou un groupe - CH(OH)- ou un groupe de formule G 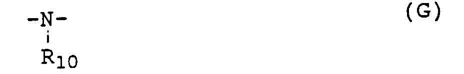 et dans cette dernière
R₁₀ signifie de l'hydrogène, alkoxycarbonyle ayant de 1 à 4 atomes de carbone ou alkyle ayant de 1 à 4 atomes de carbone, ce dernier pouvant porter éventuellement un ou plusieurs substituant (s) choisi(s) dans le groupe composé d'hydroxyle, (alkyl en C 1-4 oxy)-carbonyle et phényle;
ou
Z signifie un groupe de fromule H
- S - R9 (H)
dans laquelle
R9 signifie un groupe alkyle ayant de 1 à 4 atomes de carbone, qui éventuellement peut être substitué par un groupe (alkyl en C 1-4 oxy)-carbonyle,
avec la condition supplémentaire que
a) si Q signifie un groupe de formule A,
R₁ et R₂ signifient de l'hydrogène ou ont une signification différente d'alkyle ayant de 1 à 4 atomes de carbone,
et
b) si Q signifie un groupe morpholino,
Z est un groupe de formule E,
dans laquelle
R₇ = R₈ = hydrogène
et
R₁ signifie de l'hydrogène, R₂ a une signification différente d'hydrogène et leurs sels.

2. Dérivés de 1,2,4 - triazolo[1,5 - a]pyrimidine suivant la revendication 1, caractérisé en ce que le(s) atome(s) d'halogène(s) pouvant être porté(s) par le groupe phényl-(alkyl en C 1-4) de la formule R₃ est/sont de chlore et/ou de brome.

3. Procédé suivant la revendication 1 ou 2, caractérisé en ce que l'on prépare des dérivés de 1,2,4 - triazolo[1,5 - a]pyrimidine dans lesquels le substituant alkyle ayant de 1 à 4 atomes de carbone pouvant être porté par le groupe morpholino, pipérazino ou pipéridino de formule Q et/ou la/les partie(s) alkyliques du/des groupe(s) phényl-(alkyl en C 1-4) de formule R₃ et/ou R₄ et/ou R₅ et/ou R₆ et/ou R₇ et/ou R₈ et/ou le(s) groupe(s) alkyle(s) ayant de 1 à 4 atomes de carbone de la/des partie(s) di-(alkyl en C 1-4)-aminique(s) de/des groupe(s) di-(alkyle en C 1-4)-amino-(alkyl en C 1-6) de formule R₄ et/ou R₅ et/ou le(s) groupe(s) alkyle(s) ayant de 1 à 4 atomes de carbone des parties alkyles en C 1-4 du/des groupe(s) (alkyl en C 1-4)-amino-(alkyl en C 1-8) et/ou du/des groupe(s) di-(alkyl en C 1-4)-amino-(alkyl en C 1-8) de formule R₇ et/ou R₈ et/ou le groupe alkyle ayant de 1 à 4 atomes de carbone de formule R₉ ou R₁₀ et/ou la partie alkoxy du groupe (alkyl en C 1-4 oxy)-carbonyle de formule R₁₀ et/ou le groupe alkyle ayant de 1 à 4 atomes de carbone de formule R₁ et/ou R₂ a/ont 1 ou 2 atome(s) de carbone; et/ou le groupe alkyle ayant de 1 à 8 atomes de carbone de formule R₃ a 1 à 6 et en particulier 1 à 3 atome(s) de carbone; et/ou le(s) groupe(s) alkényle(s) ayant de 2 à 6 atomes de carbone de fromule R₃ ou R₄ et/ou R₅ et/ou R₇ et/ou R₈ a/ont 2 à 4 et spécialement 3 atomes de carbone; et/ou le groupe alkyle ayant de 1 à 12 atomes de carbone de formule R₄ et/ou R₅ et/ou R₇ et/ou R₈ a/ont 1 à 8 et spécialement 1 à 6 atome(s) de carbone; et/ou le(s) groupe(s) alkyle(s) ayant de 1 à 6 atome(s) de carbone de la (des) partie(s) amino-(alkyl en C 1-6) du/des groupe(s) di-(alkyl en C 1-4)-amino-(alkyl en C 1-6) de formule R₄ et/ou R₅ a/ont 1 à 4 atome(s) de carbone; et/ou le/les groupe(s) alkyle(s) ayant 1 à 8 atomes de carbone de la (des) partie(s) amino-(alkyl en C 1-8) du/des groupe(s) (alkyl en C 1-4)-amino-(alkyl en C 1-8) et/ou di-(alkyl en C 1-4)-amine-(alkyle en C 1-8) de formule R₇ et/ou R₈ a/ont de 1 à 6 atome(s) de carbone; et/ou le(s) groupe(s) cycloalkylique(s) ayant de 3 à 8 atomes de carbone de formule R₇ et/ou R₈ a/ont 5 ou 6 atomes de carbone.

4. Dérivés de 1,2,4 - triazolo[1,5 - a]pyrimidine suivant les revendications 1 à 3, caractérisés en ce que
Q signifie un groupe morpholino, pipérazino ou pipéridino ou un groupe (alkyl en C 1-6)-thio,
R₁ et R₂ signifient indépendamment l'un de l'autre un atome d'hydrogène ou un groupe alkyle linéaire ou ramifié ayant de 1 à 4 atomes de carbone, ou ensemble ils forment un groupe (-CH₂-)₄ et
Z signifie un groupe de formule E, dans lequel R₇ et R₈ signifient indépendamment l'un de l'autre un groupe alkyle linéaire ou ramifié ayant de 1 à 6 atomes de carbone, ces derniers pouvant porter éventuellement un groupe morpholino, pipérazino ou pipéridino.

5. 2-(méthylthio)-5-[N-(3'-(morpholino)-propyl)]-amino-6,7,8,9-tétrahydro-1,2,4-triazolo[5,1-b]quinazoline, 5-(diéthylamino)-7-méthyl-2-morpholino-1,2,4-triazolo[1,5-a]pyrimidine, 2-[1'-(méthyl)-éthyl-thio]-5-(N-[3'-(morpholino)-propyl])-amino-6,7,8,9-tétrahydro-1,2,4-triazolo[5,1-b]quinazoline, 2-(éthylthio-5-(N-[2'-(morpholino)-éthyl])-amino-6,7,8,9-tétrahydro-1,2,4-triazolo[5,1-b]quinazoline et 2-(métylthio)-5-(N-[2'-(morpholino)-éthyl])-amino-6,7,8,9,10,11,12,13,14,15-décahydro-cyclododéca[d]-1,2,4-triazolo[1,5-a]pyrimidine et leurs sels.

6. Procédé pour la préparation des composés suivant les revendications 1 à 5, caractérisé en ce que l'on fait réagir des dérivés de 1,2,4 - triazolo[1,5 - a]pyrimidine de formule générale (II), 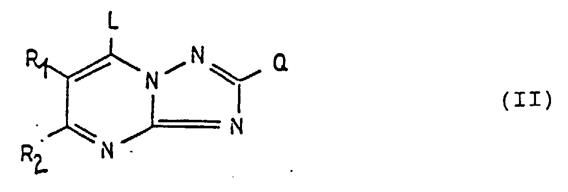 dans laquelle
Q, R₁ et R₂ ont la même signification que dans les revendications 1 à 4
et
L signifie un groupe sortant choisi dans le groupe composé d'un atome d'halogène, tri-(alkyl en C 1-4)-sylyloxy, (alkyl en C 1-4)-sulfonyloxy, arylsulfonyloxy ou (alkyl en C 1-4)-arylsulfonyloxy,
avec une amine ou un thiol de formule générale (III),
H - Z (III)
dans laquelle
Z a la même signification que dans les revendications 1 à 4,
et l'on transforme éventuellement le dérivé de 1,2,4 - triazolo[1,5 - a]pyrimidine de fromule (I) ainsi obtenu en un sel, ou un sel des dérivés de 1,2,4 - triazolo[1,5 - a]pyrimidine de fromule générale (I) ainsi obtenu en une base de formule (I) ou en un autre sel.

7. Procédé suivant la revendication 6, caractérisé en ce que la conversion des dérivés de 1,2,4 - triazolo[1,5 - a]pyrimidine de formule générale (II) est réalisée avec un excès des amines de la formule générale (III).

8. Procédé suivant la revendication 6, caractérisé en ce que la conversion des dérivés de 1,2,4 - triazolo[1,5 - a]pyrimidine de la formule générale (II), dans laquelle L signifie un atome d'halogène, un groupe (alkyl en C 1-4)-sulfonyloxy, arylsulfonyloxy ou (alkyl en C 1-4)-arylsulfonyloxy, avec l'amine ou le thiol de formule (III) est réalisée dans un solvant protique, polaire-aprotique ou apolaire-aprotique, en la présence d'un agent basique liant les acides.

9. Médicaments caractérisés en ce qu'ils contiennent comme ingrédient(s) actif(s) au moins un composé selon les revendications 1 à 5, de préférence mélangés à au moins un support inerte solide et/ou liquide pharmaceutique et/ou un diluant et/ou un additif.

10. Utilisation des composés selon les revendications 1 à 5 pour préparer des médicaments, en particulier ceux ayant un effet inotropique positif antianginal anti-inflammatoire et inhibant l'ulcère et la sécrétion d'acide gastrique.

## Revendications (Revendications pour l'(les) Etat(s) contractant(s) suivant(s): ES, GR)

1. Procédé pour la préparation des dérivés de 1,2,4 - triazolo[1,5 - a]pyrimidine de formule générale (1) 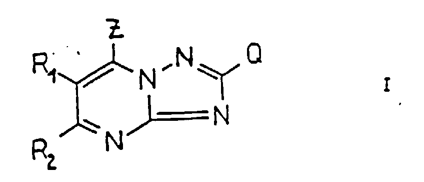 dans laquelle
Q représente un groupe morpholino, pipérazino ou pipéridinyle, qui porte éventuellement un substituant alkyle en C 1-4 ou benzyle;
ou un groupe de formule A
S(O)ₚ-R₃ (A)
dans laquelle
p est 0,1 ou 2
et
R₃ représente un reste alkyle linéaire ou ramifié,
ayant de 1 à 8 atomes de carbone, alkényle ayant de 2 à 6 atomes de carbone ou phényl-(alkyl en C 1-4),
ce dernier pouvant porter éventuellement un ou plusieurs substituant(s) halogène ou nitro ou phényle éventuellement substitué par un ou plusieurs atome(s) d'azote et/ou un/des groupe(s) trifluorométhyle;
ou un groupe de la formule B, 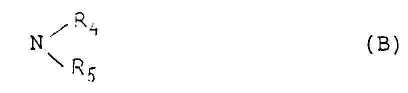 dans laquelle
R₄ et R₅ représentent indépendamment l'un de l'autre de l'hydrogène, un reste alkyle linéaire ou ramifié ayant de 1 à 12 atomes de carbone, alkényle ayant de 2 à 6 atomes de carbone, phényl-(alkyl en C 1-4) comportant ceux qui ont plus d'un groupe phényle, ou di-(alkyl en C 1-4)-amino-(alkyl en C 1-6),
R₁ et R₂ représentent indépendamment l'un de l'autre un atome d'hydrogène ou un reste alkyle linéaire ou ramifié, ayant de 1 à 4 atomes de carbone ou
R₁ et R₂ forment ensemble un groupe de formule C,
- (CH₂) - Y - (CH₂) - (C)
où dans ce dernier
Y signifie un groupe CH₂, un atome de soufre ou un groupe de formule D 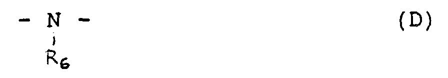 dans laquelle
R₆ représente un atome d'hydrogène ou un reste phényl-(alkyl en C 1-4),
n et m représentent chacun 0, 1, 2, 3, 4 ou 5
et
Z signifie un groupe de formule E 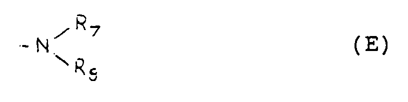 et dans cette dernière
R₇ et R₈ signifient indépendamment l'un de l'autre de l'hydrogène, un alkyle linéaire ou ramifié ayant de 1 à 12 atomes de carbone, qui éventuellement peut porter un ou plusieurs substituant(s) choisi(s) dans le groupe hydroxyle, carboxyle, amino, morpholinocarbonyle, [4-(2'-(hydroxy)-éthyl)-pipérazine-1'-yle]-carbonyle et (alkyl en C 1-4)-carbonyle, ou ils ont un groupe morpholino, pipérazino ou pipéridino pouvant être substitué par un groupe alkyle ayant de 1 à 4 atomes de carbone ou benzyle; un groupe alkényle ayant de 2 à 6 atomes de carbone, un groupe cycloalkyle ayant de 3 à 8 atomes de carbone, un groupe adamantyle ou un groupe phényl-(alkyl en C 1-4), dans lequel ce dernier peut éventuellement porter un ou plusieurs substituant(s) choisi(s) dans le groupe halogène, hydroxyle, alkyle ayant de 1 à 4 atomes de carbone, alkoxy ayant de 1 à 4 atomes de carbone, amino et nitro; (alkyl en C 1-4)-amino-(alkyl en C 1-8) et di-(alkyl en C 1-4)-amino-(alkyle en C 1-8);
ou
R₇ et R₈ signifient ensemble un groupe de formule F
- (CH₂)j - W - (CH₂)k - (F)
dan laquelle
- j et k signifient indépendammant l'un de l'autre 1, 2 ou 3
et
W signifie de l'oxygène ou un groupe -CH₂- ou un groupe - CH(OH)- ou un groupe de formule G 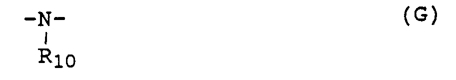 et dans cette dernière
R₁₀ signifie de l'hydrogène, un groupe alkoxycarbonyle ayant de 1 à 4 atomes de carbone ou alkyle ayant de 1 à 4 atomes de carbone, ce dernier pouvant porter éventuellement un ou plusieurs substituant(s) choisi(s) dans le groupe composé d'hydroxyle, (alkyle en C 1-4 oxy)-carbonyle et phényle;
ou
Z signifie un groupe de fromule H
- S - R₉ (H)
dans laquelle
R₉ signifie un groupe alkyle ayant de 1 à 4 atomes de carbone, qui éventuellement peut être substitué par un groupe (alkyl en C 1-4 oxy)-carbonyle,
avec la condition supplémentaire que
a) si Q signifie un groupe de formule A,
R₁ et R₂ signifient de l'hydrogène ou ont une signification différente d'alkyle ayant de 1 à 4 atomes de carbone,
et
b) si Q signifie un groupe mrpholino,
Z est un groupe de formule E,
dans laquelle
R₇ = R₈ = hydrogène
et
R₁ signifie de l'hydrogène,
R₂ a une signification différente de l'hydrogène
et leurs sels, caractérisé en ce que l'on fait réagir des dérivés de 1,2,4 - triazolo[1,5 - a]pyrimidine de formule générale (II), 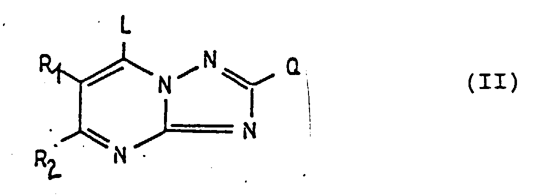 dans laquelle
Q, R₁ et R₂ ont la même signification que dans les revendications 1 à 4
et
L signifie un groupe sortant choisi dans le groupe composé d'un atome d'halogène ou d'un groupe tri-(alkyl en C 1-4)-syloxy, (alkyl en C 1-4)-sulfonyloxy, arylsulfonyloxy ou (alkyl en C 1-4)-arylsulfonyloxy,
avec une amine ou un thiol de formule générale (III),
H - Z (III)
dans laquelle
Z a la même signification que dans les revendications 1 à 4, et l'on transforme un dérivé de 1,2,4 - triazolo[1,5 - a]pyrimidine de formule générale (I) ainsi obtenu en un sel ou l'on transforme un sel des dérivés de 1,2,4 - triazolo[1,5 - a]pyrimidine de formule générale (I) ainsi obtenu en une base libre de formule générale (I) ou en un autre sel.

2. Procédé selon la revendication 1, caractérisé en ce que l'on prépare des dérivés de 1,2,4 - triazolo[1,5 - a]pyrimidine, dans lesquels le(s) halogène(s) porté(s) par le groupe phényl-(alkyl en C 1-4) de formule R3 est/sont un/des atome(s) de chlore/brome.

3. Procédé suivant les revendications 1 ou 2, caractérisé en ce que l'on prépare des dérivés de 1,2,4 - triazolo[1,5 - a]pyrimidine, dans lesquels le substituant alkyle ayant de 1 à 4 atomes de carbone pouvant être porté par le groupe morpholino, pipérazino ou pipéridino de formule Q et/ou la/les partie(s) alkyliques du/des groupe(s) phényl-(alkyl en C 1-4) de formule R₃ et/ou R₄ et/ou R₅ et/ou R₆ et/ou R₇ et/ou R₈ et/ou le(s) groupe(s) alkyle(s) ayant de 1 à 4 atomes de carbone de la/les partie(s) di-(alkyl en C 1-4)-aminique(s) du/des groupe(s) di-(alkyl en C 1-4)-amino-(alkyl en C 1-6) de formule R₄ et/ou R₅ et/ou le(s) groupe(s) alkyle(s) ayant de 1 à 4 atomes de carbone des parties (alkyl en C 1-4) du/des groupe(s) (alkyl en C 1-4)-amino-(alkyl en C 1-8) et/ou du/des groupe(s) di-(alkyl en C 1-4)-amino-(alkyl en C 1-8) de formule R₇ et/ou R₈ et/ou le groupe alkyle ayant de 1 à 4 atomes de carbone de formule R₉ ou R₁₀ et/ou la partie alkoxy du groupe (alkyl en C 1-4 oxy)-carbonyle de formule R₁₀ et/ou le groupe alkyle ayant de 1 à 4 atomes de carbone de formule R₁ et/ou R₂ a/ont 1 ou 2 atome(s) de carbone; et/ou le groupe alkyle ayant de 1 à 8 atomes de carbone de formule R₃ a 1 à 6 et en particulier 1 à 3 atome(s) de carbone; et/ou le(s) groupe(s) alkényle(s) ayant de 2 à 6 atomes de carbone de fromule R₃ ou R₄ et/ou R₅ et/ou R₇ et/ou R₈ a/ont 2 à 4 et en particulier 3 atomes de carbone; et/ou le groupe alkyle ayant de 1 à 12 atomes de carbone de formule R₄ et/ou R₅ et/ou R₇ et/ou R₈ a/ont 1 à 8 et en particulier 1 à 6 atome(s) de carbone; et/ou le(s) groupe(s) alkyle(s) ayant de 1 à 6 atome(s) de carbone de(s) partie(s) amino-(alkyl en C 1-6) du/des groupe(s) di-(alkyl en C 1-4)-amino-(alkyl en C 1-6) de formule R₄ et/ou R₅ a/ont 1 à 4 atome(s) de carbone; et/ou le/les groupe(s) alkyle(s) ayant 1 à 8 atomes de carbone de(s) partie(s) amino-(alkyl en C 1-8) du/des groupe(s) (alkyl en C 1-4)-amino(alkyl en C 1-8) et/ou di-(alkyl en C 1-4)-amino-(alkyl en C 1-8) de formule R₇ et/ou R₈ a/ont 1 à 6 atome(s) de carbone; et/ou le(s) groupe(s) cycloalkylique(s) ayant de 3 à 8 atomes de carbone de formule R₇ et/ou R₈ a/ont 5 ou 6 atomes de carbone.

4. Procédé selon les revendications 1 à 3, caractérisé en ce que l'on prépare des dérivés de 1,2,4 - tiazolo[1,5 - a]pyrimidine, dans lesquels
Q signifie un groupe morpholino, pipérazino ou pipéridino ou un groupe (alkyl en C 1-6)-thio,
R₁ et R₂ signifient indépendamment l'un de l'autre un atome d'hydrogène ou un groupe alkyle linéaire ou ramifié ayant de 1 à 4 atomes de carbone, ou ensemble ils forment un groupe (-CH₂-)₄ et
Z signifie un groupe de formule E, dans lequel R₇ et R₈ signifient indépendamment l'un de l'autre un groupe alkyle linéaire ou ramifié ayant de 1 à 6 atomes de carbone, ces derniers pouvant porter éventuellement un groupe morpholino, pipérazino ou pipéridino.

5. Procédé selon les revendications 1 à 4, caractérisé en ce que l'on prépare de la 2-(méthylthio)-5-[N-(3'-(morpholino)-propyl)]-amino-6,7,8,9-tétrahydro-1,2,4-triazolo[5,1-b]quinazoline, de la 5-(diéthylamino)-7-méthyl-2-morpholino-1,2,4-triazolo[1,5-a]pyrimidine, de la 2-[1'-(méthyl)-éthyl-thio]-5-(N-[3'-(morpholino)-propyl])-amino-6,7,8,9-tétrahydro-1,2,4-triazolo[5,1-b]quinazoline, de la 2-(éthylthio-5-(N-[2'-(morpholino)-éthyl])-amino-6,7,8,9-tétrahydro-1,2,4-triazolo[5,1-b]quinazoline et de la 2-(métylthio)-5-(N-[2'-(morpholino)-éthyl])-amino-6,7,8,9,10,11,12,13,14,15-décahydro-cyclododéca[d]-1,2,4-triazolo[1,5-a]pyrimidine et leurs sels.

6. Procédé selon les revendications 1 à 5, caractérisé en ce que la conversion des dérivés de 1,2,4 - triazolo[1,5 - a]pyrimidine de formule générale (II) est réalisée avec un excès des amines de formule générale (III).

7. Procédé selon les revendications 1 à 6, caractérisé en ce que la conversion des dérivés de 1,2,4 - triazolo[1,5 - a]pyrimidine de formule générale (II), dans laquelle L signifie un atome d'halogène ou un groupe (alkyl en C 1-4)-sulfonyloxy, arylsulfonyloxy ou (alkyl en C 1-4)-sulfonyloxy, avec l'amine ou le thiol de formule générale (III), est réalisée dans un solvant protique, polaire-aprotique ou apolaire-aprotique, en la présence d'un agent basique liant les acides.
